# EUROPEAN PATENT SPECIFICATION

(11) **EP 4 243 732 B1**
(45) Date of publication and mention of the grant of the patent: **29.04.2026**
(21) Application number: 21820412.1
(22) Date of filing: 12.11.2021
(51) Int. Cl.: A61F 2/24, A61F 2/95

(54) **DEVICES FOR CONTROLLING FLUID FLOW IN A DELIVERY APPARATUS**
VORRICHTUNGEN ZUR STEUERUNG DES FLÜSSIGKEITSSTROMS IN EINER ABGABEVORRICHTUNG
DISPOSITIFS DE RÉGULATION D'UN DÉBIT DE FLUIDE DANS UN APPAREIL D'ADMINISTRATION

(30) Priority: 13.11.2020 US 202063113322 P
(43) Date of publication of application: 20.09.2023
(73) Proprietor: Edwards Lifesciences Corporation, Irvine, CA 92614-5688 (US)
(72) Inventor: REED, Kurt Kelly, Irvine, CA 92614 (US)
(74) Representative: Eisenführ Speiser
(86) International application number: PCT/US2021/059075
(87) International publication number: WO 2022/104013

(56) References cited:
- WO-A1-2018/118717
- WO-A2-02/32326
- US-A1- 2006 030 923

## Description

### CROSS REFERENCE TO RELATED APPLICATION

This application claims the benefit of U.S. Provisional Patent Application No. 63/113,322, filed November 13, 2020.

### FIELD

The present disclosure relates to delivery apparatuses for docking devices configured to secure a prosthetic valve at a native heart valve and associated flow systems.

### BACKGROUND

Prosthetic valves can be used to treat cardiac valvular disorders. Native heart valves (e.g., the aortic, pulmonary, tricuspid and mitral valves) function to prevent backward flow or regurgitation, while allowing forward flow. These heart valves can be rendered less effective by congenital, inflammatory, infectious conditions, etc. Such conditions can eventually lead to serious cardiovascular compromise or death. In the past, such disorders may be treated with surgical repair or replacement of the valve during open heart surgery.

A transcatheter technique for introducing and implanting a prosthetic heart valve using a catheter in a manner that is less invasive than open heart surgery can reduce complications associated with open heart surgery. In this technique, a prosthetic valve can be mounted in a compressed state on a distal end portion of a delivery apparatus and advanced through a blood vessel of the patient until the valve reaches the implantation site. The valve at the distal end portion of the delivery apparatus can then be expanded to its functional size at the site of the defective native valve, such as by inflating a balloon on which the valve is mounted. Alternatively, the valve can have a resilient, self-expanding stent or frame that expands the valve to its functional size when it is advanced from a delivery sheath at the distal end of the delivery apparatus. Optionally, the valve can have a balloon-expandable, self-expanding, mechanically expandable frame, and/or a frame expandable in multiple or a combination of ways.

Transcatheter heart valves (THVs) may be appropriately sized to be placed inside many native aortic valves. However, native mitral and tricuspid valve may have a different geometry than typical aortic valve, and, mitral and tricuspid valve anatomy can vary significantly from person to person. Thus, it can be difficult to appropriately size and shape a prosthetic valve for many patients. Further, when treating valve insufficiency, the surrounding tissue may not be strong enough to hold certain types of valves in position as desired.

In some examples, a docking device can be implanted first within the native valve and can be configured to receive a prosthetic valve and secure (e.g., anchor) the prosthetic valve in a desired position within the native valve. For example, the docking device can form a more circular and/or stable anchoring site at the native valve annulus in which a prosthetic valve can be expanded and implanted. A transcatheter delivery apparatus can be used to deliver the docking device to the implantation site. The docking device can be arranged within the delivery apparatus, coaxial with additional components of the delivery apparatus. Multiple lumens can be disposed between the coaxial components of the delivery apparatus, and a flush fluid may be provided to these lumens, during an implantation procedure, in order to reduce or prevent thrombosis between components, including around the docking device. However, since these lumens can have different resistances than one another and the resistance of the lumens can change during the implantation procedure, it can be difficult to maintain a constant flow of flush fluid in the various lumens. Accordingly, improvements to the transcatheter delivery apparatus to ensure a specified flow of fluid through the various lumens of the delivery apparatus to prevent thrombus formation is desirable.

Exemplary systems and methods usable in delivering a docking device to a native valve of a patient's heart are disclosed in WO 2018/118717 A1 (WO'717). A distal region of a delivery catheter can be positioned in an atrium of the heart and a distal tip can be positioned at or near a commissure of the native valve. The docking device can be located within the delivery catheter. A pusher, such as a pusher wire or tube, of a pusher tool can be advanced distally through the delivery catheter, wherein the pusher can push the docking device along within the delivery catheter. The docking device can be connected to the pusher tool by a line, such as a suture. A member of the pusher tool can be rotatable to change the amount of the suture extending from the pusher tool (cf. abstract of WO'717).

### SUMMARY

Described herein are docking devices, prosthetic heart valves, delivery apparatuses, and methods for implanting docking devices and prosthetic heart valves within the docking devices. Also described herein are examples of delivery apparatuses, flow mechanisms, and related methods for providing a consistent flow of fluid through lumens of a flow system. In some examples, the lumens are part of a delivery apparatus configured to deliver a docking device to a target implantation site in a patient. The docking device can be configured to receive a prosthetic valve therein and securely hold the prosthetic valve in place at the implantation site. By providing a consistent flow of fluid through lumens of such a delivery apparatus, blood stagnation within the delivery apparatus can be reduced or avoided, thereby reducing thrombus formation.

The claimed invention is defined in independent claim 1 and relates to a delivery apparatus configured to deliver a docking device to a target implantation site in a patient, wherein the docking device is configured to receive a prosthetic valve therein and securely hold the prosthetic valve in place at the implantation site. Preferred configurations of the claimed invention are defined in dependent claims 2 to 15. Also described herein are related aspects, examples, embodiments and arrangements useful for understanding the claimed invention.

The delivery apparatus defined in claim 1 comprises an outer shaft configured to retain the docking device in a delivery configuration; an inner shaft disposed within the outer shaft and configured to interface with an end of the docking device and move axially relative to the outer shaft; and a sleeve shaft disposed within the outer shaft, a portion of the sleeve shaft disposed between the outer shaft and the inner shaft, and the sleeve shaft configured to cover the docking device in the delivery configuration. The inner shaft includes one or more openings defined therein that extend between an inner surface and an outer surface of the inner shaft and that are configured to fluidly couple an inner lumen of the inner shaft with a lumen disposed between the outer surface of the inner shaft and an inner surface of the sleeve shaft.

**In** a representative example, a delivery apparatus includes an outer shaft configured to retain a prosthetic implant in a delivery configuration; an inner shaft disposed within the outer shaft and configured to interface with an end of the prosthetic implant and move axially relative to the outer shaft, the inner shaft comprising: a rigid, main tube; and a polymeric distal end portion that comprises a flexible polymer and extends distal to the main tube. The polymeric distal end portion comprises one or more apertures defined therein that extend between an inner surface and an outer surface of the polymeric distal end portion. The delivery apparatus further includes a sleeve shaft disposed within the outer shaft, a portion of the sleeve shaft disposed between the outer shaft and the inner shaft, the sleeve shaft configured to cover the prosthetic implant in the delivery configuration.

In another representative example, a delivery apparatus includes an outer shaft configured to retain a prosthetic implant in a delivery configuration and an inner shaft disposed within the outer shaft and configured to interface with an end of the prosthetic implant and move axially relative to the outer shaft. The inner shaft comprises a rigid, main tube including a distal end portion covered by an outer polymer layer; a polymeric distal end portion that comprises a flexible polymer, is arranged distal to the main tube, and is continuous with the outer polymer layer; and one or more apertures that extend between an outer surface of the inner shaft and an inner surface of the inner shaft, through the outer polymer layer and the main tube. The delivery apparatus further comprises a sleeve shaft disposed within the outer shaft, a portion of the sleeve shaft disposed between the outer shaft and the inner shaft, the sleeve shaft configured to cover the prosthetic implant in the delivery configuration.

The various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. Rather, the claimed invention is defined by independent claim 1. The foregoing and other objects, features, and advantages of the disclosure will become more apparent from the following detailed description, claims, and accompanying figures.

### BRIEF DESCRIPTION OF THE DRAWINGS

FIG. 1 schematically illustrates a docking device delivery apparatus implanting a docking device for a prosthetic heart valve at a mitral valve of a patient, according to an example.
FIG. 2A schematically illustrates the docking device of FIG. 1 fully implanted at the mitral valve of the patient after the docking device delivery apparatus has been removed from the patient.
FIG. 2B schematically illustrates a prosthetic heart valve delivery apparatus implanting a prosthetic heart valve in the implanted docking device of FIG. 2A at the mitral valve of the patient, according to an example.
FIG. 3 is a side perspective view of a docking device in a helical configuration, according to one example.
FIG. 4 is a side view of an exemplary example of a delivery apparatus for a docking device, the delivery apparatus including a handle assembly and an outer shaft extending distally from the handle assembly.
FIG. 5 is a side view of a hub assembly of the handle assembly of the delivery apparatus of FIG. 4.
FIG. 6 is a first cross-sectional view of the hub assembly of FIG. 5 which illustrates fluid flow through lumens of the handle assembly.
FIG. 7 is a second cross-sectional view of a more detailed view of the hub assembly of FIG. 6 which illustrates fluid flow through lumens of the handle assembly.
FIG. 8 is a cross-sectional perspective view of a portion of the delivery apparatus of FIG. 4, arranged between a distal end portion and the hub assembly of the delivery apparatus, which illustrates a flow of fluid through internal components of the delivery apparatus.
FIG. 9A is a cross-sectional view of a distal end portion of the delivery apparatus of FIG. 4, which illustrates a flow of fluid through internal components of the delivery apparatus when a pusher shaft is spaced away from a docking device.
FIG. 9B is another cross-sectional view of the distal end portion of the delivery apparatus of FIG. 4, which illustrates a flow of fluid through internal components of the delivery apparatus when the pusher shaft is arranged against the docking device.
FIG. 10 is a perspective view of a distal end portion of the delivery apparatus of FIG. 4, which illustrates an exemplary docking device deployed from an outer shaft of the delivery apparatus and covered by a sleeve shaft of the delivery apparatus.
FIG. 11 is a perspective view of a distal end portion of the delivery apparatus of FIG. 4, which illustrates the exemplary docking device of FIG. 10 deployed from the outer shaft of the delivery apparatus with the sleeve shaft removed from the docking device.
FIG. 12 is a top perspective view of an example of a flow mechanism configured to maintain a consistent relative flow rate between two or more flow paths, the flow mechanism including two paddle gears rotatably coupled to one another.
FIG. 13 is a side perspective view of the flow mechanism of FIG. 12.
FIG. 14 is a top view of the flow mechanism of FIG. 12, which illustrates a flow of fluid through the flow mechanism.
FIG. 15 is a top view of another example of a flow mechanism that includes paddle gears with different diameter gears.
FIG. 16 is a top view of another example of a flow mechanism that includes paddle gears with paddles having different geometries.
FIG. 17 is a perspective view of another example of a flow mechanism that includes more than two flow paths and two corresponding paddle gears.
FIG. 18 is a perspective view of another example of a flow mechanism that includes four flow paths and four paddle gears, each paddle gear including a paddle rotatably coupled to a common rotating member shared by a paddle of another paddle gear.
FIG. 19 is a perspective view of another example of a flow mechanism that includes include a spacer disposed between a first paddle of a first paddle gear and a second paddle of a second paddle gear.
FIG. 20 is a perspective view of another example of a flow mechanism that includes two paddle gears with paddles arranged at offset heights.
FIG. 21 is a top view of an exemplary example of a single fluid supply coupled to the flow mechanism of FIG. 12.
FIG. 22 is a top view of another example of a flow mechanism that includes a driving member configured to drive rotation of paddle gears of the flow mechanism at a specified rate.
FIG. 23 show an exemplary arrangement of a flow throttle configured to control a flow of fluid into two flow lumens of a delivery apparatus disposed within the hub assembly of FIG. 7.
FIG. 24 is a perspective view of an example of a flow throttle configured to control a flow of fluid into at least two separate flow lumens.
FIG. 25 is an end view of the flow throttle of FIG. 24.
FIG. 26 is a cross-sectional view of the flow throttle of FIG. 24 disposed in a larger lumen of the two flow lumens and sealed around a smaller lumen of the two flow lumens.
FIG. 27 is an end view of another example of a flow throttle configured to control a flow of fluid into at least three separate flow lumens.
FIG. 28 is an end view of another example of a flow throttle configured to control a flow of fluid into at least two separate flow lumens.
FIG. 29 is a schematic illustrating four major components of an exemplary pusher shaft of a delivery apparatus for a docking device.
FIG. 30 is a side cross-sectional view of an example of a pusher shaft of a delivery apparatus for a docking device.
FIG. 31 is a side cross-sectional view of an exemplary distal end of the pusher shaft of FIG. 30.
FIG. 32 is a proximal end view of the pusher shaft of FIG. 30
FIG. 33 is a side view of a main tube of the pusher shaft of FIG. 30.
FIG. 34 is a cross-sectional side view of an exemplary arrangement of the pusher shaft of FIG. 30 assembled together with a sleeve shaft and outer shaft of a delivery apparatus, with the assembly in a first configuration, during deployment of a docking device from the delivery apparatus.
FIG. 35 is a cross-sectional side view of the pusher shaft and sleeve shaft assembly of FIG. 34, with the assembly in a second configuration, after retracting the sleeve shaft from the deployed docking device.
FIG. 36 is a perspective view of an example of a distal tip of a pusher shaft, the distal tip including a slot disposed therein.
FIG. 37 is a side view of the distal tip of FIG. 36.
FIG. 38 is a perspective view of another example of a distal tip of a pusher shaft, the distal tip including two slots disposed therein.
FIG. 39 is a perspective view of another example of a distal tip of a pusher shaft, the distal tip including a slot with a varying width disposed therein.
FIG. 40 is a side view of the distal tip of FIG. 39.
FIG. 41 is a side view of an example of a distal end portion of a main tube of a pusher shaft with one or more apertures disposed therein that are configured to provide a path for fluid to flow out of the pusher shaft.
FIG. 42 is a side view of another example of a distal end portion of a main tube of a pusher shaft with multiple apertures disposed therein that are configured to provide a path for fluid to flow out of the pusher shaft.
FIG. 43 is a cross-sectional side view of an example of a distal end portion of a pusher shaft which includes a distal tip with one or more apertures that are configured to provide one or more additional flow paths for fluid to flow out of the pusher shaft.
FIG. 44 is a side view of the distal end portion of the pusher shaft of FIG. 43.
FIG. 45 is a cross-sectional side view of another example of a distal end portion of a pusher shaft which includes a polymeric tip with one or more apertures disposed therein that are configured to provide one or more additional flow paths for fluid to flow out of the pusher shaft.
FIG. 46 is a perspective view of the distal end portion of the pusher shaft of FIG. 45.
FIG. 47 is a cross-sectional view of a distal end portion of a delivery apparatus, which illustrates a flow of fluid through internal components of the delivery apparatus when the pusher shaft of FIGS. 45 and 46 is arranged against a docking device.
FIG. 48 is a side cross-sectional view of an exemplary sleeve shaft for a delivery apparatus for a docking device.
FIG. 49 is a perspective view of a proximal section of the sleeve shaft of FIG. 48.
FIG. 50 is a perspective view of an exemplary hemostatic seal configured to seal around a sleeve shaft of a delivery apparatus for a docking device.
FIG. 51 is a perspective view of the hemostatic seal of FIG. 50 positioned around a cut portion of the sleeve shaft of FIG. 49.
FIG. 52 is a perspective view of a sleeve shaft arranged around a pusher shaft of a delivery apparatus for a docking device, wherein a proximal extension of the pusher shaft extends out of an opening in a cut portion of the sleeve shaft.
FIG. 53 is a cross-sectional view of the cut portion of the sleeve shaft of FIG. 52 illustrating sharp edges of a cut surface of the cut portion.
FIG. 54A is a schematic showing a laser being applied to the cut surface of the cut portion of FIG. 53 in order to melt and round the sharp edges of the cut surface.
FIG. 54B is a schematic showing a rounded surface achieved from the laser applied in FIG. 54A.
FIG. 55 is a perspective view of an exemplary cut portion of a sleeve shaft showing a first portion of the cut portion prior to application of the laser and a second portion of the sleeve shaft following application of the laser which results in rounded edges and/or a rounded surface at the cut surface of the cut portion.
FIG. 56 is a schematic showing a deburring machine bit being applied to and run along the cut surface of the cut portion of FIG. 53 in order to deburr and/or round the sharp edges of the cut surface.

### DETAILED DESCRIPTION

### General Considerations

For purposes of this description, certain aspects, advantages, and novel features of examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

### Examples of the Disclosed Technology

Described herein are various systems, apparatuses, methods, or the like, that, in some examples, can be used in or with delivery apparatuses for docking devices. In some examples, such systems, apparatuses, and/or methods can provide a consistent flow of fluid through two or more lumens of the delivery apparatus.

The delivery apparatus is configured to deliver and implant a docking device at an implantation site, such as a native valve annulus. The docking device can be configured to more securely hold an expandable prosthetic valve (e.g., a transcatheter heart valve) implanted within the docking device, at the native valve annulus. For example, a docking device can provide or form a more circular and/or stable anchoring site, landing zone, or implantation zone at the implant site, in which a prosthetic valve can be expanded or otherwise implanted. By providing such anchoring or docking devices, replacement prosthetic valves can be more securely implanted and held at various valve annuluses, including at the mitral annulus which does not have a naturally circular cross-section.

The docking device is arranged within an outer shaft of the delivery apparatus and a sleeve shaft (also referred to herein as a delivery sleeve) covers/ surrounds the docking device within the delivery apparatus and during implantation at the target implantation site. A pusher shaft is disposed within the outer shaft, proximal to the docking device, and configured to push the docking device out of the outer shaft to position the docking device at the target implantation site. The sleeve shaft also surrounds the pusher shaft within the outer shaft of the delivery apparatus. After positioning the docking device at the target implantation site, the sleeve shaft can be removed from the docking device and retracted back into the outer shaft of the delivery apparatus.

Fluid (e.g., a flush fluid, such as heparinized saline) can be provided to a pusher shaft lumen defined within an interior of the pusher shaft and a delivery shaft lumen defined between the sleeve shaft and the outer shaft of the delivery apparatus. Fluid from the pusher shaft lumen can then flow to a sleeve shaft lumen defined between the docking device and the sleeve shaft and the sleeve shaft and the pusher shaft. By providing a consistent flow of fluid through these lumens of the delivery apparatus, stagnation of blood within the delivery apparatus can be reduced or avoided, thereby decreasing or preventing thrombus formation.

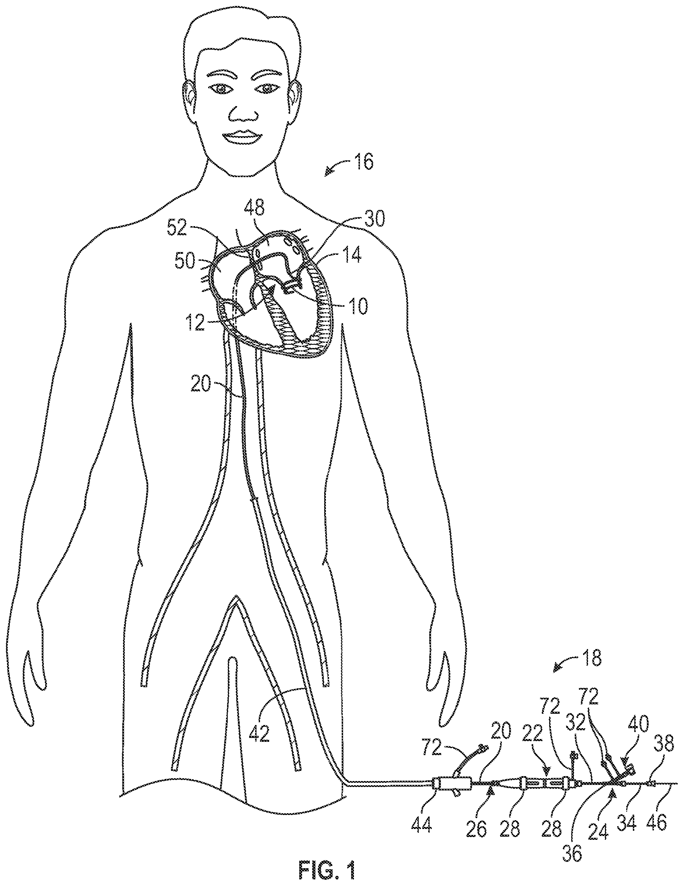

An exemplary transcatheter heart valve replacement procedure which utilizes a first exemplary delivery apparatus to deliver a docking device to a native valve annulus and then a second exemplary delivery apparatus to deliver a transcatheter prosthetic heart valve (THV) inside the docking device is depicted in the schematic illustrations of FIGS. 1-2B.

As introduced above, defective native heart valves may be replaced with transcatheter prosthetic heart valves (THVs). However, such THVs may not be able to sufficiently secure themselves to the native tissue (e.g., to the leaflets and/or annulus of the native heart valve) and may undesirably shift around relative to the native tissue, leading to paravalvular leakage, valve malfunction, and/or other issues. Thus, a docking device may be implanted first at the native valve annulus and then the THV can be implanted within the docking device to help anchor the THV to the native tissue and provide a seal between the native tissue and the THV.

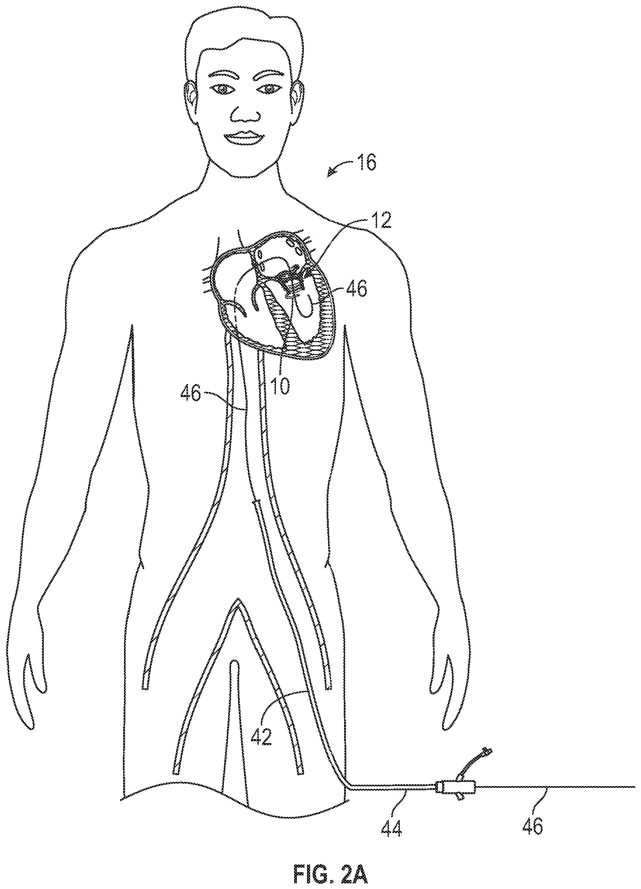

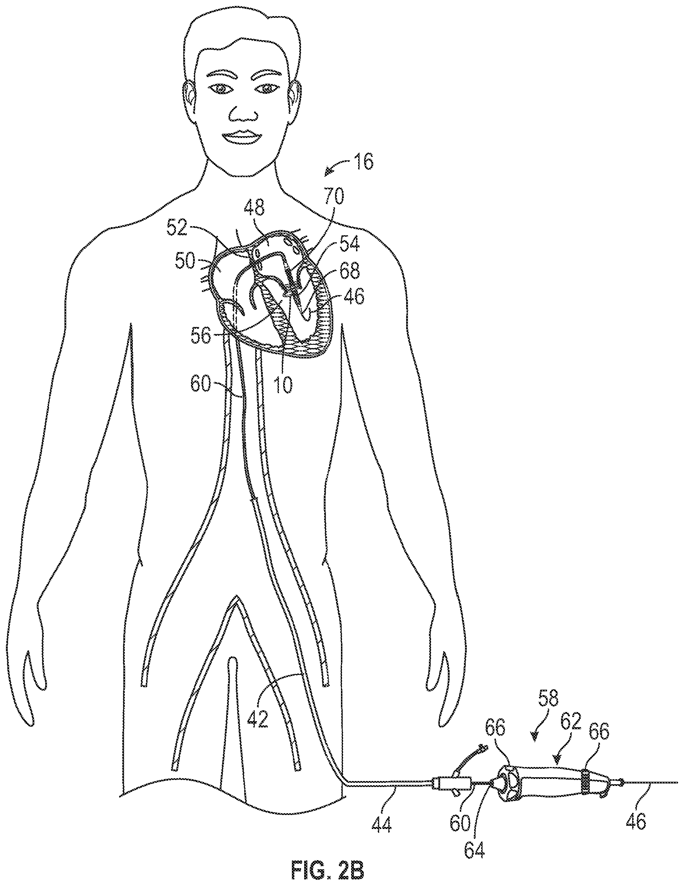

FIGS. 1-2B depict an exemplary transcatheter heart valve replacement procedure which utilizes a docking device, according to one example. During the procedure, a user first delivers and implants the docking device at a patient's native heart valve using a docking device delivery apparatus (FIG. 1), then removes the docking device delivery apparatus from the patient after implanting the docking device (FIG. 2A), and finally implants the prosthetic valve within the implanted docking device using a prosthetic valve delivery apparatus (FIG. 2B).

FIG. 1 depicts a first stage in an exemplary mitral valve replacement procedure where a docking device 10 is being implanted at a mitral valve 12 of a heart 14 of a patient 16 using a docking device delivery apparatus 18 (which may also be referred to herein as "catheter" and/or "docking device delivery device").

In general, the docking device delivery apparatus 18 comprises a delivery shaft 20, a handle 22, and a pusher assembly 24. The delivery shaft 20 is configured to extend into the patient's vasculature and provide a passageway for the docking device 10 to reach the implantation site (e.g., mitral valve 12). Specifically, the delivery shaft 20 may be configured to be advanced through the patient's vasculature to the implantation site by the user and may be configured to receive and/or retain the docking device 10 therein. In some examples, the delivery shaft 20 may comprise an outer sheath or shaft that defines a lumen, and the pusher assembly 24 and/or docking device 10 may be configured to be received and/or advanced within this lumen.

Handle 22 is configured to be gripped and/or otherwise held by the user to advance the delivery shaft 20 through the patient's vasculature. Specifically, the handle 22 is coupled to a proximal end 26 of the delivery shaft 20 and is configured to remain accessible to the user (e.g., outside the patient 16) during the docking device implantation procedure. In this way, the user can advance the delivery shaft 20 through the patient's vasculature by exerting a force on (e.g., pushing) the handle 22. In some examples, the delivery shaft 20 may be configured to carry the pusher assembly 24 and/or docking device 10 with it as it advances through the patient's vasculature. In this way, the docking device 10 and/or pusher assembly 24 may advance through the patient's vasculature in lockstep with the delivery shaft 20 as the user grips the handle 22 and pushes the delivery shaft 20 deeper into the patient's vasculature.

In some examples, the handle 22 may comprise one or more articulation members 28 that are configured to aid in navigating the delivery shaft 20 through the patient's vasculature. Specifically, the articulation members 28 may comprise one or more of knobs, buttons, wheels, and/or other types of physically adjustable control members that are configured to be adjusted by the user to flex, bend, twist, turn, and/or otherwise articulate a distal end 30 of the delivery shaft 20 to aid in navigating the delivery shaft 20 through the patient's vasculature.

Pusher assembly 24 is configured to deploy and/or implant the docking device 10 at the implantation site (e.g., native valve). Specifically, the pusher assembly 24 is configured to be adjusted by the user to advance the docking device 10 through the delivery shaft 20 and push the docking device 10 out of the distal end 30 of the delivery shaft 20. As described above, the pusher assembly 24 may be configured to extend through the delivery shaft 20, within the lumen defined by the outer sheath of the delivery shaft 20. The pusher assembly 24 also may be coupled to the docking device 10 such that the pusher assembly 24 pushes the docking device 10 through and/or out of the delivery shaft 20 as the pusher assembly 24 advances through the delivery shaft 20. Stated slightly differently, because it is retained by, held, and/or otherwise coupled to the pusher assembly 24, the docking device 10 may advance in lockstep with the pusher assembly 24 through and/or out of the delivery shaft 20.

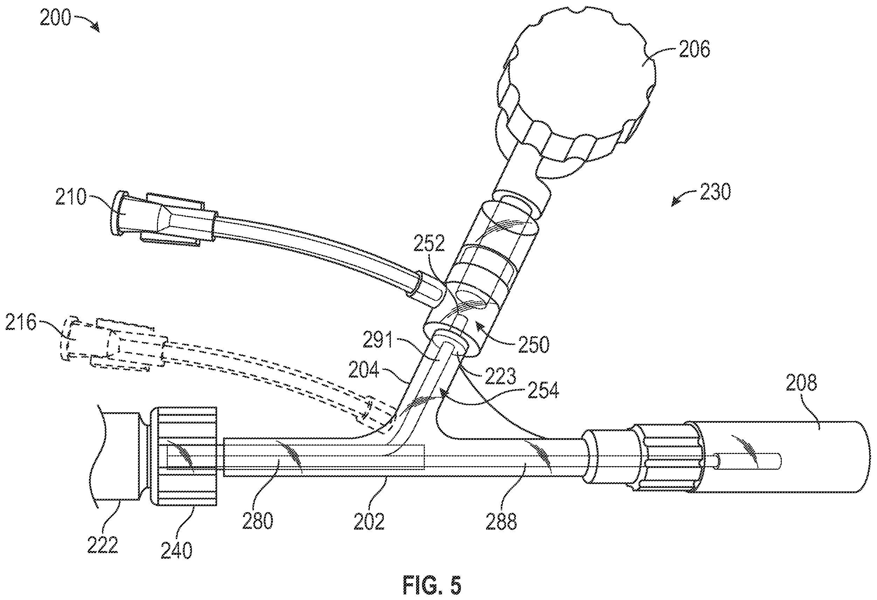

The pusher assembly 24 comprises a pusher shaft 32 and, in some examples, may also include a sleeve shaft 34. The pusher shaft 32 is configured to advance the docking device 10 through the delivery shaft 20 and out of the distal end 30 of the delivery shaft 20, while the sleeve shaft 34, when included, may be configured to cover the docking device 10 within the delivery shaft 20 and while pushing the docking device 10 out of the delivery shaft 20 and positioning the docking device 10 at the implantation site. In some examples, the pusher shaft 32 can be covered by the sleeve shaft 34 and arranged within an outer shaft or connector of a pusher handle (or hub assembly) 36 (e.g., as shown in FIG. 5-7, as described further below).

In some examples, the pusher assembly 24 may comprise the pusher handle (also referred to herein as a hub assembly) 36 that is coupled to the pusher shaft 32 and that is configured to be gripped and pushed by the user to translate the pusher shaft 32 axially relative to the delivery shaft 20 (e.g., to push the pusher shaft 32 into and/or out of the distal end 30 of the delivery shaft 20). The sleeve shaft 34 may be configured to be retracted and/or withdrawn from the docking device 10, after positioning the docking device 10 at the implantation site. For example, the pusher assembly 24 may include a sleeve handle 38 that is coupled to the sleeve shaft 34 and is configured to be pulled by a user to retract (e.g., axially move) the sleeve shaft 34 relative to the pusher shaft 32.

The pusher assembly 24 may be removably coupled to the docking device 10 and as such may be configured to release, detach, decouple, and/or otherwise disconnect from the docking device 10 once the docking device 10 has been deployed at the implantation site. As just one example, the pusher assembly 24 (e.g., pusher shaft 32) may be removably coupled to the docking device 10 via a thread, string, yarn, suture, or other suitable material that is tied or sutured to the docking device 10.

In some examples, the pusher assembly 24 comprises a suture lock assembly 40 that is configured receive and/or hold the thread or other suitable material that is coupled to the docking device 10 via the suture. Thus, the thread or other suitable material that forms the suture may extend from the docking device 10, through the pusher assembly 24, to the suture lock assembly 40. The suture lock assembly 40 may also be configured to cut the thread to release, detach, decouple, and/or otherwise disconnect the docking device 10 from the pusher assembly 24. For example, the suture lock assembly 40 may comprise a cutting mechanism that is configured to be adjusted by the user to cut the thread.

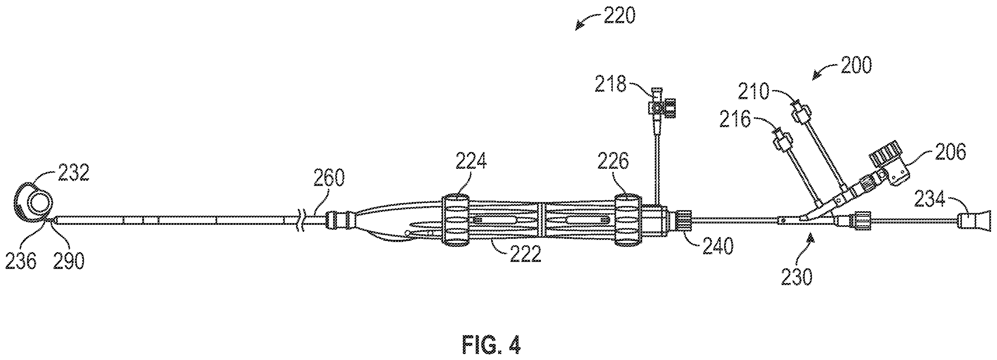

Further details of the docking device delivery apparatus and its variants are described further below with reference to FIGS. 4-11 and are described in International Application No. PCT/US20/36577.

Before inserting the docking device delivery apparatus 18 into the vasculature of the patient 16, the user may first make an incision in the patient's body to access a blood vessel 42. For example, in the example illustrated in FIG. 1, the user may make an incision in the patient's groin to access a femoral vein. Thus, in such examples, the blood vessel 42 may be a femoral vein.

After making the incision at the blood vessel 42, the user may insert an introducer device 44, a guidewire 46, and/or other devices (e.g., delivery shaft 20, pusher shaft 32, and/or sleeve shaft 34 of the docking device delivery apparatus 18, catheters, and/or other delivery apparatuses, docking device 10, prosthetic valves, etc.) through the incision and into the blood vessel 42. The introducer device 44 (which can include an introducer sheath) is configured to facilitate the percutaneous introduction of the guidewire 46 and/or the other devices (e.g., docking device delivery apparatus 18) into and through the blood vessel 42 and may extend through only a portion of the blood vessel 42 even when it is fully inserted by the user (i.e., it may extend through the blood vessel 42 towards the heart 14, but may stop short of the heart 14). The guidewire 46 on the other hand, is configured to guide the delivery apparatuses (e.g., docking device delivery apparatus 18, prosthetic valve delivery apparatuses, catheters, etc.) and their associated devices (e.g., docking device, prosthetic heart valve, etc.) to the implantation site within the heart 14, and thus may extend all the way through the blood vessel 42 and into a left atrium 48 of the heart 14. Specifically, the user may advance the guidewire 46 through the blood vessel 42 (e.g., through the femoral vein and inferior vena cava) to a right atrium 50 of the heart 14. The user may make a small incision in an atrial septum 52 of the heart 14 to allow the guidewire 46 to pass from the right atrium 50 to the left atrium 48 of the heart 14 and may then advance the guidewire 46 through the incision in the atrial septum 52 into the left atrium 48. Thus, the guidewire 46 may provide a pathway that the docking device delivery apparatus 18 can follow as it advances through the patient's vasculature to ensure that the docking device delivery apparatus 18 does not perforate the walls of the blood vessel 42 and/or other vasculature tissue.

After positioning the guidewire 46 within the left atrium 48, the user may insert the docking device delivery apparatus 18 (e.g., delivery shaft 20) into the patient 16 by advancing the docking device delivery apparatus 18 through the introducer device 44 and over the guidewire 46. The user may then continue to advance the docking device delivery apparatus 18 through the patient's vasculature along the guidewire 46 until the docking device delivery apparatus 18 reaches the left atrium 48, as illustrated in FIG. 1. Specifically, the user may advance the delivery shaft 20 of the docking device delivery apparatus 18 by gripping and exerting a force on (e.g., pushing) the handle 22 of the docking device delivery apparatus 18. While advancing the delivery shaft 20 through the patient's vasculature, the user may adjust the one or more articulation members 28 of the handle 22 to navigate the various turns, corners, constrictions, and/or other obstacles in the patient's vasculature.

Once the delivery shaft 20 reaches the left atrium 48, the user may position the distal end 30 of the delivery shaft 20 at and/or near the posteromedial commissure of the mitral valve 12 using the handle 22 (e.g., the articulation members 28). The user may then push the docking device 10 out of the distal end 30 of the delivery shaft 20 with the pusher assembly 24 to deploy and/or implant the docking device 10 at the mitral valve 12. For example, the user may actuate the pusher handle 36 to axially translate the pusher shaft 32, in a distal direction, relative to the delivery shaft 20, such that the docking device 10 (which can be covered by the sleeve shaft 34) is deployed out of the delivery shaft 20 and moved into a desired position at the implantation site.

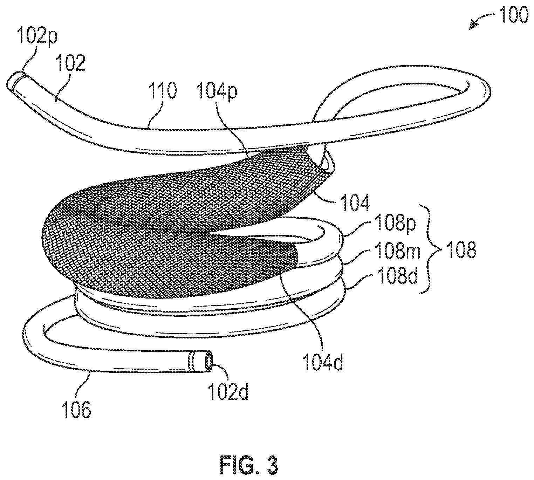

In some examples, the docking device 10 may be constructed from, formed of, and/or comprise a shape memory material, and as such, may return to its original, pre-formed shape when it exits the delivery shaft 20 and is no longer constrained by the delivery shaft 20. As one example, the docking device 10 may originally be formed as a coil, and thus may wrap around the ventricular side of the leaflets as it exits the delivery shaft 20 and returns to its original coiled configuration (e.g., as shown in FIG. 3, as described further below).

After pushing the ventricular portion of the docking device 10 (i.e., the portion of the docking device 10 that is configured to be positioned/disposed within a left ventricle 56 and/or on the ventricular side of the mitral valve leaflets), the user may then release the remaining portion of the docking device 10 (the atrial portion of the docking device 10) from the delivery shaft 20 within the left atrium 48. Specifically, the user may retract the delivery shaft 20 relative to the docking device 10, away from the posteromedial commissure of the mitral valve 12. In some examples, the user may maintain the position of the pusher shaft 32 (e.g., by exerting a holding and/or pushing force on the pusher shaft 32) while retracting the delivery shaft 20 so that the delivery shaft 20 withdraws and/or otherwise retracts relative to the docking device 10 and the pusher shaft 32. In this way, the pusher shaft 32 may hold the docking device 10 in place while the user retracts the delivery shaft 20, thereby releasing the docking device 10 from the delivery shaft 20. In some examples, the user may also retract the sleeve shaft 34 from the docking device 10 to uncover the docking device 10, and in some examples, deploy an expandable sleeve of the docking device 10.

After deploying and/or implanting the docking device 10, the user may decouple and/or otherwise disconnect the docking device delivery apparatus 18 from the docking device 10 by, for example, cutting the thread that is sutured to the docking device 10. As just one example, the user may cut the thread with the cutting mechanism of the suture lock assembly 40. Once the docking device 10 is disconnected from the docking device delivery apparatus 18, the user may retract the entire docking device delivery apparatus 18 (the delivery shaft 20, handle 22, and pusher assembly 24) from the patient 16 so that the user can deliver and implant the THV at the mitral valve 12. For example, the docking device 10 and the THV may be delivered on two different, separate delivery apparatuses, and thus the user may need to remove the docking device delivery apparatus 18 from the patient 16 to make room for the THV delivery apparatus. As another example, the user may need to remove the docking device delivery apparatus 18 from the patient 16 to load the THV onto the delivery apparatus. In either example, the user may need to remove the docking device delivery apparatus 18 from the patient 16 before implanting the THV.

FIG. 2A depicts this second stage in the mitral valve replacement procedure, where the docking device 10 has been fully deployed and implanted at the mitral valve 12 and the docking device delivery apparatus 18 (including the delivery shaft 20) has been removed from the patient 16 such that only the guidewire 46 and the introducer device 44 remain inside the patient 16. The introducer device 44 may remain inside the patient 16 to help percutaneously insert the THV and the valve delivery apparatus into the patient 16, while the guidewire 46 may remain within the patient's vasculature to help advance the THV and the valve delivery apparatus through the patient's vasculature. Specifically, the guidewire 46 may ensure that the THV and the valve delivery apparatus do not perforate the walls of the blood vessel 42 and/or other vasculature tissue as they advance through the patient's vasculature. In some examples, the user may advance the guidewire 46 through the mitral valve 12 and into the left ventricle 56 to ensure that the guidewire 46 routes the THV and the valve delivery apparatus all of the way to the mitral valve 12 and into the docking device 10.

As illustrated in FIG. 2A, the docking device 10 may be configured to wrap around the ventricular side of the leaflets of the mitral valve 12 and squeeze the leaflets radially inward (i.e., radially compress the leaflets) to adjust the size and/or shape of the opening between the two leaflets of the mitral valve 12. For example, the docking device 10 may be configured to reduce the size of the opening of the mitral valve 12 and/or to change the shape of the opening to more closely match the cross-sectional shape and/or profile of the THV (e.g., make the opening more circular for a cylindrical THV). By constricting the mitral valve 12 in this manner, the docking device 10 may provide a tighter fit, and thus a better seal, between the THV and the mitral valve 12.

FIG. 2B depicts a third stage in the mitral valve replacement procedure where the user is delivering and/or implanting a prosthetic heart valve 54 (which may also be referred to herein as "heart valve," "transcatheter prosthetic heart valve" or "THV" for short, "replacement heart valve," and/or "prosthetic mitral valve") within the docking device 10 and/or at the mitral valve 12 using a prosthetic heart valve delivery apparatus 58. Thus, the docking device 10 and prosthetic heart valve 54 may be delivered on different delivery apparatuses at different stages in the mitral valve replacement procedure. Specifically, the docking device 10 may be delivered to the mitral valve 12 with the docking device delivery apparatus 18 during the first stage of the mitral valve replacement procedure and the prosthetic heart valve 54 may then be delivered with the prosthetic heart valve delivery apparatus 58.

The prosthetic heart valve delivery apparatus 58 comprises a delivery shaft 60 and a handle 62 coupled to a proximal end 64 of the delivery shaft 60. The delivery shaft 60 is configured to extend into the patient's vasculature to deliver, implant, expand, and/or otherwise deploy the prosthetic heart valve 54 within the docking device 10 at the mitral valve 12. The handle 62 may be the same as, or similar to, handle 22 of the docking device delivery apparatus 18 and is similarly configured to be gripped and/or otherwise held by the user to advance the delivery shaft 60 through the patient's vasculature.

In some examples, handle 62 may comprise one or more articulation members 66 that are configured to aid in navigating the delivery shaft 60 through the patient's vasculature. Specifically, the articulation members 66 may comprise one or more of knobs, buttons, wheels, and/or other types of physically adjustable control members that are configured to be adjusted by the user to flex, bend, twist, turn, and/or otherwise articulate a distal end 68 of the delivery shaft 60 to aid in navigating the delivery shaft 60 through the patient's vasculature.

In some examples, the prosthetic heart valve delivery apparatus 58 may comprise an expansion mechanism 70 that is configured to radially expand and deploy the prosthetic heart valve 54. For example, the expansion mechanism 70 may comprise an inflatable balloon that is configured to be inflated to radially expand the prosthetic heart valve 54 within the docking device 10. The expansion mechanism 70 may be included in and/or coupled to the delivery shaft 60 at and/or proximate to the distal end 68 of the delivery shaft 60. In other examples, the prosthetic heart valve 54 may be self-expanding and may be configured to radially expand on its own without the expansion mechanism 70. In other examples, the prosthetic heart valve 54 may be mechanically expandable and the prosthetic heart valve delivery apparatus 58 can include one or more mechanical actuators configured to radially expand the prosthetic heart valve 54.

Prosthetic heart valve 54 may be coupled to the delivery shaft 60 at and/or proximate to the distal end 68 of the delivery shaft 60. In examples where the prosthetic heart valve delivery apparatus 58 includes the expansion mechanism 70, prosthetic heart valve 54 may be mounted on the expansion mechanism 70 in a radially compressed configuration. In some examples, prosthetic heart valve 54 may be removably coupled to the delivery shaft 60 such that, after the prosthetic heart valve 54 is radially expanded and deployed from the prosthetic heart valve delivery apparatus 58, the prosthetic heart valve delivery apparatus 58 can be retracted away from the implanted prosthetic heart valve 54 and removed from the patient 16.

Prosthetic heart valve 54 is configured to be received and/or retained within the docking device 10. That is, docking device 10 is configured to receive the prosthetic heart valve 54 and help anchor the prosthetic heart valve 54 to the mitral valve 12. As will be explained in further detail below, docking device 10 is also configured to provide a seal between the prosthetic heart valve 54 and the leaflets of the mitral valve to reduce paravalvular leakage around the prosthetic heart valve 54. Specifically, as introduced above, the docking device 10 may initially constrict the leaflets of the mitral valve 12. The prosthetic heart valve 54 may then push the leaflets against the docking device 10 as it radially expands within the docking device 10 (e.g., via inflation of the expansion mechanism 70). Thus, the docking device 10 and the prosthetic heart valve 54 may be configured to sandwich the leaflets of the mitral valve 12 when the prosthetic heart valve 54 is expanded within the docking device 10. In this way, the docking device 10 may provide a seal between the leaflets of the mitral valve 12 and the prosthetic heart valve 54.

In some examples, one or more of the docking device delivery apparatus 18, the prosthetic heart valve delivery apparatus 58, and/or the introducer device 44 may comprise one or more flushing ports 72 (FIG. 1) that are configured to supply flushing fluid to the lumens thereof (e.g., lumens of the delivery shaft 20 of docking device delivery apparatus 18, the delivery shaft 60 of the prosthetic heart valve delivery apparatus 58, and/or the introducer device 44) to prevent and/or reduce the likelihood of blood clot (e.g., thrombus) formation.

Like when delivering the docking device 10, the user may insert the prosthetic heart valve delivery apparatus 58 (e.g., delivery shaft 60) into the patient 16 by advancing the prosthetic heart valve delivery apparatus 58 through the introducer device 44 and over the guidewire 46. The user may continue to advance the prosthetic heart valve delivery apparatus 58 along the guidewire 46 (through the patient's vasculature) until the prosthetic heart valve delivery apparatus 58 reaches the mitral valve 12, as illustrated in FIG. 2B. Specifically, the user may advance the delivery shaft 60 of the prosthetic heart valve delivery apparatus 58 by gripping and exerting a force on (e.g., pushing) the handle 62 of the prosthetic heart valve delivery apparatus 58. While advancing the delivery shaft 60 through the patient's vasculature, the user may adjust the one or more articulation members 66 of the handle 62 to navigate the various turns, corners, constrictions, and/or other obstacles in the patient's vasculature.

The user may advance the delivery shaft 60 along the guidewire 46 until the prosthetic heart valve 54 and/or expansion mechanism 70 is/are positioned/disposed within the docking device 10 and/or the mitral valve 12. For example, the user may advance the delivery shaft 60 along the guidewire 46 until the delivery shaft 60 extends through the mitral valve 12, such that the distal end 68 of the delivery shaft 60 is positioned/disposed within the left ventricle 56. Once the prosthetic heart valve 54 is appropriately positioned/disposed within the docking device 10, the user may radially expand the prosthetic heart valve 54, such as with the expansion mechanism 70, to its fully expanded position or configuration. In some examples, the user may lock the prosthetic heart valve 54 in its fully expanded position (e.g., with a locking mechanism) to prevent the valve from collapsing. After expanding and deploying the prosthetic heart valve 54, the user may decouple and/or otherwise disconnect the delivery shaft 60 from the prosthetic heart valve 54 and remove the delivery shaft 60 from the patient.

Although FIGS. 1-2B specifically depict a mitral valve replacement procedure, it should be appreciated that the same and/or similar procedure may be utilized to replace other heart valves (e.g., tricuspid, pulmonary, and/or aortic valves). Further, the same and/or similar delivery apparatuses (e.g., docking device delivery apparatus 18, prosthetic heart valve delivery apparatus 58, introducer device 44, and/or guidewire 46), docking devices (e.g., docking device 10), replacement heart valves (e.g., prosthetic heart valve 54), and/or components thereof may be utilized for replacing these other heart valves.

For example, when replacing a native tricuspid valve, the user may also access the right atrium 50 via a femoral vein but may not need to cross the atrial septum 52 into the left atrium 48. Instead, the user may leave the guidewire 46 in the right atrium 50 and perform the same and/or similar docking device implantation process at the tricuspid valve. Specifically, the user may push the docking device 10 out of the delivery shaft 20 around the ventricular side of the tricuspid valve leaflets, release the remaining portion of the docking device 10 from the delivery shaft 20 within the right atrium 50, and then remove the delivery shaft 20 of the docking device delivery apparatus 18 from the patient 16. The user may then advance the guidewire 46 through the tricuspid valve into the right ventricle and perform the same and/or similar prosthetic heart valve implantation process at the tricuspid valve, within the docking device 10. Specifically, the user may advance the delivery shaft 60 of the prosthetic heart valve delivery apparatus 58 through the patient's vasculature along the guidewire 46 until the prosthetic heart valve 54 is positioned/disposed within the docking device 10 and the tricuspid valve. The user may then expand the prosthetic heart valve 54 within the docking device 10 before removing the prosthetic heart valve delivery apparatus 58 from the patient 16. In another example, the user may perform the same and/or similar process to replace the aortic valve but may access the aortic valve from the outflow side of the aortic valve via a femoral artery.

Further, although FIGS. 1-2B depict a mitral valve replacement procedure that accesses the mitral valve 12 from the left atrium 48 via the right atrium 50 and femoral vein, it should be appreciated that the mitral valve 12 may alternatively be accessed from the left ventricle 56. For example, the user may access the mitral valve 12 from the left ventricle 56 via the aortic valve by advancing one or more delivery apparatuses through an artery to the aortic valve, and then through the aortic valve into the left ventricle 56.

FIG. 3 shows an example of a docking device 100 configured to receive a prosthetic heart valve. For example, the docking device 100 can be implanted within a native valve annulus, as described above with reference to FIGS. 1 and 2A. As depicted in FIGS. 1-2B, the docking device 100 can be configured to receive and secure a prosthetic valve within the docking device, thereby securing the prosthetic valve at the native valve annulus.

Referring to FIG. 3, the docking device 100 can comprise two main components: a coil 102 and a guard member 104 covering at least a portion of the coil 102. In certain examples, the coil 102 can include a shape memory material (e.g., Nitinol) such that the docking device 100 (and the coil 102) can move from a substantially straight configuration (also referred to as "delivery configuration") when disposed within a delivery sleeve (e.g., sleeve shaft) of a delivery apparatus (as described more fully below) to a helical configuration (also referred to as "deployed configuration," as shown in FIG. 3) after being removed from the delivery sleeve (e.g., sleeve shaft).

The coil 102 has a proximal end 102p and a distal end 102d. When being disposed within the delivery sleeve (e.g., during delivery of the docking device into the vasculature of a patient), a body of the coil 102 between the proximal end 102p and distal end 102d can form a generally straight delivery configuration (e.g., without any coiled or looped portions) so as to maintain a small radial profile when moving through a patient's vasculature. After being removed from the delivery sleeve and deployed at an implant position, the coil 102 can move from the delivery configuration to the helical deployed configuration and wrap around native tissue adjacent the implant position. For example, when implanting the docking device at the location of a native valve, the coil 102 can be configured to surround native leaflets of the native valve (and the chordae tendineae that connects native leaflets to adjacent papillary muscles, if present).

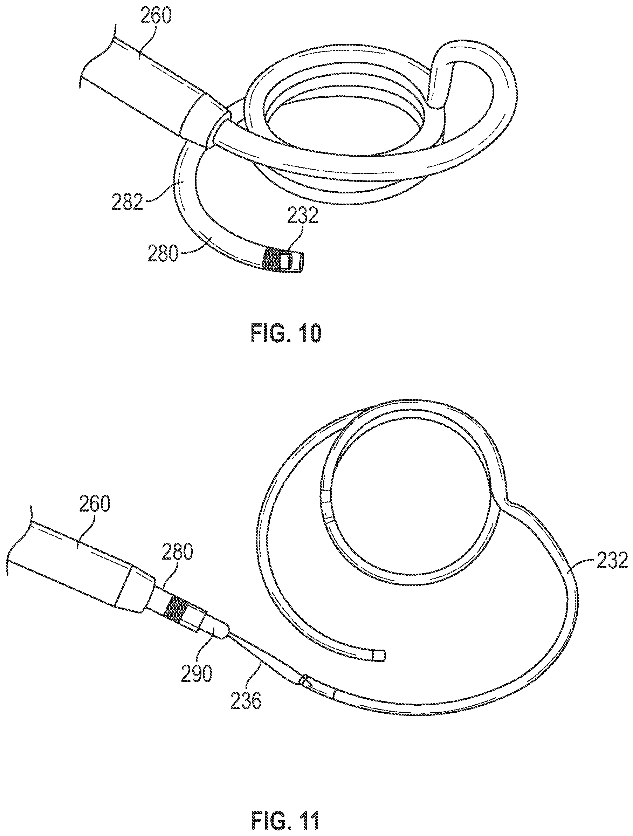

The docking device 100 can be releasably coupled to a delivery apparatus. In certain examples, the docking device 100 can be coupled to a delivery apparatus via a release suture that can be configured to be tied to the docking device 100 and cut for removal (as described further below with reference to FIGS. 4 and 11). In one example, the release suture can be tied to the docking device 100 through an eyelet or eyehole located adjacent the proximal end 102p of the coil. In another example, the release suture can be tied around a circumferential recess that is located adjacent the proximal end 102p of the coil 102.

In some examples, the docking device 100 in the deployed configuration can be configured to fit at the mitral valve position. In other examples, the docking device can also be shaped and/or adapted for implantation at other native valve positions as well, such as at the tricuspid valve. In some examples, the geometry of the docking device 100 can be configured to engage the native anatomy, which can, for example, provide for increased stability and reduction of relative motion between the docking device 100, the prosthetic valve docked therein, and/or the native anatomy. Reduction of such relative motion can, among other things, prevent material degradation of components of the docking device 100 and/or the prosthetic valve docked therein and/or prevent damage or trauma to the native tissue.

As shown in FIG. 3, the coil 102 in the deployed configuration can include a leading turn 106 (or "leading coil"), a central region 108, and stabilization turn 110 (or "stabilization coil"). The central region 108 can possess one or more helical turns having substantially equal inner diameters. The leading turn 106 can extend from a distal end of the central region 108 and has a diameter greater than the diameter of the central region 108 (in one or more configurations). The stabilization turn 110 can extend from a proximal end of the central region 108 and has a diameter greater than the diameter of the central region 108 (in one or more configurations).

In certain examples, the central region 108 can include a plurality of helical turns, such as a proximal turn 108p in connection with the stabilization turn 110, a distal turn 108d in connection with the leading turn 106, and one or more intermediate turns 108m disposed between the proximal turn 108p and the distal turn 108d. In the example shown in FIG. 3, there is only one intermediate turn 108m between the proximal turn 108p and the distal turn 108d. In other examples, there are more than one intermediate turns 108m between the proximal turn 108p and the distal turn 108d. Some of the helical turns in the central region 108 can be full turns (i.e., rotating 360 degrees). In some examples, the proximal turn 108p and/or the distal turn 108d can be partial turns (e.g., rotating less than 360 degrees, such as 180 degrees, 270 degrees, etc.).

A size of the docking device 100 can be generally selected based on the size of the desired prosthetic valve to be implanted into the patient. In certain examples, the central region 108 can be configured to retain a radially expandable prosthetic valve. For example, the inner diameter of the helical turns in the central region 108 can be configured to be smaller than an outer diameter of the prosthetic valve when the prosthetic valve is radially expanded so that additional radial tension can act between the central region 108 and the prosthetic valve to hold the prosthetic valve in place. The helical turns (e.g., 108p, 108m, 108d) in the central region 108 are also referred to herein as "functional turns."

The stabilization turn 110 can be configured to help stabilize the docking device 100 in the desired position. For example, the radial dimension of the stabilization turn 110 can be significantly larger than the radial dimension of the coil in the central region 108, so that the stabilization turn 110 can flare or extend sufficiently outwardly so as to abut or push against the walls of the circulatory system, thereby improving the ability of the docking device 100 to stay in its desired position prior to the implantation of the prosthetic valve. In some examples, the diameter of stabilization turn 110 is desirably larger than the annulus, native valve plane, and atrium for better stabilization. In some examples, the stabilization turn 110 can be a full turn (i.e., rotating about 360 degrees). In some examples, the stabilization turn 110 can be a partial turn (e.g., rotating between about 180 degrees and about 270 degrees).

In one particular example, when implanting the docking device 100 at the native mitral valve location, the functional turns in the central region 108 can be disposed substantially in the left ventricle and the stabilization turn 110 can be disposed substantially in the left atrium. The stabilization turn 110 can be configured to provide one or more points or regions of contact between the docking device 100 and the left atrial wall, such as at least three points of contact in the left atrium or complete contact on the left atrial wall. In certain examples, the points of contact between the docking device 100 and the left atrial wall can form a plane that is approximately parallel to a plane of the native mitral valve.

As noted above, the leading turn 106 can have a larger radial dimension than the helical turns in the central region 108. The leading turn 106 can help more easily guide the coil 102 around and/or through the chordae tendineae geometry and adequately around all native leaflets of the native valve (e.g., the native mitral valve, tricuspid valve, etc.). For example, once the leading turn 106 is navigated around the desired native anatomy, the remaining coil (such as the functional turns) of the docking device 100 can also be guided around the same features. In some examples, the leading turn 106 can be a full turn (i.e., rotating about 360 degrees). In some examples, the leading turn 106 can be a partial turn (e.g., rotating between about 180 degrees and about 270 degrees). In some examples, when a prosthetic valve is radially expanded within the central region 108 of the coil, the functional turns in the central region 108 can be further radially expanded. As a result, the leading turn 106 can be pulled in the proximal direction and become a part of the functional turn in the central region 108.

In certain examples, at least a portion of the coil 102 can be surrounded by a first cover. The first cover can be constructed of various native and/or synthetic materials. In one particular example, the first cover can include expanded polytetrafluoroethylene (ePTFE). In certain examples, the first cover is configured to be fixedly attached to the coil 102 (e.g., by means of textured surface resistance, suture, glue, thermal bonding, or any other means) so that relative axial movement between the first cover and the coil 102 is restricted or prohibited.

The guard member 104 can constitute a part of a cover assembly for the docking device 100. In some examples, the cover assembly can also include the first cover.

In a typical example as shown in FIG. 3, when the docking device 100 is in the deployed configuration, the guard member 104 can be configured to cover a portion of the stabilization turn 110 of the coil 102. In certain examples, the guard member 104 can be configured to cover at least a portion of the central region 108 of the coil 102, such as a portion of the proximal turn 108p. In certain examples, the guard member 104 can extend over the entirety of the coil 102.

In some examples, the guard member 104 can radially expand so as to help prevent and/or reduce paravalvular leakage. Specifically, the guard member 104 can be configured to radially expand such that an improved seal is formed closer to and/or against a prosthetic valve deployed within the docking device 100. In some examples, the guard member 104 can be configured to prevent and/or inhibit leakage at the location where the docking device 100 crosses between leaflets of the native valve (e.g., at the commissures of the native leaflets).

In another example, when the docking device 100 is deployed at a native atrioventricular valve and the guard member 104 covers predominantly a portion of the stabilization turn 110 and/or a portion of the central region 108, the guard member 104 can help cover an atrial side of an atrioventricular valve to prevent and/or inhibit blood from leaking through the native leaflets, commissures, and/or around an outside of the prosthetic valve by blocking blood in the atrium from flowing in an atrial to ventricular direction (i.e., antegrade blood flow)-other than through the prosthetic valve.

In some examples, the guard member 104 can be positioned on a ventricular side of an atrioventricular valve to prevent and/or inhibit blood from leaking through the native leaflets, commissures, and/or around an outside of the prosthetic valve by blocking blood in the ventricle from flowing in a ventricular to atrial direction (i.e., retrograde blood flow).

In some examples, a distal end portion 104d of the guard member 104 can be fixedly coupled to the coil 102 (e.g., via a distal suture), and a proximal end portion 104p of the guard member 104 can be axially moveable relative to the coil 102.

In certain examples, when the guard member 104 is in the radially expanded state, the proximal end portion 104p of the guard member 104 can have a tapered shape as shown in FIG. 3, such that the diameter of the proximal end portion 104p gradually increases from a proximal terminal end of the guard member 104 to a distally located body portion of the guard member 104. This can, for example, help to facilitate loading the docking device into a delivery sleeve (e.g., sleeve shaft) of the delivery apparatus and/or retrieval and/or repositioning of the docking device into the delivery apparatus during an implantation procedure.

FIGS. 4-11 illustrate examples of a delivery apparatus (which can also be referred to as a delivery system) 220 configured to deliver a docking device (such as docking device 100 described above with reference to FIG. 3) to a target implantation site (e.g., a heart and/or native valve of an animal, human, cadaver, cadaver heart, anthropomorphic ghost, and/or the like). In some examples, the delivery apparatus 220 can be a transcatheter delivery apparatus that can be used to guide the delivery of a docking device through a patient's vasculature, as explained above with reference to FIGS. 1 and 2A.

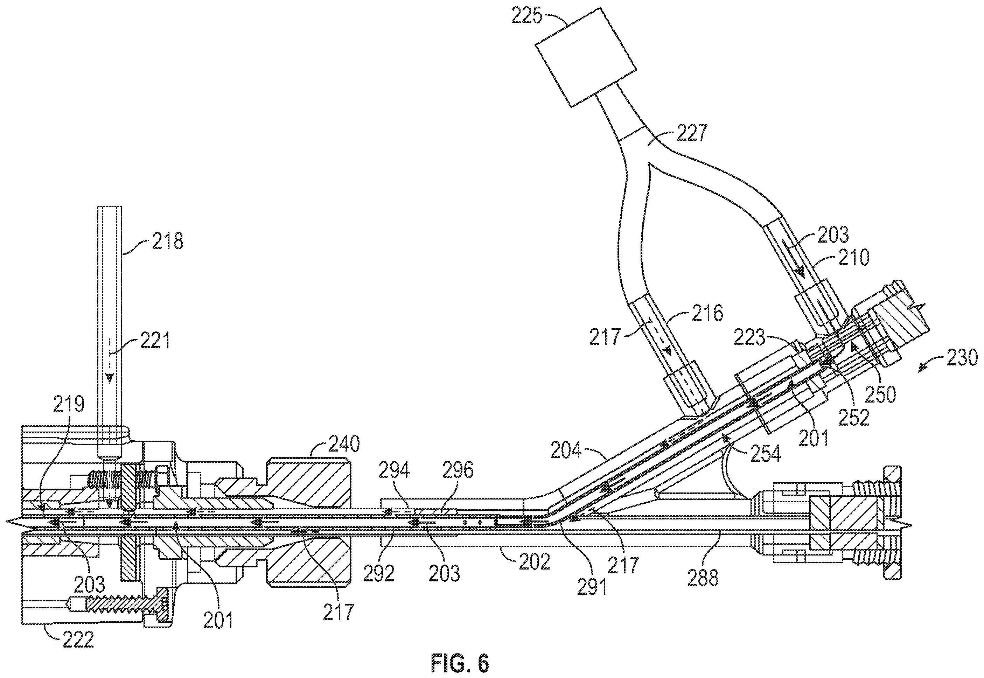

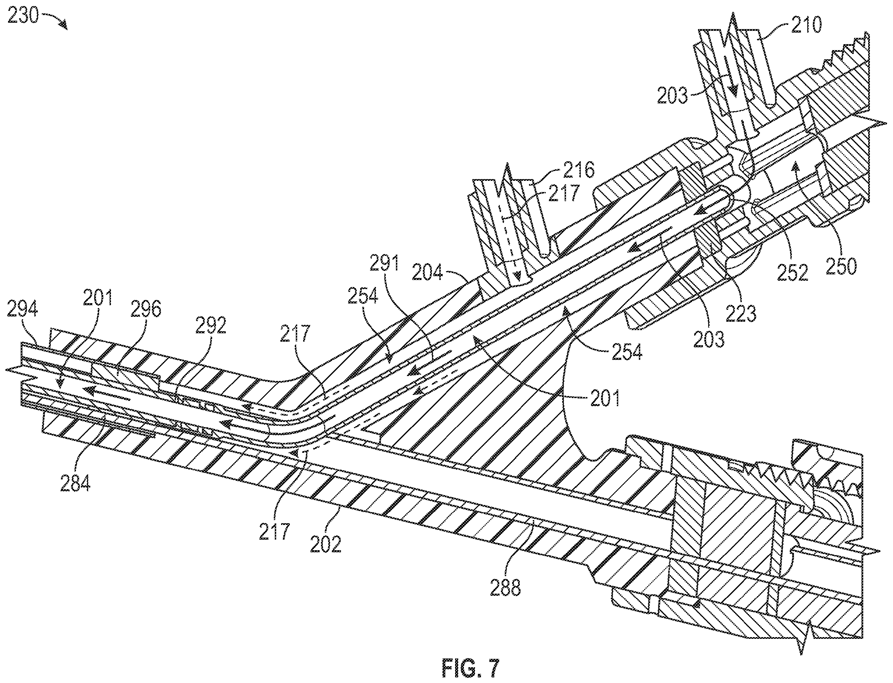

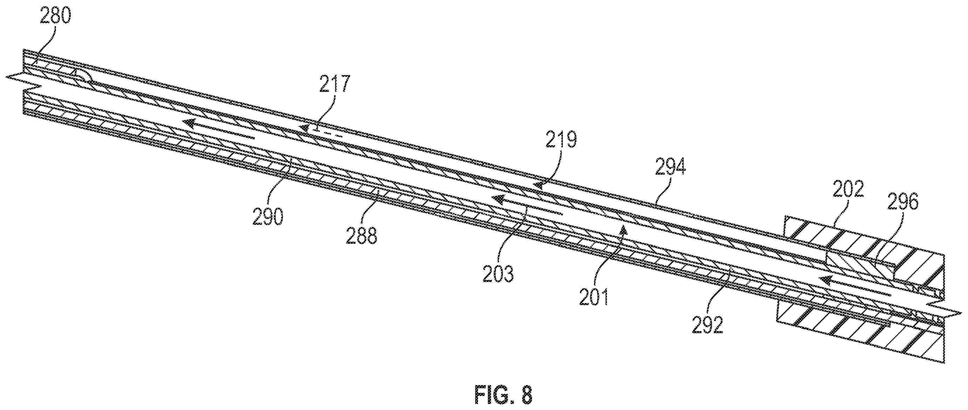

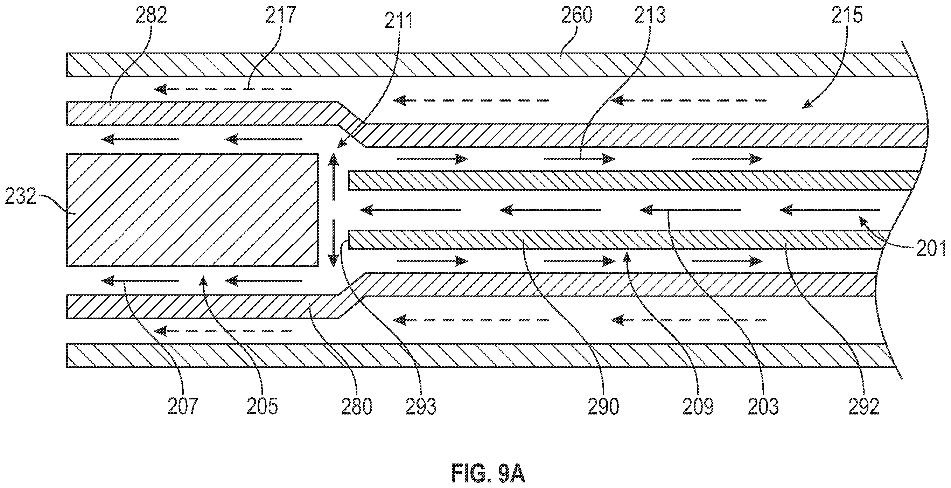

The exemplary delivery apparatus 220 is shown in FIG. 4 with a docking device 232 at least partially deployed from a distal end of the delivery apparatus 220 (e.g., for illustration purposes). In some examples, the docking device 232 can be the docking device 100 described above with reference to FIG. 3. The delivery apparatus 220 can include a handle assembly 200 and an outer shaft (e.g., delivery catheter) 260 extending distally from the handle assembly 200. The handle assembly 200 can include a handle 222 and a hub assembly 230 extending from a proximal end of the handle 222. A more detailed view of the hub assembly 230 is shown in FIG. 5, as described further below. Additionally, FIGS. 6 and 7 are cross-sectional views illustrating examples of internal components of the hub assembly 230 and a flow of flush fluid through the internal components of the hub assembly 230. FIGS. 8 and 9A illustrate the flow of flush fluid through internal components of the delivery apparatus 220, distal to the handle assembly 200, including a distal end portion of the delivery apparatus 220 (FIGS. 9A-9B) and a portion of the delivery apparatus 220 arranged between the distal end portion and the hub assembly 230 (FIG. 8).

As shown in FIG. 4, the handle assembly 200 can include a handle 222 including one or more knobs, buttons, wheels, or the like. For example, as shown in FIG. 4, the handle 222 can include knobs 224 and 226 which can be configured to control flexing of the delivery system (e.g., the outer shaft 260). The outer shaft 260 extends distally from the handle 222 while the hub assembly 230 extends proximally from the handle 222. Further details on delivery systems and apparatuses, such as delivery apparatus 220, that are configured to deliver a docking device to a target implantation site can be found in U.S. Patent Publication Nos. US2018/0318079, US2018/0263764, and US2018/0177594.

During delivery of some docking devices at the target implantation site, there is potential for the docking device to catch, get stuck on, and/or be obstructed by portions of the native anatomy, such as on the heart wall, trabeculae, native leaflets, chordae tendineae, or the like. This can be due to multiple factors such as friction forces between the docking device and the native anatomy, a distal end or tip of the docking device becoming caught in the trabeculae and/or chordae of the native anatomy, size differentials between the inner diameter of functional turns of the docking device and the outer diameter of native leaflets, and the like.

Some docking devices can have a woven or braided texture and/or covering on the outer surface of the docking device to increase friction (e.g., to enhance a retention force between the docking device and a prosthetic valve deployed and implanted therein, as described above with reference to FIG. 2B). However, this friction can create difficulties when advancing the docking device around and/or through that native anatomy (e.g., such as increased resistance to movement of the docking device around the native anatomy and/or sticking or catching of the docking device on the native anatomy).

Once the docking device runs into an obstacle, such as the native leaflets, chordae, and/or trabeculae, the doctor, surgeon, or other medical professional or user may need to retract the docking device into the delivery apparatus (e.g., delivery apparatus 220) and try again to deploy the docking device. However, this method can cause damage to the native tissue due to textures or braids existing on the docking device rubbing against and/or catching portions of tissue and dragging it back into the delivery apparatus, which can potentially damage or clog the delivery apparatus. Further, this can increase the amount of time for the deployment and implantation procedure.

To address these challenges, the docking device (e.g., docking device 232 of FIG. 4) can be configured to have a lubricous outer surface (e.g., on an entirety of the docking device or certain portions of the docking device, such as the functional turns) during delivery and implantation at the native anatomy and a higher-friction outer surface, at least at the functional coils/turns, after implantation and during subsequent deployment of a prosthetic valve therein.

In some examples, this can be accomplished with a removable lubricous sleeve or sheath that can be placed over the docking device during delivery, and which is retractable from the docking device after the docking device is in a desired position/location at the implantation site. In some examples, a lubricous or low-friction sleeve/sheath can be incorporated into a delivery apparatus, such as the delivery apparatus 220 of FIG. 4.

For example, the delivery apparatus 220 can include a pusher shaft 290 (FIGS. 4-11) and a sleeve shaft 280 (FIGS. 5-11) which are coaxially located within the outer shaft 260 and each have portions that extend into the handle assembly 200. The pusher shaft 290 can be configured to deploy the docking device 232 from inside a distal end portion of the outer shaft 260, upon reaching the target implantation site, and the sleeve shaft 280 can be configured to cover the docking device 232 while inside the delivery apparatus 220 and while being positioned at the target implantation site (FIG. 11). Further, the delivery apparatus 220 can be configured to adjust an axial position of the sleeve shaft 280 to remove a sleeve portion (e.g., distal section) of the sleeve shaft 280 from the docking device 232, after implantation at the target implantation site, as explained further below. FIGS. 10 and 11 are perspective views showing the exemplary docking device 232 deployed from the outer shaft 260 of the delivery apparatus 220, covered by a distal (or sleeve) portion 282 of the sleeve shaft 280 (FIG. 10), and the exemplary docking device 232 after the sleeve shaft 280 has been retracted back into the outer shaft 260 (FIG. 11).

As shown in FIGS. 4 and 11, during delivery, the docking device 232 can be coupled to the delivery apparatus 220 via a release suture (or other retrieval line comprising a string, yarn, or other material that can be configured to be tied around the docking device and cut for removal) 236 that can extend through the pusher shaft 290. As explained further below with reference to FIG. 5, the release suture 236 can extend through the delivery apparatus 220, through an inner lumen of the pusher shaft 290, to a suture lock assembly 206 of the delivery apparatus 220.

As shown in FIGS. 4 and 5, the hub assembly 230 can include the suture lock assembly (e.g., suture lock) 206 and a sleeve handle attached thereto. A first example of a sleeve handle 234 is shown in FIG. 4 and a second example of a sleeve handle 208 is shown in FIG. 5. The hub assembly 230 can be configured to control the pusher shaft 290 and the sleeve shaft 280 of the delivery apparatus 220, together (e.g., move them axially together), while the sleeve handle (sleeve handle 234 in FIG. 4 and sleeve handle 208 in FIG. 5) can control an axial position of the sleeve shaft 280 relative to the pusher shaft 290. In this way, operation of the various components of the handle assembly 200 can actuate and control operation of the components arranged within the outer shaft 260. In some examples, as shown in FIGS. 4 and 5, the hub assembly 230 can be coupled to the handle 222 via a connector 240.

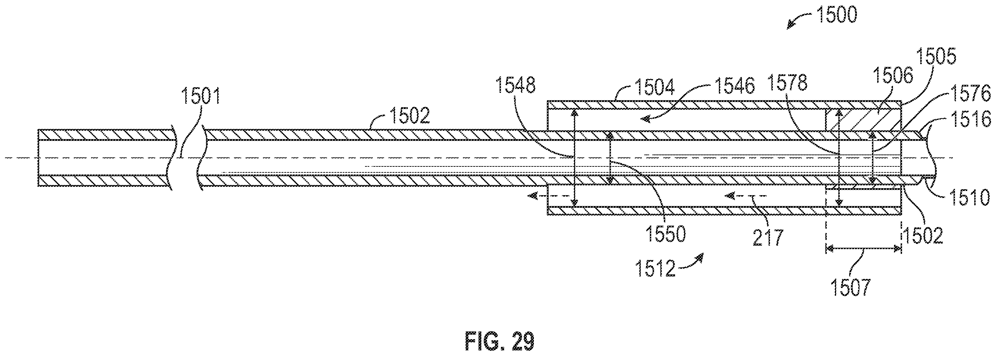

Further details on a suture lock assembly and a pusher shaft and sleeve shaft assembly for a delivery apparatus for a docking device are described in International Patent Application No. PCT/US20/36577. Additionally, further examples of a pusher shaft for a delivery apparatus, such as delivery apparatus 220, are described further below with reference to FIGS. 29-43.

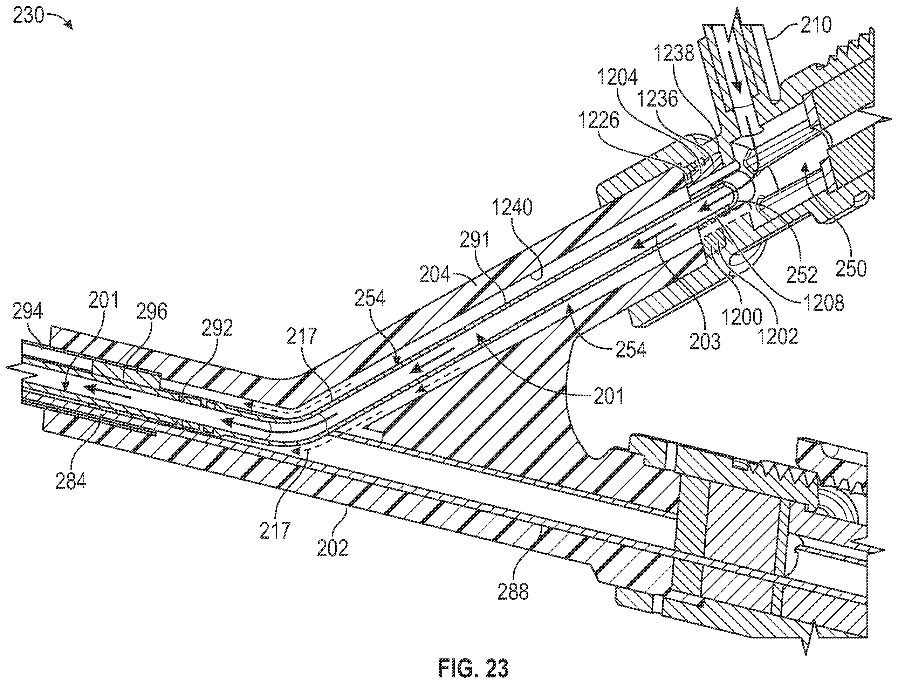

As shown in FIGS. 4-7 and described further below, the handle assembly 200 can further include one or more flushing ports to supply flush fluid to one or more lumens arranged within the delivery apparatus 220 (e.g., annular lumens arranged between coaxial components of the delivery apparatus 220) in order to reduce potential thrombus formation. One example where the delivery apparatus 220 includes three flushing ports (e.g., flushing ports 210, 216, and 218) is shown in FIGS. 4, 6, and 7. In alternate examples, the delivery apparatus 220 may not include flushing port 216 (e.g., as shown in FIG. 5 and FIG. 23, as described further below).

FIG. 5 shows an example of the hub assembly 230 for the delivery apparatus 220 in more detail. In some examples, as shown in FIG. 5, the hub assembly 230 can comprise a Y-shaped connector (e.g., adaptor) having a straight section (e.g., straight conduit) 202 and at least one branch (e.g., branch conduit) 204 (though, in some examples, it can include more than one branch). In some examples, the suture lock assembly (e.g., suture lock) 206 can be attached to the branch 204 and the sleeve handle (e.g., sleeve actuating handle) 208 can be arranged at a proximal end of the straight section 202.

The hub assembly 230 can be adapted and configured to allow a proximal extension 291 of the pusher shaft 290 (or another, similar pusher shaft) to extend to the suture lock assembly 206 arranged at the end of the branch 204, while a cut portion 288 (which may also be referred to as a proximal portion) of the sleeve shaft 280 extends to the sleeve handle 208, arranged at the end of the straight section 202. With this configuration, a medical professional can execute the deployment of the docking device (e.g., docking device 232 of FIG. 4) by manipulating the position of the handle assembly 200 (e.g., moving it in the axial direction) and also execute retraction of the sleeve shaft 280 (off of and away from the implanted docking device) by pulling back, in the axial direction, on the sleeve handle 208.

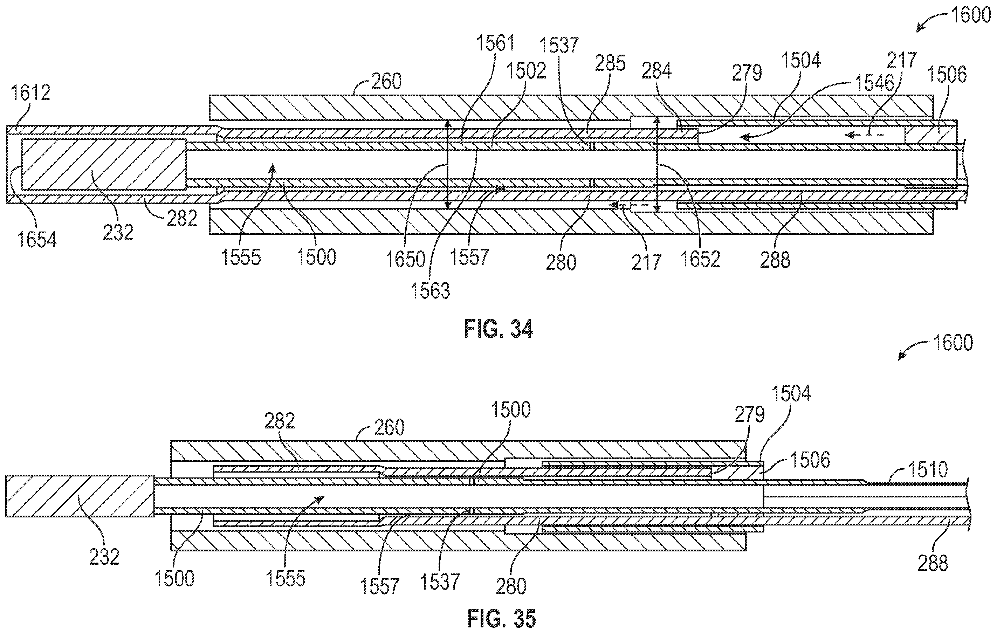

In this way, the sleeve shaft 280 and pusher shaft 290 can be configured to work together such that they can be moved simultaneously together when deploying and positioning the docking device at the native valve (e.g., by moving the entire hub assembly 230 forward and/or backward, in the axial direction), but can also to move independently so the pusher shaft 290 can hold the docking device in position while the sleeve shaft 280 is retracted off of the docking device (e.g., by holding the hub assembly 230 in place relative to the outer shaft 260 of the delivery apparatus 220 and/or other parts of the delivery apparatus 220 and/or docking device while pulling proximally on the sleeve handle 208 to withdraw the sleeve shaft 280). As introduced above and shown in FIGS. 34 and 35, as described further below, the sleeve shaft 280 and the pusher shaft 290 can be coaxial along some, all, or a majority of the delivery apparatus 220 to facilitate this cooperative interaction.

As introduced above and shown in FIGS. 4-7, the handle assembly 200 can include one or more flushing or fluid ports, such as one or more of flushing ports 210, 216, and 218, that are configured to receive fluid and provide the received fluid to selected lumens (e.g., annular spaces) arranged between the axially-extending and coaxial components of the delivery apparatus 220. For example, the configuration of the flushing ports shown in FIGS. 4-7 and the various other flow system examples described in detail herein can enable flushing of and/or a constant flow of fluid through the selected lumens of the delivery apparatus 220 during an implantation procedure, in order to reduce or prevent thrombosis between components, including around the docking device.

For example, as shown in FIG. 9A which is a schematic of a distal end portion of the delivery apparatus 220, various lumens formed between the docking device 232, the pusher shaft 290, the sleeve shaft 280, and the outer shaft 260 are configured to receive fluid during a delivery and implantation procedure.

A first, pusher shaft lumen 201 can be formed within an interior of the pusher shaft (e.g., within an interior of a main tube 292 of the pusher shaft 290). The pusher shaft lumen 201 can receive fluid from a first fluid source, which may be fluidly coupled to a portion of the handle assembly (e.g., the branch 204, as described further below). The flush fluid flow 203 through the pusher shaft lumen 201 can travel along a length of the main tube 292 of the pusher shaft 290, to a distal end 293 of the pusher shaft 290. When the distal end 293 of the pusher shaft 290 is spaced away from a proximal end of the docking device 232, as shown in FIG. 9A, at least a portion of the flush fluid flow 203 can flow into a first portion 205 of a second, sleeve shaft lumen 211, which is arranged between an outer surface of the docking device 232 and an inner surface of the distal section 282 of the sleeve shaft 280, as flush fluid flow 207. Further, in some examples, a portion of the flush fluid flow 203 can also flow into a second portion 209 of the sleeve shaft lumen 211, which is arranged between an outer surface of the pusher shaft 290 and an inner surface of the sleeve shaft 280, as flush fluid flow 213. In this way, the same, first fluid source may provide flush fluid to each of the pusher shaft lumen 201, the first portion 205 of the sleeve shaft lumen 211, and the second portion 209 of the sleeve shaft lumen 211, via the pusher shaft lumen 201.

As also shown in FIG. 9A, a third, delivery shaft lumen 215 can be formed in an annular space formed between an inner surface of the outer shaft 260 and an outer surface of the sleeve shaft 280. The delivery shaft lumen 215 can receive fluid from one or more second fluid sources, which may be fluidly coupled to a portion of the handle assembly (e.g., branch 204 and/or handle 222, as described further below), and/or the first fluid source. Fluid from one or more of these sources can result in flush fluid flow 217 flowing through the delivery shaft lumen 215, to a distal end of the outer shaft 260.

Providing fluid (e.g.., flush fluid) to the above-described lumens can reduce or prevent thrombosis on and around the docking device 232 and other concentric parts of the delivery apparatus 220 during deployment of the docking device 232 from the delivery apparatus 220 and implantation of the docking device 232 at a target implantation site.

FIGS. 4-7 show different examples of an arrangement of possible fluid (e.g., flushing) ports configured to provide flush fluid to the lumens described above with reference to FIG. 9A. Additionally, FIG. 8 illustrates a flow of the flush fluid through a portion of the delivery apparatus 220 arranged between the hub assembly 230 (FIGS. 4-7) and the distal end portion of the delivery system (FIG. 9A).

In a first example of a flushing port arrangement, the handle assembly 200 can include two flushing ports (which can also be referred to herein as fluid ports) arranged on the branch 204 (which may be referred to as a suture lock branch) of the hub assembly 230, one of which provides the flush fluid flow 203 to the pusher shaft lumen 201 and another of which provides the flush fluid flow 217 to the delivery shaft lumen 215. For example, the two flushing ports on the branch 204 can include a first flushing port 210 and a second flushing port 216, the first flushing port 210 arranged proximal to the second flushing port 216 on the branch 204 (FIGS. 6 and 7). In some examples, the location of the second flushing port 216 on the branch 204 can be closer to or farther away from the first flushing port 210 than shown in FIGS. 6 and 7. Additionally or alternatively, in some examples, the first flushing port 210 can be arranged at a more proximal position along the branch 204, such as at a free end of the branch 204 and/or coupled with the suture lock assembly 206.

As shown in FIGS. 5-7, the first flushing port 210 has an inner flow lumen that is fluidly connected to an internal cavity 250 in the branch 204. An open, proximal end 252 of the proximal extension 291 of the pusher shaft 290 can be fluidly coupled to and/or arranged within the internal cavity 250 (as shown in FIGS. 5-7). The proximal extension 291 routes through the branch 204, into the straight section 202 of the hub assembly 230, and connects to the main tube 292 of the pusher shaft 290 (FIGS. 6 and 7). Thus, the pusher shaft lumen 201 is formed by and within the main tube 292 and the proximal extension 291. As such, the flush fluid flow 203 from the first flushing port 210 enters the pusher shaft lumen 201 at the proximal end 252 of the proximal extension 291 and continues into and through an entirety of the main tube 292 of the pusher shaft 290, to the distal end 293 (as shown in FIG. 9A).

The second flushing port 216 has an inner flow lumen that is fluidly connected to an elongate space or cavity 254 (which may be annular along at least a portion of the cavity) surrounding an exterior of the proximal extension 291 within the branch 204 and extending into the straight section 202, in a space between an inner surface of the cut portion 288 of the proximal section 284 of the sleeve shaft 280 and the proximal extension 291 (FIGS. 6 and 7). Thus, the flush fluid flow 217 from the second flushing port 216 can enter the cavity 254 and flow through the cavity 254, around the proximal extension 291, and into an annular cavity 219 (FIGS. 6 and 8). The flush fluid flow 217 can flow through the annular cavity 219 and exit a distal end of a shell 294 of the pusher shaft 290, as shown in FIGS. 34 and 35 (described further below), to enter the delivery shaft lumen 215.

In some examples, as shown in FIGS. 4 and 6, the delivery shaft lumen 215 can be provided with additional flush fluid from a third flushing port 218 (in addition to the fluid from the second flushing port 216) fluidly coupled to the annular cavity 219, downstream of (e.g., distal to) a plug 296 of the pusher shaft 290 (further details on components of the pusher shaft 290, including the plug 296 and shell 294 are described below with reference to FIGS. 29-33). In this way, in some examples, supplemental flush fluid 221 can be combined with the flush fluid flow 217 (FIG. 6) and supplied to the delivery shaft lumen 215.

In some examples, as shown in FIGS. 4 and 6, the third flushing port 218 can be arranged on a portion of the handle 222. In alternate examples, the third flushing port 218 can be arranged at a more distal location on the handle than shown in FIGS. 4 and 6. In some examples, the third flushing port 218 may not be used during an implantation procedure, but instead, may only be used for flushing the delivery shaft lumen 215 prior to insertion of the delivery apparatus 220 into a patient.

In some examples, the delivery apparatus 220 may not include the third flushing port 218.

Various examples of the hub assembly 230, including the first example of the flushing port arrangement described above, can include a gasket 223 located within branch 204, between the two flushing ports on the branch 204, to create separate and distinct fluid flow lumens fed by the two flushing ports on the branch 204 (e.g., first flushing port 210 and second flushing port 216, as shown in FIGS. 5-7). For example, the gasket 223 can be configured as a disc with a single (e.g., central in some examples) hole configured to tightly receive the proximal extension 291 therein. The gasket 223 may not include any additional holes and can be further configured to provide a seal between the internal cavity 250 and the cavity 254. As a result, all of the flush fluid flow 203 entering the internal cavity 250 from the first flushing port 210 can enter the pusher shaft lumen 201, without entering the cavity 254 and flowing to the delivery shaft lumen 215. Likewise, all of the flush fluid flow 217 entering the cavity 254 from the second flushing port 216 can enter the annular cavity 219 and the delivery shaft lumen 215.

In a second example of a flushing port arrangement, the handle assembly 200 can include two flushing ports arranged on the branch 204 (which may be referred to as a suture lock branch) of the hub assembly 230, one of which provides the flush fluid flow 203 to the pusher shaft lumen 201 and another of which provides the flush fluid flow 217 to the delivery shaft lumen 215. However, in the second example, the flushing port providing the flush fluid flow 203 to the pusher shaft lumen 201 can be arranged on a proximal end of the branch 204, at an end of the suture lock assembly 206.

Flushing port arrangement examples possessing multiple flushing ports, such as the first and second examples described above, can be supplied with flush fluid independently (e.g., with two separate fluid supply sources) or together with a common fluid supply source. For example, in some examples, each flushing port (e.g., first flushing port 210 and second flushing port 216) can be supplied with flush fluid from two separate infusion pumps (one fluidly coupled to each of the flushing ports) or another set of fluid sources. In alternate examples, a single infusion device (e.g., pump) 225 can be connected to multiple flushing ports, such as through a Y-connector 227 that connects a single fluid line to multiple flushing ports, as shown in FIG. 6.

In some instances, it may be desirable to provide a constant flow of fluid (e.g., flush fluid such as a heparinized saline solution) through each of the pusher shaft lumen 201 and the delivery shaft lumen 215 in order to avoid stagnation of fluid within the delivery apparatus 220, which may in some cases lead to thrombosis. Thrombi may, in some instances, contribute to patient complications if they are dislodged during implantation of a docking device. Additionally, thrombi can increase a force experienced during removal of the distal portion of the sleeve shaft 280 from the docking device due to causing increased friction between the sleeve shaft 280 and the docking device.

Thus, in the case of two dedicated flushing or fluid ports (e.g., flushing port 210 and flushing port 216, as described above) it may be desirable to individually control the flow of fluid into each of the two fluid ports to ensure relatively constant or constant flow through the pusher shaft lumen 201 and the delivery shaft lumen 215. As one example, two separate infusion devices (e.g., pumps) can be used to provide specified flow rates of flush fluid to the pusher shaft lumen 201 and the delivery shaft lumen 215. However, such configurations may be more complicated to control and increase procedure costs and/or setup times, as opposed to controlling only a single device. If only a single flow supply (e.g., infusion device) is utilized for the two flushing ports, the amount of fluid going into each flushing port is not controlled, but rather dependent on the resistance in each flow lumen (e.g., the pusher shaft lumen 201 and the delivery shaft lumen 215). However, the resistance of each of the pusher shaft lumen 201 and the delivery shaft lumen 215 and the ratio of resistance between each of the pusher shaft lumen 201 and the delivery shaft lumen 215 can vary during a procedure. In some examples, the pusher shaft lumen 201 can have increased resistance relative to the delivery shaft lumen 215. As such, flow from a single fluid source may preferentially flow through the delivery shaft lumen 215, thereby increasing a risk of thrombus formation within the pusher shaft lumen 201 and/or the sleeve shaft lumen 211.

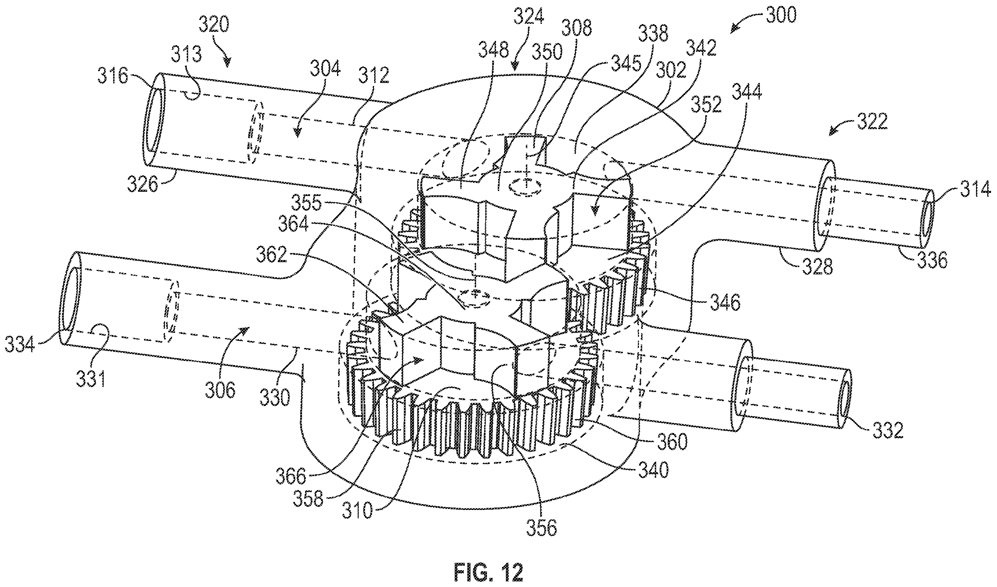

Thus, in some examples, it may be desirable to balance or equalize the flow rate of fluid into each of the two flushing ports (e.g., first flushing port 210 and second flushing port 216) and through each of the respective lumens (e.g., the pusher shaft lumen 201 and the delivery shaft lumen 215) such that target flow rates are achieved in all of the flow lumens of the delivery apparatus that may reduce or prevent thrombus formation. Examples of a flow mechanism configured to provide a consistent flow rate ratio between two or more flow paths (e.g., the first flushing port 210 to the pusher shaft lumen 201 and the second flushing port 216 to the delivery shaft lumen 215) are discussed below with reference to FIGS. 12-22. As a result, a consistent relative flow rate of fluid between the two flushing ports can be achieved, independent of fluctuating resistance in each of the flow paths (e.g., the pusher shaft lumen 201 and the delivery shaft lumen 215).

As used herein, "flow rate ratio" can be defined as the ratio of a first flow rate of fluid through a first flow path to a first flow rate of fluid through a second flow path. Thus, though individual flow rates can change, the ratio of the first flow rate to the second flow rate can be maintained at a constant or consistent ratio, as described further herein.

Turning now to FIGS. 12-22, examples of a mechanical flow mechanism configured to maintain a consistent relative flow rate (or flow rate ratio) between two or more flow paths, independent of varying resistances in the two or more flow paths. In some examples, the flow mechanism examples described below with reference to FIGS. 12-22 can be used to control the flow of fluid into two or more flow lumens in a delivery apparatus for an implantable device, such as the pusher shaft lumen and delivery shaft lumen of the delivery apparatus 220 (as shown in FIGS. 5-9A). In some examples, the flow mechanism examples described below with reference to FIGS. 12-22 can be used to control the flow of fluid through two or more flow paths in an alternate flow system (e.g., such as two or more parallel flow paths in an alternate flow system).

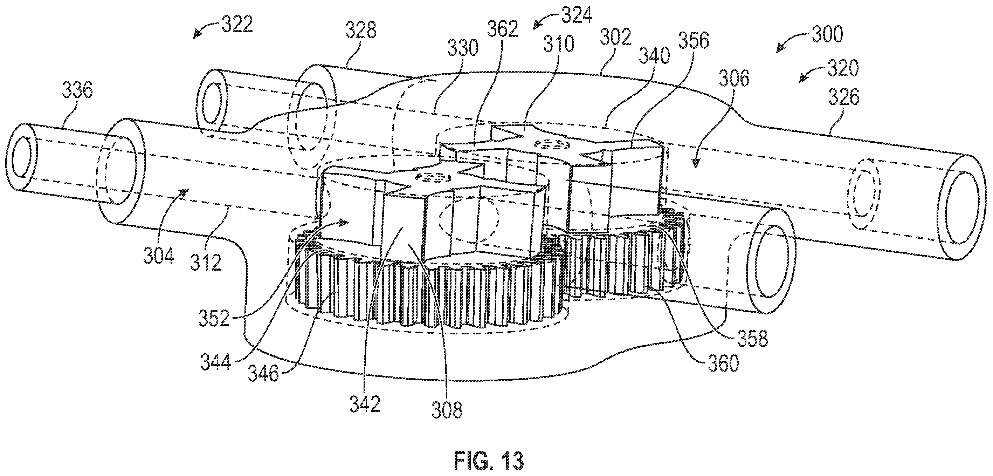

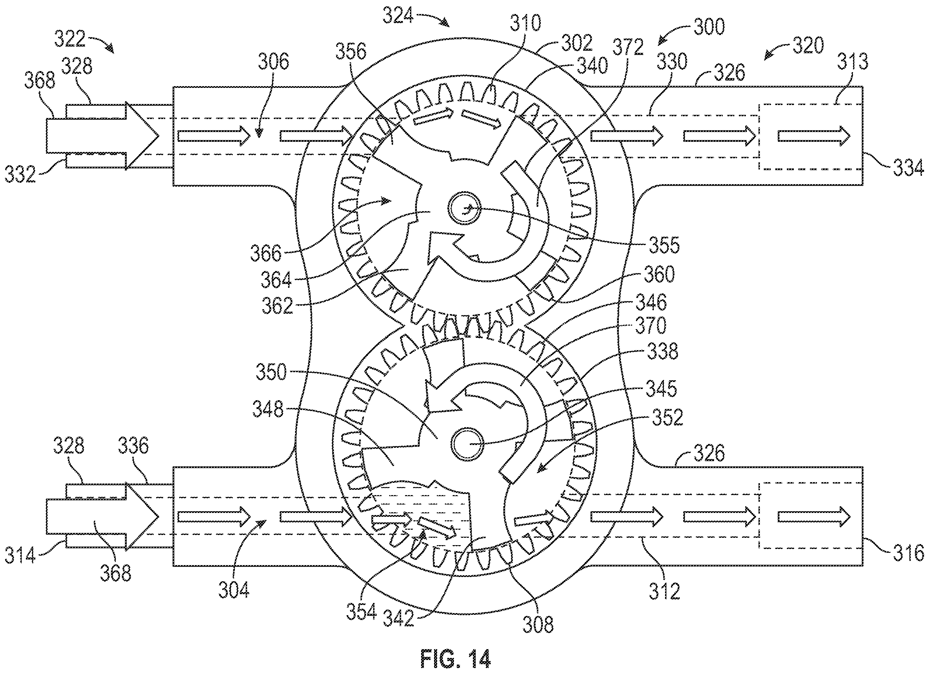

FIGS. 12-14 show an example of a flow mechanism 300 configured to maintain a consistent relative flow rate between two or more flow paths (e.g., a consistent flow rate ratio in two or more flow paths). FIG. 12 shows a top perspective view of the flow mechanism 300, FIG. 13 shows a side perspective view of the flow mechanism 300, and FIG. 14 shows a top view of the flow mechanism 300 which also illustrates a flow of fluid through the flow mechanism 300.

The flow mechanism 300 comprises a housing (e.g., outer housing) 302 and at least two paddle gears disposed within the housing 302. The housing defines at least two flow paths, each flow path corresponding to one of the at least two paddles gears.

For example, as shown in FIGS. 12-14, the housing 302 comprises a first end portion 320, a second end portion 322, and a central portion 324 arranged between the first end portion 320 and the second end portion 322. In some examples, the first end portion 320 can be an outlet end portion (as shown in FIG. 14) including two or more conduits (e.g., outlets or outlet conduits) 326 (two shown in the example of FIGS. 12-14) configured to direct flow away from a corresponding paddle gear and out of the flow mechanism 300. The second end portion 322 can be an inlet end portion including two or more conduits (e.g., inlets or inlet conduits) 328 (two shown in the example of FIGS. 12-14) configured to receive flow from a fluid source and direct flow to the corresponding paddle gear. However, in alternate examples, the conduits 326 can instead be configured as inlets and the conduits 328 can instead be configured as outlets.

The housing 302 of the flow mechanism 300 defines a first flow path 304 and a second flow path 306. The first flow path 304 and the second flow path 306 are configured to receive a fluid and are fluidly isolated from one another (e.g., no flow interaction or mixing occurs between fluid in the first flow path 304 and fluid the second flow path 306).

The first flow path 304 can be fluidly coupled to a first paddle gear 308 disposed within the central portion 324 of the housing 302. The first flow path 304 is defined by a first inner channel 312 formed in the housing 302, between a first flow inlet opening (also referred to as a flow inlet) 314 and a first flow outlet opening (also referred to as a flow outlet) 316. In some examples, the first inner channel 312 can have a relatively constant inner diameter. In some examples, the first inner channel 312 can have a larger diameter (or stepped) portion 313 within the first end portion 320, which connects to the first flow outlet opening 316. As a result, a flow connector or conduit of or coupled to a fluid system can extend into the first flow outlet opening 316 and the larger diameter portion 313 of the first inner channel 312, thereby coupling the flow mechanism 300 to a conduit or flow path of a fluid system configured to receive a metered volume of fluid from the first flow path 304.

Similarly, the second flow path 306 can be fluidly coupled to a second paddle gear 310 disposed within the central portion 324 of the housing 302. The second flow path 306 is defined by a second inner channel 330 formed in the housing 302, between a second flow inlet opening (also referred to as a flow inlet) 332 and a second flow outlet opening (also referred to as a flow outlet) 334. In some examples, the second inner channel 330 can have a relatively constant inner diameter. In some examples, the second inner channel 330 can have a larger diameter (or stepped) portion 331 within the first end portion 320, which connects to the second flow outlet opening 334. As a result, a flow connector or conduit of or coupled to a fluid system can extend into the second flow outlet opening 334 and the larger diameter portion 331 of the second inner channel 330, thereby coupling the flow mechanism 300 to a conduit or flow path of a fluid system configured to receive a metered volume of fluid from the second flow path 306.

In some examples, one or both of the conduits 328 at the second end portion 322 can have a smaller diameter (or stepped) portion 336 that is configured to receive a flow connector or fluid conduit thereon, thereby coupling the flow mechanism 300 to a flow conduit of or coupled to a fluid supply or source.

The housing 302 can further define at least two cavities, each configured to receive a paddle gear therein. For example, as shown in FIGS. 12-14, the housing 302 defines a first cavity 338, the first paddle gear 308 arranged within the first cavity 338, and a second cavity 340, the second paddle gear 310 arranged within the second cavity 340. The first cavity 338 can be fluidly coupled to the first inner channel 312 and the second cavity 340 can be fluidly coupled to the second inner channel 330. Further, as shown in FIGS. 12-14, the first inner channel 312 (and thus the first flow path 304) can extend on either side of the first cavity 338 and the second inner channel 330 (and thus the second flow path 306) can extend on either side of the second cavity 340. In some examples, the first flow path 304 can extend through the first cavity 338 and the second flow path 306 can extend through the second cavity 340.

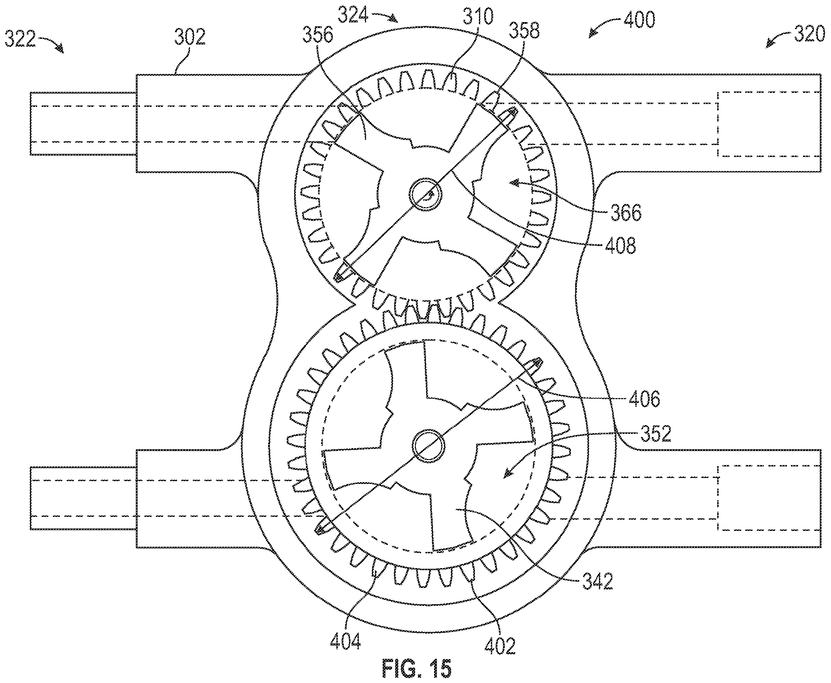

In FIGS. 12-14 (and similarly for the other examples shown in FIGS. 15-22) interior portions of the housing 302, including the first flow path 304, the second flow path 306, the first cavity 338, and the second cavity 340, are illustrated with dashed lines to denote their internal orientation relative to an exterior of the housing 302. It should be noted that, though the first and second paddle gears 308 and 310 are also disposed within an interior of the housing 302, these components are illustrated with solid lines for increased clarity.

The first paddle gear 308 includes a first paddle 342 and a first gear 344 rotatably coupled to one another and configured to rotate about a rotational axis 345. In some examples, as shown in FIGS. 12-14, the first gear 344 includes a plurality of teeth 346 around its circumference. The first paddle 342 can include a plurality of arms 348 extending radially outward from a central portion 350 of the paddle 342. Cavities 352 that are configured to receive fluid and rotate (upon rotation of the first gear 344) are formed between adjacent arms 348 of the first paddle 342 and walls of a portion of the first cavity 338 in which the first paddle 342 is arranged. A volume of the cavities 352 defines a predetermined metered volume of fluid which the first paddle gear 308 is configured to flow through the first flow path 304. The volume of the cavities 352 can be defined by a geometry of the first paddle 342. For example, the volume of the cavities 352 can be increased by increasing a length of the arms 348 (e.g., the length defined in a radial direction relative to the rotational axis 345), increasing a height of the arms 348 (and the first paddle 342, e.g., the height define along a direction parallel to the rotational axis 345) and/or decreasing a width (e.g., the width defined in a circumferential direction) of the arms 348. In this way, a geometry of the first paddle 342 can be selected according to a specified metered volume of fluid (or a specified flow rate of fluid, e.g., volume/time) to provide via the first flow path 304. The volume of fluid 354 in one of the cavities 352 is illustrated in FIG. 14, as described further below.

Similarly, the second paddle gear 310 includes a second paddle 356 and a second gear 358 rotatably coupled to one another and configured to rotate about a rotational axis 355. In some examples, as shown in FIGS. 12-14, the second gear 358 includes a plurality of teeth 360 around its circumference. The second paddle 356 can include a plurality of arms 362 extending radially outward from a central portion 364 of the second paddle 356. Cavities 366 that are configured to receive fluid and rotate (upon rotation of the second gear 358) are formed between adjacent arms 362 of the second paddle 356 and walls of a portion of the second cavity 340 in which the second paddle 356 is arranged. A volume of the cavities 366 defines a predetermined metered volume of fluid (or flow rate) which the second paddle gear 310 is configured to flow through the second flow path 306. The volume of the cavities 366 can be defined by a geometry of the second paddle 356, as described above with reference to the first paddle 342. As such, a geometry of the second paddle 356 can be selected according to a specified metered volume of fluid to provide via the second flow path 306.

In some examples, as shown in FIGS. 12-14, teeth 346 of the first gear 344 can be in meshing engagement with teeth 360 of the second gear 358. As such, the first gear 344 and the second gear 358 can rotate together (e.g., rotation of the first gear 344 can cause rotation of the second gear 358 and vice versa). Thus, as explained further below, the rotation of the first paddle gear 308 and the rotation of the second paddle gear 310 are linked to one another. In some examples, as shown in FIG. 14, the first gear 344 can rotate in a first direction 370 (e.g., counter-clockwise in FIG. 14) and the second gear 358 can rotate in a second direction 372 (e.g., clockwise in FIG. 14), the second direction 372 opposite the first direction 370.

In other examples, an additional toothed gear can be arranged between and in meshing engagement with each of the first gear 344 and the second gear 358, thereby enabling the first gear 344 and the second gear 358 to rotate in a same direction.

FIG. 14 illustrates an exemplary flow of fluid through the flow mechanism 300. In FIG. 14, the flow of a fluid (e.g., a flush fluid such as heparinized saline and/or another fluid configured to reduce thrombus formation) is shown by arrows 368. As shown in FIG. 14, the fluid enters the first inner channel 312 and the second inner channel 330 at the first flow inlet opening 314 and the second flow inlet opening 332, respectively. The fluid shown by arrows 368 then continues through the first flow path 304 and the second flow path to the first cavity 338 and the second cavity 340. The cavities 352 and 366 formed by the respective first paddle 342 and second paddle 356 can then catch the incoming flow of fluid from the inflow ends of the respective first and second flow paths 304 and 306, which causes the first paddle gear 308 and the second paddle gear 310 to rotate.

As an example, as shown in FIG. 14, the flow of fluid can enter one of the cavities 352 formed by the first paddle 342 (e.g., the cavity 352 arranged adjacent to an opening between the first cavity 338 and the inlet end of the first inner channel 312). The volume of fluid 354 within this cavity 352 then travels toward an outlet end of the first inner channel 312, as the cavity 352 rotates due to rotation of the first paddle gear 308. As such, the cavities 352 and 366 can be referred to herein as rotating cavities. When the cavity 352 containing the volume of fluid 354 reaches an opening between the first cavity 338 and the outlet end of the first inner channel 312, the volume of fluid 354 is expelled into the outlet end of the first inner channel 312, toward the first flow outlet opening 316. A similar flow of fluid occurs through the second paddle gear 310, as shown in FIG. 14.

A gear ratio between the first gear 344 and the second gear 358 can determine the respective volume of fluid metered to each of the first flow path 304 and the second flow path 306 (and thus the respective flow rates of fluid). For example, as shown in FIGS. 12-14, the gear ratio can be 1:1, and thus, the volume of fluid metered through the first flow path 304 (e.g., volume of fluid 354 depicted in FIG. 14) and the second flow path 306 can be the same. Similarly, the flow rate of fluid through the first flow path 304 and the second flow path 306 can be the same (and have a flow rate ratio of 1:1).

In this example, a flow rate of fluid through the first flow path 304 and a flow rate of fluid through the second flow path 306, and the flow conduits coupled to and configured to receive metered flow from the first flow path and the second flow path 306 (e.g., via conduits or outlets 326), can be the same or substantially the same. For example, a ratio of the predetermined volume of fluid metered through the first flow path 304 and the predetermined volume of fluid metered through the second flow path 306 (e.g., 1:1 in this case) remains constant during rotation of the first paddle gear 308 and the second paddle gear 310. Thus, even if the separate flow conduits or flow paths coupled to the flow mechanism 300 and configured to receive fluid via the flow mechanism 300 have different resistances, they can each receive a constant flow rate of fluid via the flow mechanism 300.

In other examples, if the first gear 344 and the second gear 358 have different diameters but the geometry of the first paddle 342 and the second paddle 356 remain the same, thereby providing a different gear ratio, the volume of fluid metered through the first flow path 304 and the second flow path 306 would be different. In this way, the geometry of the gears of the paddle gears of the flow mechanism 300 can be varied based on a specified metered volume of fluid to be provided to the different flow paths or conduits to which the flow mechanism 300 is coupled.

An exemplary flow mechanism 400 having paddle gears with different diameter gears is shown in FIG. 15. The flow mechanism 400 can be similar to the flow mechanism 300 of FIGS. 12-14, except a first paddle gear 402 has a first gear 404 with a larger diameter than the second paddle gear 310 and the first paddle gear 308 of the flow mechanism 300.

For example, as shown in FIG. 15, the flow mechanism 400 includes the first paddle gear 402 with the first gear 404 having a first diameter 406 and the second paddle gear 310 with the second gear 358 having a second diameter 408, the first diameter 406 larger than the second diameter 408. A geometry of the first paddle 342 of the first paddle gear 402 and the second paddle 356 of the second paddle gear 310 can be the same. As a result, the gear ratio between the first gear 404 and the second gear 358 can be greater than 1:1 (e.g., 1.2:1, 1.5:1, or the like). As a result, during one full rotation of the second paddle gear 310, the first paddle gear 402 does not complete a full rotation (due to its larger diameter). Thus, in the example of FIG. 15, the first paddle gear 402 can provide a smaller metered volume of fluid in a set time frame than the second paddle gear 310. Said another way, the first paddle gear 402 can provide a smaller flow rate of fluid (e.g., volume/time) than the second paddle gear 310.

As introduced above, a volume of the cavities 352 and 366 formed between the housing 302 and the first and second paddle gears 308 and 310, respectively, can also define the predetermined metered volume of fluid (or flow rate of fluid) through the first flow path 304 and the second flow path 306. Since the volume of the cavities 352 and 366 can be defined by a geometry of the first paddle 342 and the second paddle 356, respectively, changing the geometry of the first paddle 342 and/or the second paddle 356 can change the volume of the cavities 352 and/or 366.

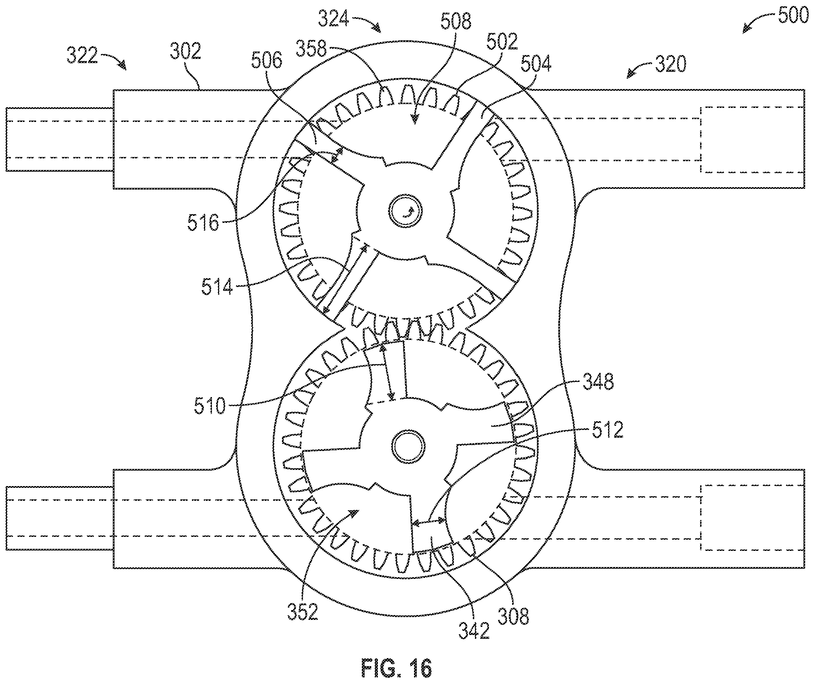

FIG. 16 shows an exemplary flow mechanism 500 having paddle gears with paddles having different geometries, and thus, differently sized cavities between the housing 302 and the corresponding paddles. The flow mechanism 500 can be similar to the flow mechanism 300 of FIGS. 12-14, except a second paddle gear 502 has a second paddle 504 with a geometry that defines cavities 508 having a larger volume than a volume of cavities 352 defined by a geometry of the first paddle 342 of the first paddle gear 308 (e.g., in contrast to the flow mechanism 300 where cavities 352 and 366 can have a same volume).

For example, as shown in FIG. 16, the first paddle 342 has arms 348 with a first length 510 and first width 512 and the second paddle 504 has arms 506 with a second length 514 and second width 516, the second length 514 longer than the first length 510 and the second width 516 shorter than the first width 512. Thus, the cavities 508 defined by the second paddle 504 have a larger volume that the cavities 352 defied by the first paddle 342. As a result, the first paddle gear 308 can provide a smaller metered volume of fluid in a set time frame (e.g., flow rate of fluid) than the second paddle gear 502.

In this way, a geometry of the paddles and/or the gears of two or more paddle gears of a flow mechanism can be selected to provide a variety of specified flow rate ratios between two or more flow paths corresponding to the two or more paddle gears.

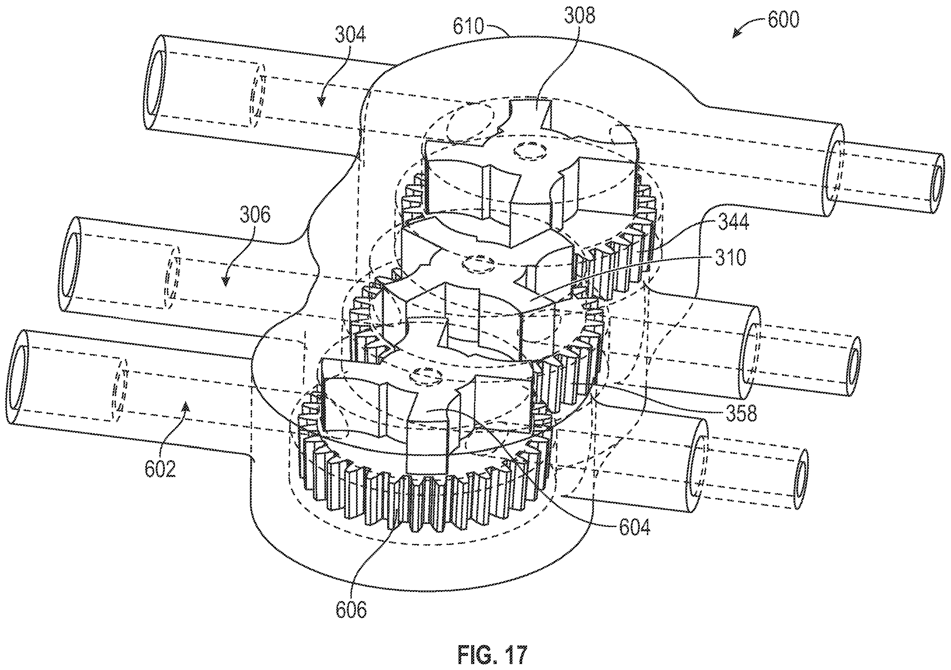

In some examples, as shown in FIG. 17, a flow mechanism 600 (which can be similar to flow mechanism 300), can have more than two flow paths and two corresponding paddle gears, thereby providing a constant ratio of flow rates between the more than two flow paths.

For example, as shown in FIG. 17, the flow mechanism 600 includes a housing 610 defining a first flow path 304, a second flow path 306, and a third flow path 602. The flow mechanism 600 can further include three paddle gears, including a first paddle gear 308 fluidly coupled with the first flow path 304, a second paddle gear 310 fluidly coupled with the second flow path 306, and a third paddle gear 604 fluidly coupled with the third flow path 602. Similar to the flow mechanism 300 of FIGS. 12-14, each paddle gear of the flow mechanism 600 can include a paddle and a gear rotatably coupled to one another.

As shown in FIG. 17, the first paddle gear 308, the second paddle gear 310, and the third paddle gear 604 can be arranged adjacent one another within the housing 610. Further, all of the gears of the first paddle gear 308, the second paddle gear 310, and the third paddle gear 604 can be in meshing engagement with one another. For example, as shown in FIG. 17, the second gear 358 is disposed between and in meshing engagement with the first gear 344 and a third gear 606 of the third paddle gear 604.

In other examples, the flow mechanism 600 and the other flow mechanism described herein can have a different number of paddle gears and corresponding flow paths, such as four, five, or the like.

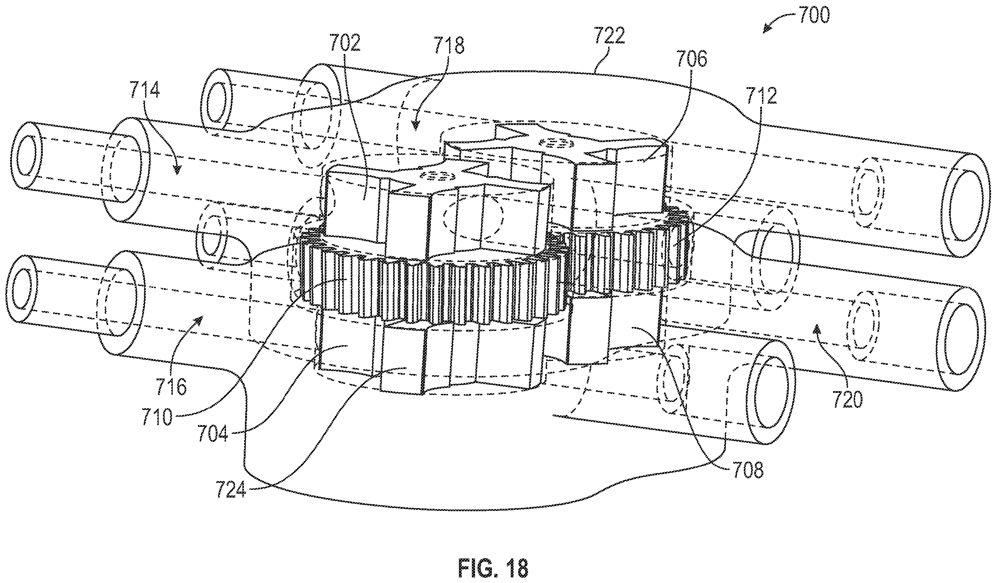

In some examples, additional flow paths can be included in a flow mechanism by including paddles gears with a paddle arranged on either side of a common gear. For example, FIG. 18 shows an exemplary flow mechanism 700 including four flow paths and four paddle gears, each paddle gear including a paddle rotatably coupled to a common rotating member (e.g., a gear) shared by a paddle of another paddle gear. The paddles and gears of the paddle gears of the flow mechanism 700 can be configured similar to the paddles and gears of the first and second paddle gears 308 and 310 of the flow mechanism 300 of FIGS. 12-14, except that two paddles can share and be rotatably coupled to a common gear.

For example, as shown in FIG. 18, the flow mechanism 700 includes a housing 722 and a first paddle gear 702, a second paddle gear 704, a third paddle gear 706, and a fourth paddle gear 708 disposed within the housing 722. Each of the first, second, third, and fourth paddle gears 702, 704, 706, and 708 includes a paddle 724 (which may be configured similar to the first paddle 342 and the second paddle 356 of FIGS. 12-14) rotatably coupled to a common rotatable member that is shared by two paddles 724. In the example of FIG. 18, the common rotatable member is a gear and the flow mechanism 700 includes a first gear 710 and a second gear 712.

The paddle 724 of the first paddle gear 702 is fluidly coupled with a first flow path 714 formed in the housing 722, the paddle 724 of the second paddle gear 704 is fluidly coupled with a second flow path 716 formed in the housing 722, the paddle 724 of the third paddle gear 706 is fluidly coupled with a third flow path 718 formed in the housing 722, and the paddle 724 of the fourth paddle gear 708 is fluidly coupled with a fourth flow path 720 formed in the housing 722. Thus, the flow mechanism 700 can be configured to meter flow to four flow paths (e.g., four separate flow paths or lumens of a flow system coupled to the flow mechanism 700).

As shown in FIG. 18, the paddle 724 of the first paddle gear 702 is arranged on a first side of the first gear 710 and the paddle 724 of the second paddle gear 704 is arranged on an opposite, second side of the first gear 710. The paddles 724 of the first paddle gear 702 and the second paddle gear 704 can each be rotatably coupled to the first gear 710. As a result, the first gear 710 and the paddles 724 of the first paddle gear 702 and the second paddle gear 704 can all rotate together (e.g., as one), thereby providing a same and constant flow rate of fluid through the first flow path 714 and the second flow path 716.

Similarly, the paddle 724 of the third paddle gear 706 is arranged on a first side of the second gear 712 and the paddle 724 of the fourth paddle gear 708 is arranged on an opposite, second side of the second gear 712. The paddles 724 of the third paddle gear 706 and the fourth paddle gear 708 can each be rotatably coupled to the second gear 712. As a result, the second gear 712 and the paddles 724 of the third paddle gear 706 and the fourth paddle gear 708 can all rotate together (e.g., as one), thereby providing a same and constant flow rate of fluid through the third flow path 718 and the fourth flow path 720.

In some examples, as shown in FIG. 18, teeth of the first gear 710 are in meshing engagement with teeth of the adjacent second gear 712, thereby linking rotation of the first and second paddle gears 702 and 704 and the third and fourth paddle gears 706 and 708 to maintain a consistent ratio of flow rates between the four flow paths.

In other examples, the common rotating member disposed between paddles of two paddle gears can be a spacer instead of a gear (e.g., the spacer can be configured as a cylinder or block without teeth), thereby providing a same flow rate of fluid to the two flow paths to which the two paddle gears are fluidly coupled (e.g., for paddles with a same geometry).

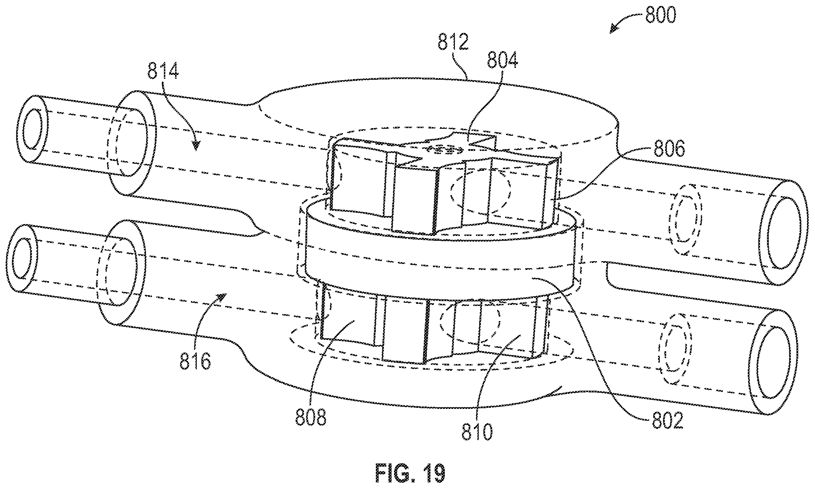

For example, as shown in FIG. 19, a flow mechanism 800 can include a spacer 802 disposed between a first paddle 804 of a first paddle gear 806 and a second paddle 808 of a second paddle gear 810 within a housing 812. The first paddle 804 can be fluidly coupled with a first flow path 814 formed in the housing 812 and the second paddle 808 can be fluidly coupled with a second flow path 816 formed in the housing 812.

Since both the first paddle 804 and the second paddle 808 can be rotatably coupled to the spacer 802, the first paddle 804 and the second paddle 808 can rotate together (e.g., as fluid flows into the first flow path 814 and the second flow path 816) and a same flow rate of fluid can be provided through the first flow path 814 and the second flow path 816 (e.g., when the first paddle 804 and the second paddle 808 have cavities of the same size). In some examples, as described above, a geometry of one of the first paddle 804 and the second paddle 808 can be changed to increase or decrease the volume of fluid, and thus the flow rate of fluid, provided to the corresponding flow path.

In some examples, additional flow paths and paddles, separated by additional spacers, can be added to the flow mechanism 800, thereby creating additional flow paths for coupling to separate conduits or lumens of a flow system (e.g., an additional spacer and paddle can be added to the flow mechanism 800 to create three separate flow paths, all rotating together at a same rotational speed).

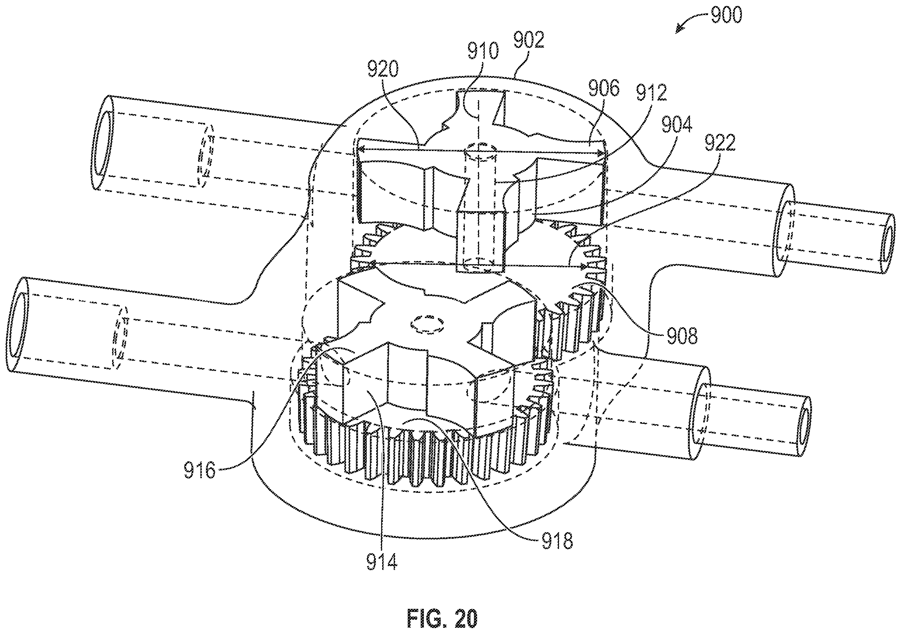

In some examples, in order to accommodate a paddle with an outer diameter that is larger than the outer diameter of the gears to which it is coupled, the paddle of one paddle gear can be arranged at an offset height relative to a paddle of an adjacent paddle gear. For example, as shown in FIG. 20, a flow mechanism 900 can include a first paddle gear 904 including a first paddle 906 rotatably coupled to a first gear 908, with the first paddle 906 spaced away from the first gear 908 in an axial direction relative to a rotational axis 910 of the first paddle gear 904. In some examples, the first paddle 906 can be rotatably coupled to the first gear 908 via a central shaft 912 that is elongated relative to a combined height (measured in the axial direction) of the first paddle 906 and the first gear 908).

The flow mechanism 900 can further include a second paddle gear 914 including a second paddle 916 rotatably coupled to a second gear 918. As shown in FIG. 20, the first gear 908 and the second gear 918 can be arranged adjacent one another and be in meshing engagement with one another. Further, the first paddle 906 can be offset, in the axial direction, from the second paddle 916 (e.g., the first paddle 906 and the second paddle 916 are arranged at different heights). As a result, outer diameters 920 of the first paddle 906 and the second paddle 916 can be larger than outer diameters 922 of their respective gears (not including the teeth of the gear), as shown in FIG. 20.

In this way, a total volume of fluid per time or flow rate of fluid passing through each flow path of a flow mechanism (such as one of the flow mechanisms described above with reference to FIGS. 12-20) can be variable, but a ratio of the flow rates between the flow paths of the flow mechanism can remain the same. This can be due to the linked rotation of the paddles of the paddle gears, as discussed above.

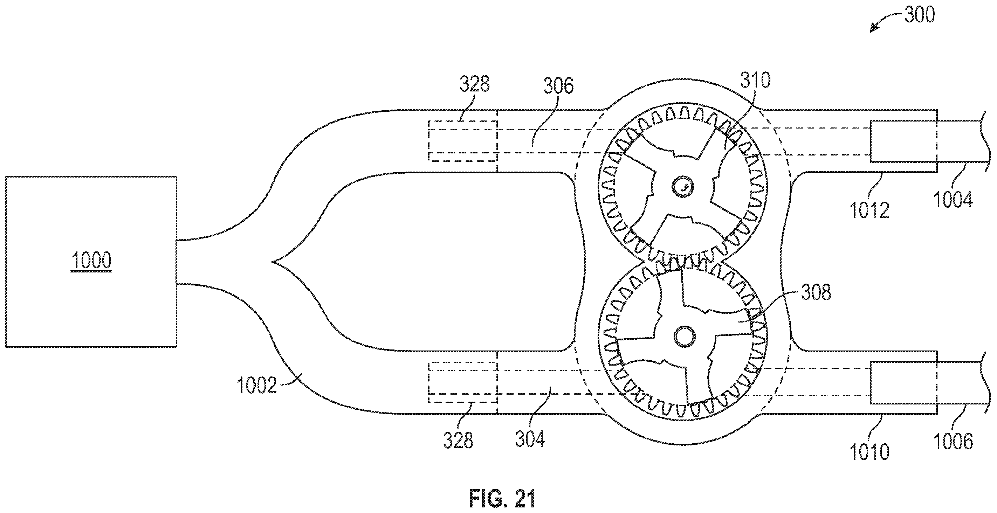

In some examples, any of the flow mechanisms described herein can be used with a single fluid supply, such as a single infusion pump configured to provide a controlled flow rate of fluid to all inlets of the flow mechanism, thereby providing a consistent and predictable flow rate of fluid out of each outlet of the flow mechanism. For example, FIG. 21 shows an exemplary example of a single infusion pump 1000 fluidly coupled to inlet conduits 328 of the flow mechanism 300. For example, as shown in FIG. 21, tubing or a flow conduit 1002 can extend from the single infusion pump 1000 to the inlet conduits 328 to the first flow path 304 and the second flow path 306 of the flow mechanism 300.

Additionally, in some examples, as shown in FIG. 21, a first conduit 1006 can fluidly couple to a first outlet conduit 1010 of the flow mechanism 300 and a second conduit 1004 can fluidly couple to a second outlet conduit 1012 of the flow mechanism 300. As such, metered flow from the first paddle gear 308 can flow into the first conduit 1006 and metered flow from the second paddle gear 310 can flow into the second conduit 1004.

In some examples, the first conduit 1006 can be the first flushing port 210 (or a flow conduit coupled to the first flushing port 210) of the delivery apparatus 220 and the second conduit 1004 can be the second flushing port 216 (or a flow conduit coupled to the second flushing port 216) of the delivery apparatus 220 (FIGS. 4-7).

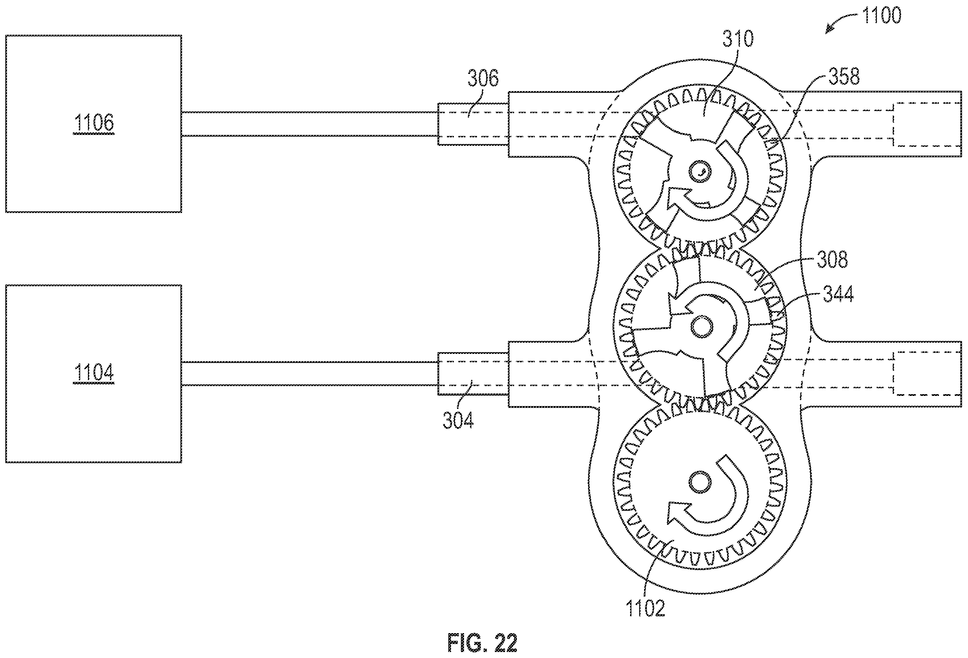

In some examples, as illustrated in FIG. 22, a flow mechanism 1100 (which can be similar to any of the flow mechanisms described herein with reference to FIGS. 12-21) can include a driving member configured to drive rotation of the paddle gears (e.g., first paddle gear 308 and second paddle gear 310 shown by way of example in FIG. 22) at a specified rate. In some examples, as shown in FIG. 22, the driving member can be configured as a toothed gear (e.g., toothed drive gear) 1102 in meshing engagement with at least one gear of the paddle gears of the flow mechanism 1100. For example, as shown in FIG. 22, the toothed gear 1102 is in meshing engagement with the first gear 344 of the first paddle gear 308 and the first gear 344 is in meshing engagement with the second gear 358 of the second paddle gear 310. As a result, driving (e.g., rotating) of the toothed gear 1102 drives rotation of the first gear 344 and the second gear 358.

In some examples, the toothed gear 1102 or other driving member can be part of or coupled to a driving mechanism, such as a motor. In this way, a driving member (e.g., the toothed gear 1102) can drive rotation of the paddle gears of the flow mechanism 1100 at a set rate. In lieu of or in addition to the toothed gear and/or the driving mechanism, a fluid pressure differential can be used to drive rotation of the paddle gears of the flow mechanism at a set rate.

In some examples, as shown in FIG. 22, the flow paths (e.g., flow paths 304 and 306) of the flow mechanism 1100 can each be coupled to a different fluid source (e.g., a fluid reservoir), such as first fluid source 1104 and second fluid source 1106. In other examples, the flow paths of the flow mechanism 1100 can each be coupled to a same fluid source (e.g., fluid reservoir).

In this way, the flow mechanisms described above with reference to FIGS. 12-22 can be configured to maintain a consistent relative flow rate between two or more flow paths. As a result, a flow of fluid through two or more parallel flow paths fluidly coupled with the two or more flow paths of the flow mechanism can be maintained at a specified flow rate ratio.

In some examples, such a flow mechanism can be implemented in a delivery apparatus configured to deliver a docking device, such as delivery apparatus 220 of FIGS. 4-11. For example, a flow mechanism, such as flow mechanism 300 (FIGS. 12-14), flow mechanism 400 (FIG. 15), flow mechanism 500 (FIG. 16), flow mechanism 800 (FIG. 19), flow mechanism 900 (FIG. 20), or flow mechanism 1100 (FIG. 22), can be fluidly coupled to the pusher shaft lumen 201 (e.g., via the first flushing port 210) and the delivery shaft lumen 215 (e.g., via the second flushing port 216). As a result, a consistent relative flow rate of fluid can be provided to the pusher shaft lumen 201 and the delivery shaft lumen 215, regardless of fluctuating resistances in and varying resistances between the pusher shaft lumen 201 and the delivery shaft lumen 215.

It should be noted that the different flow mechanism examples described above with reference to FIGS. 12-22 can be combined in any combination with one another to form a flow mechanism configured to provide a consistent and constant relative flow rate between a specified number of flow paths.

Returning to FIGS. 4-7, in a third example of a flushing port arrangement for the delivery apparatus 220, the handle assembly 200 can include a single flushing port arranged on the branch 204 of the hub assembly 230, the single flushing port configured to provide both the flush fluid flow 203 to the pusher shaft lumen 201 and the flush fluid flow 217 to the delivery shaft lumen 215. For example, certain configurations are able to flush all of the lumens described above with reference to FIGS. 4-9A with only one flushing line, such as the first flushing port 210.

In such examples, the single flushing port can provide fluid to the two separate lumens (pusher shaft lumen 201 and delivery shaft lumen 215), by incorporating a flow throttle in the branch 204 including two or more apertures configured to provide fluid from the single flushing port to the isolated pusher shaft lumen 201 and delivery shaft lumen 215. Examples of such a flow throttle are described further below with reference to FIGS. 23-28.

For example, in some examples, the flow throttle can be arranged where the gasket 223 is shown in FIGS. 6 and 7 (e.g., in place of the gasket 223 and with no second flushing port 216), or further downstream of where the gasket 223 is shown (e.g., at a proximal end of the proximal extension 291).

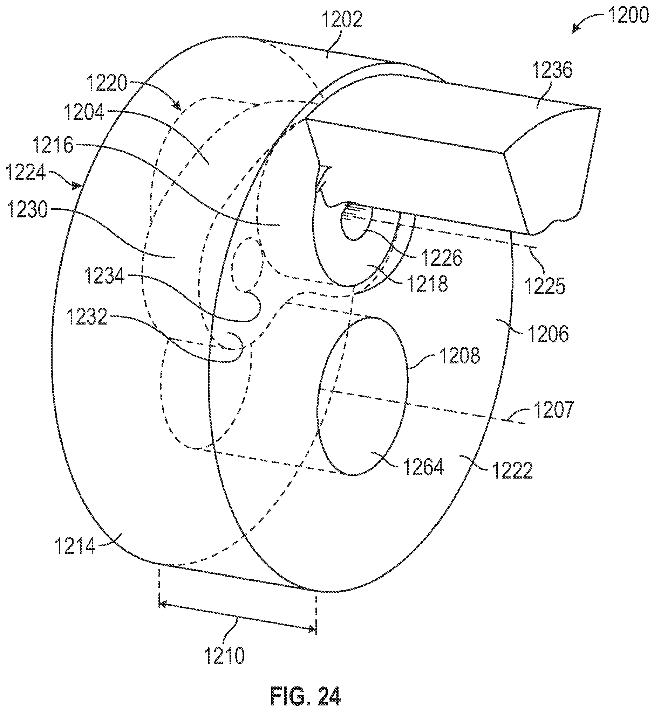

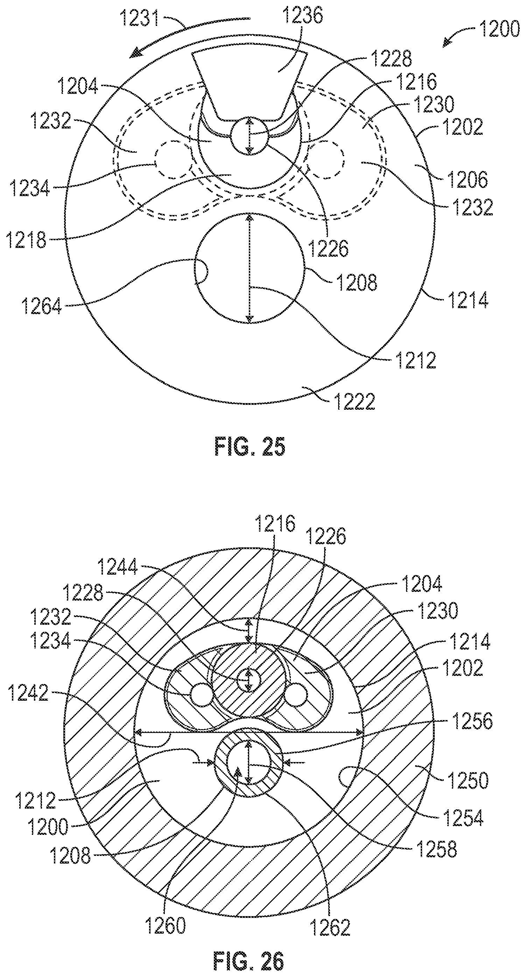

FIGS. 24 and 25 show different views of an example of a flow throttle 1200 configured to control a flow of fluid into two separate (e.g., fluidly isolated) flow lumens (or flow paths) from a single fluid source. FIG. 26 shows an exemplary cross-sectional view of the flow throttle 1200 disposed in a larger of the two flow lumens and sealed around a smaller of the two flow lumens. An exemplary arrangement of the flow throttle 1200 in the hub assembly 230 of the delivery apparatus 220 is shown in FIG. 23.

Turning first to FIGS. 24 and 25, a perspective view (FIG. 24) and an end view (FIG. 25) of the flow throttle 1200 are shown. The flow throttle 1200 can comprise a compressible sealing member 1202 and a rigid substrate 1204.

In some examples, as described further below, the rigid substrate 1204 can be at least partially embedded within the compressible sealing member 1202. In some examples, the compressible sealing member 1202 is overmolded onto and/or around a portion of the rigid substrate 1204.

As shown in FIGS. 24 and 25, portions of the rigid substrate 1204 (e.g., the second portion 1230 and portions of the first portion 1216) that are arranged within an interior of the compressible sealing member 1202 are illustrated with dashed lines to indicate their internal arrangement. In FIG. 26 (as described further below), a cross-sectional view of the flow throttle 1200, taken along a midpoint of a length 1210 of the compressible sealing member 1202, is shown disposed in a larger flow lumen. Thus, in this view, the rigid substrate 1204 (including the first portion 1216 and the second portion 1230) is illustrated with solid lines.

The compressible sealing member 1202 can comprise a compressible material that is configured to compress or change shape under pressure. In some examples, the compressible material of the compressible sealing member 1202 is silicone. In other examples, the compressible material of the compressible sealing member 1202 is another compressible material, such as another compressible polymeric material (e.g., neoprene, fluorocarbon rubber, or the like).

The compressible sealing member 1202 can comprise a body 1206 defining a first aperture 1208 that extends through the length 1210 of the compressible sealing member 1202 (FIG. 24). For example, the first aperture 1208 can be configured as an elongate aperture that extends through an entire length 1210 of the compressible sealing member 1202. The length 1210 can be in a direction parallel to an axial direction, the axial direction relative to a first central longitudinal axis 1207 of the first aperture 1208.

In some examples, when the flow throttle 1200 is implanted in a flow system, the length 1210 can be arranged in a direction parallel to a direction of flow through parallel flow lumens of the flow system.

The first aperture 1208 of the compressible sealing member 1202 can have a first diameter 1212 (FIG. 25). The first aperture 1208 can be spaced away from an outer surface 1214 (and outer perimeter) of the compressible sealing member 1202. In some examples, the first aperture 1208 can be spaced away from the outer surface 1214 around its entire circumference.

In some examples, as shown in FIGS. 24-26, the compressible sealing member 1202 is cylindrical and the outer surface 1214 is curved. In other examples, the compressible sealing member 1202 can have a different shape, such as oval, square, rectangular, or the like. The shape of the compressible sealing member 1202 can be selected based on a specified shape of a flow conduit or flow lumen within which the compressible sealing member 1202 is to be disposed.

The rigid substrate 1204 can comprise a relatively rigid material that is more rigid than the material of the compressible sealing member 1202. For example, the rigid substrate 1204 can comprise a biocompatible, hard plastic or metal material. In other examples, the rigid substrate 1204 can comprise another incompressible material that is configured to retain its shape (e.g., not compress) under pressure. As described further below, in some examples, the rigid substrate 1204 can provide structure to the compressible sealing member 1202.

As shown in FIGS. 24 and 25, the rigid substrate 1204 can comprise a first portion 1216 embedded within the compressible sealing member 1202 and extending through the length 1210 of the compressible sealing member 1202. The first portion 1216 can have a first face 1218 and a second face 1220 arranged on either end of the first portion 1216. The first face 1218 and the second face 1220 can be arranged normal to a direction parallel with the length 1210 and can be disposed at an exterior of the compressible sealing member 1202.

For example, in some examples, the first face 1218 of the first portion 1216 of the rigid substrate 1204 can be arranged at and flush with a first face 1222 of the compressible sealing member 1202 (FIGS. 24 and 25). In other examples, the first face 1218 of the first portion 1216 can extend outward from and past the first face 1222 of the compressible sealing member 1202.

Further, in some examples, the second face 1220 of the first portion 1216 of the rigid substrate 1204 can be arranged at and flush with a second face 1224 of the compressible sealing member 1202 (FIG. 24). In other examples, the second face 1220 of the first portion 1216 can extend outward from and past the second face 1224 of the compressible sealing member 1202.

The first portion 1216 of the rigid substrate 1204 can define a second aperture 1226 with a second diameter 1228 (FIG. 25). The second aperture 1226 can extend through a length of the first portion 1216, which in some examples, can be the same as the length 1210 of the compressible sealing member.

In some examples, as shown in FIG. 25, the second diameter 1228 of the second aperture 1226 can be smaller than the first diameter 1212 of the first aperture 1208. **In** other examples, the first diameter 1212 and the second diameter 1228 can be the same or the first diameter 1212 can be smaller than the second diameter 1228.

In some examples, as shown in FIGS. 24-26, the first aperture 1208 and the second aperture 1226 are radially offset and/or spaced apart from one another. For example, the first aperture 1208 can have a first central longitudinal axis 1207 and the second aperture 1226 can have a second central longitudinal axis 1225 (FIG. 24). The first central longitudinal axis 1207 and the second central longitudinal axis 1225 can be offset from one another (e.g., not overlapping).

The rigid substrate 1204 can further comprise a second portion 1230 embedded within the compressible sealing member 1202. The second portion 1230 can extend outward from the first portion 1216. As shown in FIGS. 24-26, the second portion 1230 can extend circumferentially outward from the first portion 1216 and encircle at least a portion of the first aperture 1208.

In some examples, the second portion 1230 surrounds the first portion 1216 and extends circumferentially outward from either side of the first portion 1216. For example, in some examples, the second portion 1230 can comprise extension portions or wings 1232 extending from either side of the first portion 1216 (FIGS. 24 and 25). As shown in FIGS. 24 and 25, the wings 1232 extend circumferentially outward (e.g., in a circumferential direction 1231 illustrated in FIG. 25) from the first portion 1216 and at least partially encircle the first aperture 1208 (e.g., about 100° to about 170° around a circumference of the first aperture 1208).

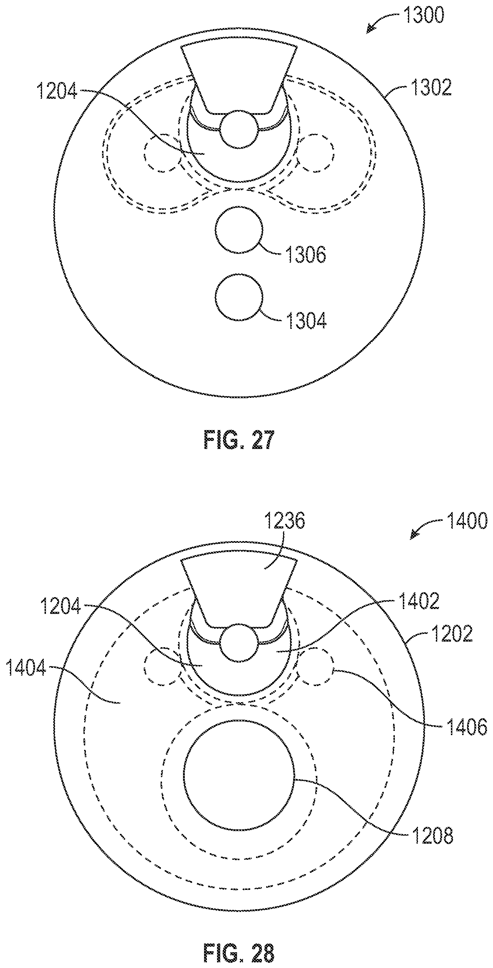

In other examples, the second portion 1230 can include extension portions or wings that extend further around and encircle a greater portion of the first aperture 1208, such as encircling about 180° to about 360° around the circumference of the first aperture 1208. An example of such an arrangement is shown in FIG. 28, as described further below.

In some examples, each wing 1232 can include an aperture 1234 defined there. The apertures 1234 can increase bonding between the compressible sealing member 1202 and the rigid substrate 1204. For example, during forming the compressible sealing member 1202 around the rigid substrate 1204 (e.g., during overmolding), the material of the compressible sealing member 1202 can enter the apertures 1234, thereby increasing contact between the compressible sealing member 1202 and the rigid substrate and holding the first portion 1216 and the second portion 1230 of the rigid substrate 1204 firmly in place within the compressible sealing member 1202.

For example, the geometry of the wings 1232 and/or the apertures 1234 can be configured to maintain the rigid substrate 1204 in place within the compressible sealing member 1202. In other examples, the wings 1232 can include additional apertures 1234 than those shown in FIGS. 24 and 25 (e.g., in examples where the wings 1232 extend further around the first aperture 1208, each wing 1232 can include more than one aperture 1234 and/or more elongate or wider apertures 1234).

In other examples, the rigid substrate 1204 may not have the wings 1232 and/or the entire second portion 1230.

The rigid substrate 1204 can further comprise a third portion or extension member 1236 extending axially outward from the first portion 1216, on one side of the compressible sealing member 1202. As shown in FIG. 24, the extension member 1236 extends axially outward from the first face 1218 of the first portion 1216 and is arranged exterior to the compressible sealing member 1202.

In some examples, the first portion 1216, the second portion 1230, and the extension member of the rigid substrate 1204 are formed (e.g., molded) as one piece.

The extension member 1236 can be configured to act as a "key" that can be received in a receiving member (e.g., recess) of a flow system in order to hold the flow throttle 1200 in place (e.g., hold the flow throttle in a specified circumferential orientation). In this way, in some examples, the extension member 1236 can ensure a specified alignment within the flow system is achieved during assembly.

As shown in FIGS. 24 and 25, the extension member 1236 can be elongated and have a trapezoid-like cross-section, but with two curved edges. In other examples, the extension member 1236 can have a different shape, such as having a cross-section with a square, triangular, or rectangular shape. In this way, the extension member 1236 can have a specified shape that is configured to mate with a correspondingly shaped recess in a flow system in which the flow throttle 1200 is to be disposed.

In other examples, the rigid substrate 1204 can include multiple extension members 1236. In still other examples, the rigid substrate 1204 may not include any extension members 1236.

In some examples, as shown in FIGS. 24-26, the compressible sealing member 1202 can extend radially outside of (e.g., past) the first portion 1216 and the second portion 1230 of the rigid substrate 1204, such that the material of the compressible sealing member 1202 forms a continuous outer surface (e.g., outer surface 1214) around the flow throttle 1200. As a result, radial compression of the flow throttle 1200 can be possible (e.g., to increase sealing within and around components of a flow system in which the flow throttle 1200 is arranged). An outer diameter 1242 of the compressible sealing member 1202 and/or a radial distance between the outer surface 1214 of the compressible sealing member 1202 and the first portion 1216 and/or second portion 1230 of the rigid substrate 1204 (FIG. 26) can be selected or adjusted based on a specified amount of radial compression of the flow throttle 1200 when positioned within a flow conduit or component of a flow system.

In some examples, the compressible sealing member 1202 can extend axially past the first face 1218 and the second face 1220 of the first portion 1216 of the rigid substrate 1204, thereby allowing for axial compression of the compressible sealing member 1202. In this way, in some examples, the length 1210 of the compressible sealing member 1202 can be longer than an axial length of the first portion 1216.

In one exemplary example, as shown in FIG. 23, the flow throttle 1200 can be disposed within the branch 204 of the hub assembly 230 of the delivery apparatus 220, between the two flushing ports on the branch 204. As shown in FIG. 23, the extension member 1236 can extend into a recess 1238 defined within the branch 204, thereby locking the flow throttle 1200 in place.

As also shown in FIG. 23, the proximal extension 291 of the pusher shaft 290 can extend through and seal with the first aperture 1208 and the cavity 254 can be fluidly coupled with the second aperture 1226. For example, as shown in FIG. 23, the second aperture 1226 can be arranged between and fluidly coupled to each of the cavity 254 and the internal cavity 250, thereby throttling fluid from the first flushing port 210 to the cavity 254 (which can be fluidly coupled with the delivery shaft lumen, as described above).

In other examples, the flow throttle 1200 can be positioned further downstream in the branch 204, with the outer surface 1214 of the compressible sealing member 1202 arranged against (e.g., in face-to-face contact) an inner surface 1240 of the branch 204 (FIG. 23).

In other examples, the flow throttle 1200 can be utilized in other flow systems including two or more isolated flow paths.

FIG. 26 shows another exemplary example of the flow throttle 1200 disposed within an outer conduit 1250 (e.g., such as the conduit of branch 204 in FIG. 23). Specifically, FIG. 26 is a cross-sectional view, taken along a midportion or midpoint of the flow throttle 1200, of the flow throttle 1200 disposed within an outer lumen (e.g., such as the cavity 254 in FIG. 23) defined by an inner surface 1254 of the outer conduit 1250. The flow throttle 1200 can be configured to fluidly isolate an outer lumen of the outer conduit 1250 from an inner lumen of an inner conduit 1256, as described further below.

As shown in FIG. 26, the outer surface 1214 of the flow throttle 1200 (and compressible sealing member 1202) can be in face-to-face contact with the inner surface 1254 of the outer conduit 1250. For example, the outer diameter 1242 of the compressible sealing member 1202 and the flow throttle 1200, within the outer conduit 1250, can be the same as the inner diameter of the outer conduit 1250 (e.g., which can be an outer diameter of the outer lumen).

As shown in FIG. 26, an inner conduit 1256 (e.g., such as the proximal extension 291 of the pusher shaft 290 in FIG. 23) extends through the first aperture 1208 of the compressible sealing member 1202. An inner diameter 1258 of the inner conduit 1256 defines an inner lumen (e.g., inner flow lumen or flow path) 1260. An outer diameter of the inner conduit 1256 can be the same as the first diameter 1212 of the first aperture 1208 when arranged within the first aperture 1208 (as shown in FIG. 26). For example, as shown in FIG. 26, an outer surface 1262 of the inner conduit 1256 can have face-to-face contact with an inner surface 1264 of the compressible sealing member 1202 that defines the first aperture 1208.

In this way, the outer surface 1214 of the compressible sealing member 1202 can seal against the inner surface 1254 of the outer conduit 1250 and the inner surface 1264 of the compressible sealing member 1202 defining the first aperture 1208 can seal against the outer surface 1262 of the inner conduit 1256.

In some examples, the inner lumen 1260 can have a greater resistance (e.g., flow resistance or resistance to flow) than the outer lumen.

As shown in FIG. 26, the second diameter 1228 of the second aperture 1226 in the rigid substrate 1204 is smaller than the outer diameter of the outer lumen (formed between the outer conduit 1250 and the inner conduit 1256). As a result, the smaller second aperture 1226 can limit an amount of fluid that can pass through the flow throttle 1200 into the larger, outer lumen. In this way, flow can be throttled into the larger outer lumen while flow can pass, unthrottled (e.g., unrestricted), into the inner lumen 1260.

Since the second aperture 1226 is formed within the rigid (incompressible) substrate 1204, its size (e.g., second diameter 1228) is not affected by axial and/or radial compression of the compressible sealing member 1202.

In some examples, the second diameter 1228 of the second aperture 1226 can be selected based on a difference in size and/or resistance between the outer lumen and the inner lumen 1260. For example, the second diameter 1228 can be selected such that a difference in resistance between the inner lumen 1260 and the outer lumen is at a level that results in continuous flow through each of the outer lumen and the inner lumen 1260. In some examples, the second diameter 1228 can be selected such that a specified relative flow rate between the inner lumen 1260 and the outer lumen is achieved.

FIG. 27 shows an end view of another example of a flow throttle 1300. In some examples, the flow throttle 1300 is similar to the flow throttle 1200 of FIGS. 24-26. The flow throttle 1300 can comprise a compressible sealing member 1302 and rigid substrate 1204. The compressible sealing member 1302 can be similar to the compressible sealing member 1202 of flow throttle 1200 (FIGS. 24-26), except the compressible sealing member 1302 defines two apertures therein, including first aperture 1304 and second aperture 1306, instead of only one aperture (e.g., first aperture 1208 of flow throttle 1200). As a result, the flow throttle 1300 can be configured to receive two flow conduits, one through each of the first aperture 1304 and the second aperture 1306, thereby isolating the two flow conduits from one another.

In some examples, a spacing between the first aperture 1304 and the second aperture 1306 and/or a spacing of the first aperture 1304 and the second aperture 1306 within the compressible sealing member 1302 can be adjusted based on a configuration of the flow system in which it is intended to be disposed.

In other examples, the rigid substrate 1204 can have more than one second aperture 1226 (e.g., two, three, or the like) for additional flow lumens.

In other examples, a flow throttle can include multiple rigid substrates 1204 spaced apart from one another within the compressible sealing member, thereby accommodating additional flow lumens.

FIG. 28 shows an end view of another example of a flow throttle 1400 comprising a compressible sealing member 1202 and rigid substrate 1402. In some examples, the flow throttle 1400 can be similar to the flow throttle 1200 of FIGS. 24-26, except the rigid substrate 1402 includes a second portion 1404 that encircles and extends around an entire circumference of the first aperture 1208. The second portion 1404 can include one or more apertures 1406 (two shown in FIG. 28, although more or less than two apertures are also possible). In this way, the second portion 1404 of the rigid substrate 1402 can be configured as a ring entirely covered in the compressible material of the compressible sealing member 1202.

Turning now to FIGS. 29-33, an example of a pusher shaft 1500 for a delivery apparatus configured to deliver a docking device (such as one of the docking devices described herein) is shown. For example, the pusher shaft 1500 can be the pusher shaft 290 included in the delivery apparatus 220, as shown in FIGS. 4-11.

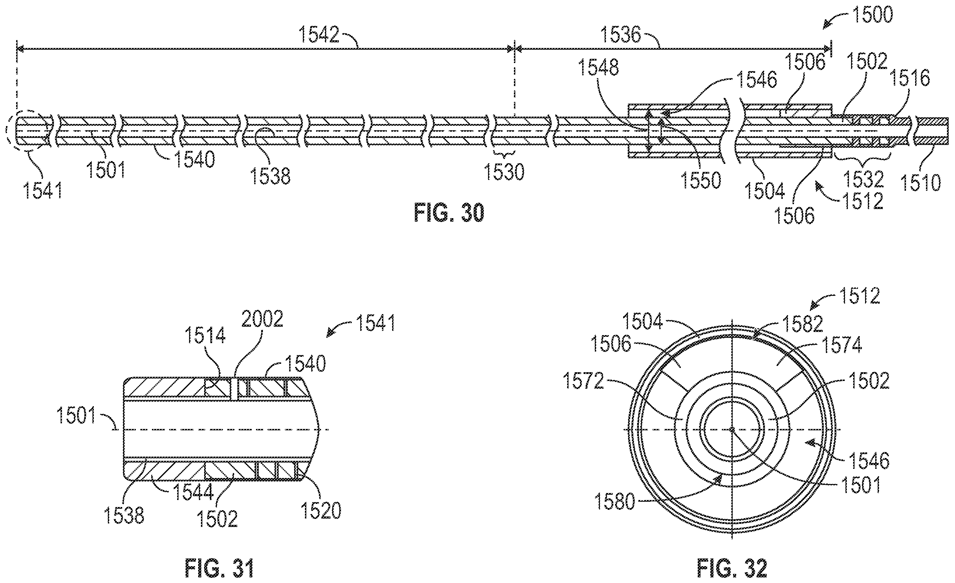

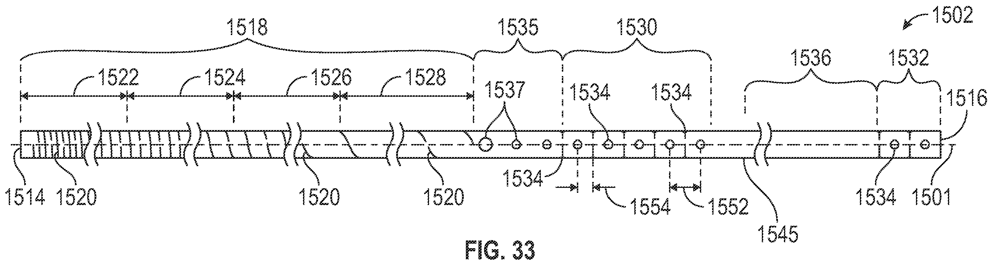

FIG. 29 schematically illustrates the four major components of the pusher shaft 1500, while FIG. 30 illustrates a more detailed example of the pusher shaft 1500. A side view of an exemplary distal end of the pusher shaft 1500 is shown in FIG. 31 and a proximal end view of the pusher shaft 1500 is shown in 32. FIG. 33 shows a main tube (which in some examples, can be a hypo tube) 1502 (FIG. 21E) of the pusher shaft 1500 alone. These figures of the pusher shaft 1500 show a central longitudinal axis 1501 of the pusher shaft 1500.

In some examples, the central longitudinal axis 1501 of the pusher shaft 1500 can be coaxial with a central longitudinal axis of a sleeve shaft (e.g., sleeve shaft 280) and an outer shaft (e.g., outer shaft) 260 of a delivery apparatus (e.g. delivery apparatus 220) when arranged within a delivery apparatus, as explained further below with reference to FIGS. 34 and 35.

As shown in FIGS. 29-33, the example pusher shaft 1500 can comprise four sections or components including the main tube (e.g., shaft) 1502 (FIGS. 29-33), a shell 1504 (FIGS. 29, 30, and 32), a plug 1506 (FIGS. 29, 30, and 32), and a proximal extension 1510 (as shown in FIGS. 29 and 30, which can be similar to the proximal extension 291 shown in FIGS. 5-7).

The main tube 1502 can be configured to advance and retract a docking device (such as one of the docking devices described herein) and house the release suture that secures the docking device to the pusher shaft. The shell 1504 surrounds a portion of the main tube 1502 and the plug 1506 connects the main tube 1502 to the shell 1504 and can be configured as a stop for the sleeve shaft. The proximal extension 1510 can be configured such that the pusher shaft 1500 routes from the inside of the sleeve shaft to the outside of the sleeve shaft, thereby allowing the two shafts to be actuated in parallel with one another and reducing an overall length of the delivery apparatus (e.g., as shown in FIGS. 4-7).

The main tube 1502 can extend from a distal end of an outer shaft (e.g., outer shaft 260 shown in FIG. 4) of the delivery apparatus into a handle assembly (e.g., handle assembly 200 of FIGS. 4 and 5) of the delivery apparatus. As shown in FIGS. 29 and 30, the pusher shaft 1500 can include a proximal end portion 1512 which can include an interface between the main tube 1502, the shell 1504, the plug 1506, and the proximal extension 1510. In some examples, as shown in FIGS. 6 and 7, as described above, the proximal end portion 1512 of the pusher shaft 1500 can be arranged within or proximate to the hub assembly (e.g., hub assembly 230) of the handle assembly of the delivery apparatus. Thus, the main tube 1502 can be an elongate tube that extends along a majority of the delivery apparatus.

In some examples, the main tube 1502 can be a hypo tube. Hypo tubes are components that can be utilized for deploying docking devices and have been previously described in U.S. Pat. Pub. No. 2018/0318079 entitled "Deployment systems, tools, and methods for delivery an anchoring device for a prosthetic valve,". In some examples, the main tube 1502 can comprise a biocompatible metal, such as stainless steel.

In various examples, the main tube 1502 (shown by itself, in greater detail in FIG. 33) is a relatively rigid tube that provides column strength for actuating (e.g., deploying) a docking device from the delivery apparatus.

The main tube 1502 can include a distal end 1514 that is configured to interface with a docking device and a proximal end 1516 that attaches to the proximal extension 1510 (as shown in FIGS. 29, 30, and 33 and discussed further below).

In some examples, as shown in FIG. 33, the main tube 1502 can have a distal section 1518 including a plurality of cuts 1520 therein that are configured to provide the main tube 1502 with increased flexibility at its distal end. Thus, the distal section 1518 may be referred to as a flexible section or portion of the main tube 1502.

In some examples, the cuts 1520 can be laser cuts formed by laser cutting into a surface (e.g., outer surface) of the main tube 1502. In alternate examples, the cuts 1520 can be another type of cut formed by another cutting process (e.g., via etching, scoring, throughcutting, etc., into the outer surface of the main tube 1502). A width and depth of the cuts 1520 can be configured to add a specified amount of flexibility to the main tube 1502.

In some examples, each of the cuts 1520 can be through-and-through cuts that penetrate through an entirety of the main tube 1502 (e.g., from one side to the other, in a direction perpendicular to the central longitudinal axis 1501). In some examples, the width of each cut 1520 can be approximately 0.05mm. In some examples, the width of each cut 1520 can be in a range of 0.03mm to 0.08 mm.

In some examples, a spacing between adjacent cuts 1520 can vary along a length of the distal section 1518. For example, as shown in FIG. 33, adjacent cuts 1520 can be arranged closest together at the distal end 1514 and then the spacing between adjacent cuts 1520 can increase from the distal end 1514 to the proximal end of the distal section 1518.

In some examples, the cuts 1520 can be formed as helical threads cut into (and through) the outer surface of the distal section 1518 of the main tube 1502. Thus, in these examples, the spacing or distance between adjacent cuts 1520 can be defined as the pitch of the cuts. As shown in FIG. 33, a first portion 1522 of the distal section 1518 can have a pitch in a range of 0.4mm to 0.64mm, a second portion 1524 of the distal section 1518 can have a pitch in a range of 0.64 to 1.2mm, a third portion 1526 of the distal section 1518 can have a pitch of 1.2mm, and a fourth portion 1528 of the distal section 1518 can have a pitch in a range of 1.2mm to 3.0mm. In some examples, the pitch of the first portion 1522 can increase from 0.4mm (at its distal end 1514) to 0.64mm along its length, the pitch of the second portion 1524 can increase from 0.64mm to 1.2mm along its length, the pitch of the third portion 1526 can be approximately 1.2mm along its length, and the pitch of the fourth portion 1528 can increase from 1.2mm to 3.0mm along its length. It should be noted that the above pitch values for the distal section 1518 are exemplary and other pitches may be possible, where the pitch values can be selected to provide the main tube 1502 with increased flexibility at its distal end 1514 and a decreasing amount of flexibility along the length of the distal section 1518. In this way, the distal section 1518 can be configured to flex and/or bend along with the outer shaft 2260 of the delivery system, as it is navigated through an inner lumen of a patient, to the target implantation site.

The main tube 1502, in some examples, can include one or more portions or sections that include a plurality of apertures 1534 that are configured to enable bonding of an outer, flexible polymer layer (e.g., covering or jacket), arranged along a portion of an outer surface of the main tube 1502, to an inner liner, the inner liner arranged along an inner surface of the main tube 1502. At the same time, the apertures 1534 can be configured to maintain a rigidity of the pusher shaft 1500.

The example of the main tube 1502 shown in FIG. 33 includes a first section 1530 and a second section 1532, spaced apart from one another, each including one or more apertures 1534 extending through a thickness of the main tube 1502 (e.g., through-holes extending from and through an outer surface 1545 to an inner surface of the main tube 1502). The apertures 1534 can be spaced around a circumference of the main tube 1502. In some examples, as shown in FIG. 33, each aperture 1534 can extend through an entirety of the main tube 1502, thereby creating two apertures 1534 arranged 180 degrees apart from one another around the circumference of the main tube 1502. Further, in some examples, adjacent sets of apertures 1534 can be offset from one another by 90 degrees (e.g., as shown in FIG. 33, the first section 1530 may include 20 apertures).

The size and/or shape of each aperture 1534 and a number and spacing between the apertures 1534 of each of the first section 1530 and the second section 1532 can be selected to allow the outer, flexible polymer layer to bond (e.g., bind) to the inner liner, with the main tube 1502 arranged therebetween, and still provide rigidity to the pusher shaft 1500. For example, the apertures 1534 can be circular with a diameter in a range of 0.4 to 0.6mm. In some examples, the diameter of the apertures 1534 can be approximately 0.5mm. In some examples, the apertures 1534 can have another shape, such as oblong, square, rectangular, star-shaped, triangular, or the like.

In some examples, along the length of the first section 1530 and the second section 1532, in the axial direction, the apertures can be spaced apart from one another at a first (center-to-center) distance 1552 and each set of apertures 1534 at the same axial position can be spaced apart from an adjacent set of apertures 1534 at a second distance 1554. In some examples, the first distance 1552 is approximately 2mm and the second distance 1554 is approximately 1.0mm. In some examples, the first distance 1552 is in a range of 1.5mm to 2.5mm and the second distance 1554 is in a range of 0.5mm to 1.5mm. In some examples the second distance 1554 is half the first distance 1552. In alternate examples, a different number of apertures 1534 and/or relative spacing between and arrangement of the apertures 1534 than that shown in FIG. 33 and described above is possible, while still providing adequate bonding between the inner liner and the outer flexible polymer and providing rigidity to the pusher shaft 1500.

As shown in FIG. 33, the second section 1532 can be arranged at the proximal end 1516 of the main tube 1502 and includes fewer apertures 1534 than the first section 1530. However, in alternate examples, the second section 1532 can include more apertures 1534 than shown in FIG. 33. In some examples, the first section 1530 can include 20 apertures 1534 and the second section 1532 can include 8 apertures. In other examples, the first section 1530 can include more or less than 20 apertures 1534 and the second section 1532 can include more or less than 8 apertures 1534.

As shown in FIG. 33, the main tube 1502 can include a third section 1536 arranged and extending between the first section 1530 and the second section 1532 which does not include any apertures 1534.

In some examples, as described further below, the main tube 1502 can include an intermediate section 1535 arranged proximal to the distal section 1518 (e.g., which includes the cuts 1520) and arranged distal to or part of the first section 1530. As described further below, the intermediate section 1535 can include one or more apertures 1537 defined in the outer surface 1545, which can be of various sizes, that are configured to permit a flow of fluid from within an interior of the main tube 1502 (e.g., a pusher shaft lumen 1555, as shown in FIGS. 34 and 35) to a lumen surrounding the pusher shaft 1500 when the pusher shaft 1500 is arranged within a sleeve shaft of a delivery apparatus (e.g., delivery apparatus 220). The outer surface 1545 of the main tube 1502 in which the one or more apertures 1537 are disposed can be an outer circumferential surface where a line normal to the outer surface 1545 intersects the central longitudinal axis 1901.

FIG. 30 illustrates an exemplary example of the components of the pusher shaft 1500. As shown in FIG. 30, the pusher shaft 1500 can include an inner liner 1538 covering an inner surface of the main tube 1502 and forming an inner surface of the proximal extension 1510. In some examples, the inner liner 1538 can extend along an entire length of the pusher shaft 1500. In some examples, the inner liner can be relatively thin and comprise a polymeric material, such as PTFE. For example, a thickness of the inner liner 1538 can be in a range of 0.012mm to 0.064mm.

Additionally, in some examples, a portion of the pusher shaft 1500 can include an outer polymer layer (also referred to as an outer covering or jacket) 1540. The outer polymer layer 1540 can be a flexible polymer, as explained further below. In some examples, the outer polymer layer 1540 is arranged over and along a fourth section 1542 (the fourth section 1542 including the distal section 1518 and the first section 1530) of the main tube 1502, while the third section 1536 of the main tube 1502 does not include the outer polymer layer 1540 (FIGS. 30, 31, and 33).

In some examples, the outer polymer layer 1540 can also be included on the second section 1532 of the main tube 1502 and forms an outer layer of the proximal extension 1510. For example, the proximal extension 1510 can comprise the inner liner 1538 and the outer polymer layer 1540 (FIG. 30).

In some examples, the outer polymer layer 1540 can be reflowed over the cuts 1520 and the apertures 1534.

In certain examples, the outer polymer layer 1540 can comprise a polyether-amide block copolymer or a blend of two or more polyether-amide block copolymers. The polymer of the outer polymer layer 1540 can have a Shore D hardness measured according to ISO 868:2003 of between about 60 and about 75, between about 65 and about 75, between about 70 and about 75, or about 72. In some examples, the outer polymer layer 1540 can have a flexural modulus measured according to ISO 178:2010 of between about 350 MPa and about 550 MPa, between about 450 MPa and about 550 MPa, between about 500 MPa and about 550 MPa, between about 500 MPa and about 525 MPa, between about 510 MPa and about 520 MPa, about 500 MPa, about 505 MPa, about 510 MPa, about 515 MPa, about 520 MPa, or about 525 MPa. In certain examples, the outer polymer layer 1540 can be one of or a blend of two or more of PEBAX^{®} grades 7033 and 7233 (Arkema S.A., France) and VESTAMID^{®} grades E62, E72, and EX9200 (Evonik Industries AG, Germany). In some examples, the outer polymer layer 1540 can be PEBAX^{®} 7233. In other examples, the outer polymer layer 1540 can be VESTAMID^{®} EX9200.

In some examples, the main tube 1502 can possess a uniform inner diameter, from its distal end 1514 to its proximal end 1516, in a range of about 1.0 mm to about 1.34mm, while the outer diameter can vary from approximately 1.8 to 2.0 mm (e.g., ±0.2 mm) in the proximal and distal sections.

An example of a distal tip 1541 of the pusher shaft 1500 is shown in FIG. 31. In some examples, the distal tip 1541 includes a more flexible, polymeric tip or distal end portion 1544 which comprises a flexible polymer. In some examples, the polymeric distal end portion 1544 can comprise the same flexible material as and/or be continuous with the outer polymer layer 1540. Thus, the polymeric distal end portion 1544 of the distal tip 1541 can be reflowed over the distal end 1514 of the main tube 1502 and bonded to the inner liner 1538.

As shown in FIGS. 29, 30, and 32, an inner diameter 1548 of the shell 1504 is larger than an outer diameter 1550 of the main tube 1502, thereby forming an annular cavity 1546 between (in the radial direction) the main tube 1502 and the shell 1504. As such, the proximal section 284 of the sleeve shaft 280 can slide within the annular cavity (e.g., space) 1546, as described further below with reference to FIGS. 34 and 35. Further, fluid (e.g., flush fluid) provided to a lumen on an exterior of the proximal extension 1510, in the hub assembly, can flow through the annular cavity 1546 and exit the distal end of the shell, as shown by arrows 217 in FIG. 29 to enter a lumen (e.g., delivery shaft lumen 215 shown in FIG. 9A) between the sleeve shaft 280 and outer shaft 260 of the delivery apparatus, as discussed above with reference to FIG. 6-9A.

The plug 1506 can be configured to be arranged within the annular cavity 1546, at a proximal end 1505 of the shell 1504 (as shown in FIGS. 29, 30, and 32). In some examples, the plug 1506 can have a length 1507, extending in a direction of the central longitudinal axis 1501 (as shown in FIG. 29). In some examples, the length 1507 is in a range of 3.0mm to 9.0mm, of 4.0mm to 8.0mm, of 5.0mm to 7.0mm, or of 5.5 to 6.5mm. In some examples, the length 1507 is approximately 6.0mm.

The plug 1506 can be configured to "plug" or fill a portion of the annular cavity 1546, at the proximal end 1505, while leaving a remainder of the portion of the annular cavity open to receive a cut portion of the sleeve shaft therein (e.g., cut portion 288 of sleeve shaft 280 shown in FIGS. 5-7). For example, as shown in FIG. 32, in some examples, the plug 1506 of the pusher shaft 1500 can include an annular portion 1572 and a crescent-shaped portion 1574 extending radially outward from one side of the annular portion 1572. An inner diameter 1576 of the annular portion 1572 can be selected such that the annular portion 1572 encircles the outer surface 1545 of the main shaft 1502 and an outer diameter 1578 of the crescent-shaped portion 1574 can be selected such that the crescent-shaped portion 1574 fills the annular space 1546 (FIG. 29). For example, the inner diameter 1576 can be selected to be slightly larger than the outer diameter 1550 of the main shaft 1502 and the outer diameter 1578 can be selected to be slightly smaller than the inner diameter 1548 of the shell 1504 (as shown in FIG. 29). In some examples, the inner diameter 1576 is approximately 1.81mm and the outer diameter 1578 is approximately 3.42mm. An arc length of the crescent-shaped portion 1574 can be in a range of 60 to 140 degrees, 80 to 120 degrees, 90 to 110 degrees, or 95 to 105 degrees.

In certain examples, the shell 1504 and the plug 1506 can be welded to the main tube 1502 to allow the cut portion of the sleeve shaft to slide between the main tube 1502 and the shell 1504. For example, as shown in the FIG. 32, a first weld 1580 can secure the annular portion 1572 of the plug 1506 to the main tube 1502 and a second weld 1582 can secure the crescent-shaped portion 1572 of the plug 1506 to the shell 1504. In some examples, each of the welds 1580 and 1582 can be tack welds that do not extend along an entirety of the mating surfaces between the plug 1506 and main shaft 1502 and shell 1504.

FIG. 30 shows the proximal extension 1510 extending distally from the second section 1932 of the main tube 1502. As noted above, the proximal extension 1510 provides the pusher shaft 1500 with flexibility such that it may be routed from the inside of the sleeve shaft (e.g., the cut portion) to the outside of the sleeve shaft, thereby allowing the two shafts to be actuated in parallel. In many examples, as discussed above, the proximal extension 1510 can be made of a flexible polymer. In certain examples, the flexible polymer is a polyether-amide block copolymer or a blend of two or more polyether-amide block copolymers, such as PEBAX^{®} grades 2533, 3533, 4033, 4533, 5533, 6333, and 7033, and 7233 (Arkema S.A., France) and VESTAMID^{®} grades E40, E47, E55, E62, E72, and EX9200 (Evonik Industries AG, Germany).

Turning now to FIGS. 34 and 35, an exemplary arrangement of the pusher shaft 1500 assembled together with the sleeve shaft 280 and outer shaft 260 of the delivery apparatus 220 (e.g., a pusher shaft and sleeve shaft assembly 1600) is shown. As introduced above, the pusher shaft 1500 and sleeve shaft 280 can be coaxial with one another, at least within the outer shaft 260 (e.g., catheter portion) of the delivery apparatus (e.g., delivery apparatus 220 of FIGS. 4-8).

As shown in FIGS. 34 and 35, the sleeve shaft 280 can be configured to cover (e.g., surround) the docking device 232 and, together, the pusher shaft 1500 and sleeve shaft 280 can be configured to deploy the docking device 70 232 from the outer shaft 260 of the delivery apparatus, upon reaching the target implantation site. FIGS. 34 and 35 illustrate different stages of the implantation process.

For example, FIGS. 34 and 35 illustrate how a proximal section 1604 of sleeve shaft 280, including the cut portion 288, passes through the proximal end portion 1512 of the pusher shaft 1500, between the main tube 1502 and the shell 1504, within the annular cavity 1546.

Specifically, FIG. 34 illustrates an example of a first configuration of the pusher shaft and sleeve shaft assembly 1600, pre-deployment or during deployment of the docking device 232, where the sleeve shaft 280 is arranged over the docking device 232 and an end surface 279 of a tube 285 of the sleeve shaft 280 is positioned away from the plug 1506.

During deploying the docking device 232 from the outer shaft 260 of the delivery apparatus, the pusher shaft 1500 and the sleeve shaft 280 can move together, in the axial direction, with the docking device 232. For example, actuation of the pusher shaft 1500, to push against the docking device 232 and move it out of the outer shaft 260 may also cause the sleeve shaft 280 to move along with the pusher shaft 1500 and the docking device 232. As such, the docking device 232 may remain covered by the distal section 282 of the sleeve shaft 280 during pushing the docking device 232 into position at the target implantation site via the pusher shaft 1500.

In some examples, as shown in FIG. 34, the outer shaft 260 can have a first inner diameter 1650 at a distal end portion of the outer shaft 260 and a second inner diameter 1652 at a more proximal end portion of the outer shaft 260. The second inner diameter 1652 can be larger than the first inner diameter 1650 in order to accommodate the wider shell 1504 therein.

Additionally, during delivery and implantation of the covered docking device 232 at the target implantation site, a distal tip 1612 of the distal section 282 of the sleeve shaft 280 can extend distal to (e.g., past) a distal end 1654 of the docking device 232, thereby providing the distal section 282 of the sleeve shaft 280 with a more atraumatic tip.

FIG. 35 illustrates a second configuration of the pusher shaft and sleeve shaft assembly 1600, after deploying the docking device 232 from the outer shaft 260 at the target implantation site and retracing the sleeve shaft 280 away from the implanted docking device 232. As shown in FIG. 35, after implanting the docking device 232 at the target implantation site, in its desired position, the sleeve shaft 280 can be pulled off the docking device 232 and retracted back into the outer shaft 260. In some examples, as shown in FIG. 35, the sleeve shaft 280 can be stopped from further retraction into the delivery apparatus upon the end surface 1645 coming into contact with the plug 1506.

Further details on a pusher shaft and sleeve shaft assembly for a delivery apparatus for a docking device, including the various material and structural makeup of the components, are described in International Patent Application No. PCT/US20/36577.

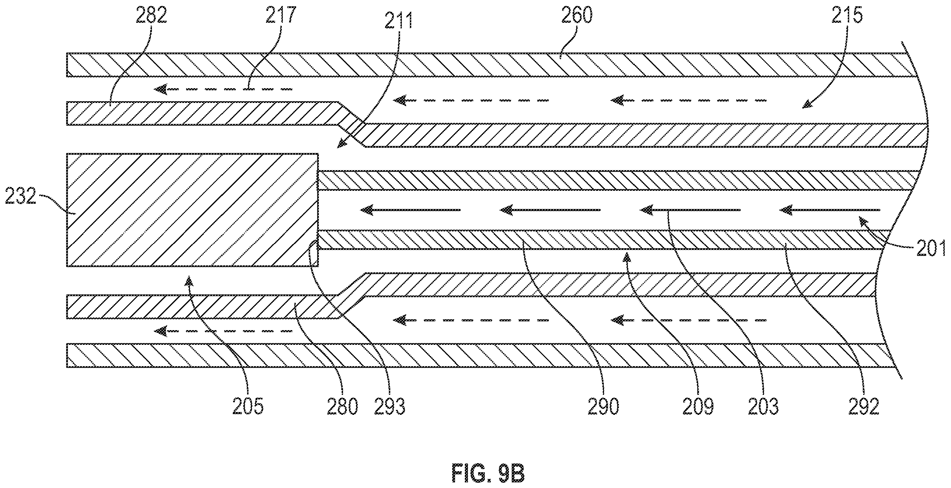

As introduced above with reference to FIGS. 5-9A, spaces or lumens are formed between various components of a delivery apparatus including a pusher shaft. Such lumens can include a pusher shaft lumen (e.g., pusher shaft lumen 201 shown in FIG. 9A and pusher shaft lumen 1555 shown in FIGS. 34 and 35) defined by an inner surface of a main tube of a pusher shaft and a delivery shaft lumen (e.g., delivery shaft lumen 215 shown in FIG. 9A). As discussed above with reference to FIG. 9A, the pusher shaft lumen can supply fluid to a sleeve shaft lumen (e.g., sleeve shaft lumen 211 shown in FIG. 9 and sleeve shaft lumen 1557 shown in FIGS. 34 and 35) formed between the sleeve shaft and a docking device and between the pusher shaft and the sleeve shaft.

As discussed herein, by maintaining a consistent flow of fluid throughout these lumens of the delivery apparatus, blood stagnation can be reduced or avoided, thereby preventing thrombosis. However, as shown in FIGS. 34 and 35, the distal tip 1541 of the pusher shaft 1500 can be arranged adjacent to a proximal end of the docking device 232. This arrangement can also be seen in the example of FIG. 9B where the distal end 293 of the pusher shaft 290 is arranged against (e.g., abuts) a proximal end of the docking device 232.

At various stages during an implantation procedure, the proximal end of the docking device 232 can compress on the distal end or tip of the pusher shaft (e.g., distal tip 1541 of the pusher shaft 1500) with varying amounts of force. This inconsistency in interaction between the distal tip 1541 of the pusher shaft 1500 and the docking device 232 can lead to varying amounts of fluid flowing out of the pusher shaft lumen 1555 and into the sleeve shaft lumen 1557 (FIGS. 34 and 35).

In some examples, the docking device 232 can fully occlude the pusher shaft lumen (e.g., due to be pushed up against the distal tip 1541), thereby stopping all flow out of the pusher shaft lumen and preventing fluid from reaching the sleeve shaft lumen. For example, as illustrated in FIG. 9B, when the distal end 293 of the pusher shaft 290 is pushed up against the docking device 232, flush fluid flow 203 is blocked from exiting the pusher shaft lumen 201 and reaching the sleeve shaft lumen 211. This can lead to an increased risk of thrombosis.

Thus, it may be desirable to create additional flow paths between the pusher shaft lumen and the sleeve shaft lumen, thereby allowing fluid to reach and flow through the sleeve shaft and preventing thrombosis (e.g., even when the distal end of the pusher shaft abuts the proximal end of the docking device, and thus, the docking device at least partially or fully blocks fluid from exiting the distal end of the pusher shaft).

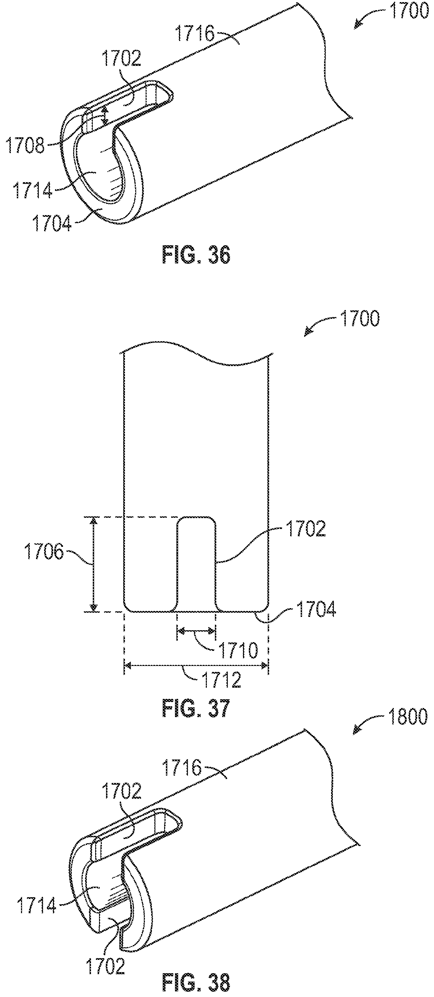

FIGS. 36-44 illustrate various modifications to and/or examples of the pusher shaft 1500 of FIGS. 29-35 which provide additional flow paths out of the pusher shaft lumen 1555 (e.g., an interior of the main tube 1502 and distal tip 1541) such that fluid communication can be increased between the pusher shaft lumen 1555 and the sleeve shaft lumen 1557.

In some examples, as introduced above with reference to FIG. 33 and also shown in FIGS. 34 and 35, one or more apertures 1537 can be included in the intermediate section 1535 of the main tube 1502. The one or more apertures 1537 can extend through each of a thickness of the main tube 1502, the inner liner 1538, and the outer polymer layer 1540. For example, each aperture 1537 can extend between and through an inner surface 1563 and an outer surface 1561 of the pusher shaft 1500 (e.g., the outer surface defined by the outer polymer layer 1540 and the inner surface defined by the inner liner 1538). As a result, when the pusher shaft 1500 is included within the pusher shaft and sleeve shaft assembly 1600 of FIGS. 34 and 35 (or another pusher shaft and sleeve shaft assembly of another delivery apparatus), fluid can pass from the pusher shaft lumen 1555 to the sleeve shaft lumen 1557 via the one or more apertures 1537.

In some examples, as shown in FIG. 33, the one or more apertures 1537 can be disposed in the pusher shaft 1500, proximal to the cuts 1520 of the distal section 1518.

In some examples, the intermediate section 1536 can include only one aperture 1537, multiple apertures 1537 at a same axial position (e.g., two apertures 1537 arranged 180° apart from one another, as shown in FIGS. 34 and 35), multiple apertures 1537 spaced axially apart from one another along the intermediate section (as shown in FIG. 33), or a combination of these. In some examples, the intermediate section 1536 can include at least two apertures 1537 spaced apart from one another around a circumference of the pusher shaft 1500.

In some examples, the one or more apertures 1537 can have various sizes (e.g., diameters), as shown in FIG. 33. In some examples, if the pusher shaft 1500 includes multiple apertures 1537, all the apertures 1537 can have a same size or one or more of the multiple apertures 1537 can have different sizes.

In some examples, the one or more apertures 1537 can have a same size (e.g., diameter) as the apertures 1534 (FIG. 33). In other examples, the one or more apertures 1537 can be larger or smaller than the apertures 1534.

In some examples, the one or more apertures 1537 can be circular. In other examples, the one or more apertures 1537 can have a different shape (or different apertures 1537 can have different shapes), such as square, rectangular, oval, rectangular, slit-shaped, or the like.

In some examples, the one or more apertures 1537 can be cut into the pusher shaft 1500, through the main tube 1502 and the surrounding inner liner 1538 and the outer polymer layer 1540. In some examples, the one or more apertures 1537 can be created by laser cutting through the pusher shaft 1500.

FIGS. 36-40 show examples of a distal tip of the pusher shaft 1500 with one or more slots arranged therein that are configured to provide a path for fluid to flow out of the pusher shaft (e.g., out of the pusher shaft lumen and into the sleeve shaft lumen 1557, as shown in FIGS. 34 and 35). The distal tips shown in FIGS. 36-40 can be the same or similar to the distal tip 1541 shown in FIG. 31, except they include one or more slots extending through a thickness of the distal tip of the pusher shaft 1500.

In some examples, the slots described below with reference to FIGS. 36-40 can be cut into the assembled pusher shaft 1500 (e.g., the pusher shaft 1500 shown in FIGS. 30 and 31). In some examples, the slots can be created by a laser cutting process. As discussed further below, in some examples, if the slot(s) extend into the main tube 1502 of the pusher shaft (e.g., past the polymeric distal end portion 1544), laser settings for cutting the slots can be set to cut through metal (e.g., stainless steel).

FIGS. 36 and 37 show an example of a distal tip 1700 of the pusher shaft 1500 including a slot 1702 cut into the distal tip 1700. FIG. 36 is a perspective view of the distal tip 1700 and FIG. 37 is side view of the distal tip 1700.

In some examples, as shown in FIGS. 36 and 37, the slot 1702 can extend from a distal end 1704 of the distal tip 1700 to a distance (e.g., axial distance) away from the distal end 1704 (and into the distal tip 1700), where the distance is an axial length 1706 of the slot 1702.

In some examples, the axial length 1706 can be selected such that it extends through the polymeric distal end portion 1544 (FIG. 31) and a distal portion of the main tube 1502. In other examples, the axial length 1706 can be selected such that it extends through only the polymeric distal end portion 1544 and not the main tube 1502.

In some examples, the slot 1702 can have a depth 1708 (in a radial direction) such that it extends though a thickness of the distal tip 1700. For example, the slot 1702 can extend between and through an inner surface 1714 and an outer surface 1716 of the distal tip 1700 (and pusher shaft 1500).

In some examples, the slot 1702 can have a width 1710. The width 1710 can be smaller than a total diameter 1712 of the distal tip 1700 (as shown in FIG. 37). In some examples, the width 1710 can be uniform along the axial length 1706. In other examples, the width 1710 can be uniform along a majority of the axial length 1706.

The axial length 1706 and the width 1710 can be selected based on a desired amount of flow between the pusher shaft lumen and the sleeve shaft lumen (e.g., increasing these dimensions can increase the flow path provided between the pusher shaft lumen and the sleeve shaft lumen). In some examples, the axial length 1706 and the width 1710 can also be selected to maintain a structural integrity of the distal tip 1700.

FIG. 38 shows another example of a distal tip 1800 of the pusher shaft 1500 including two slots 1702 cut into the distal tip 1800. As shown in FIG. 38, in some examples, the two slots 1702 can be arranged 180° apart from one another around a circumference of the distal tip 1800.

In some examples, both slots 1702 can have the same axial length 1706 and width 1710. In other examples, the two slots 1702 can have different axial lengths 1706 and/or widths 1710.

Due to the multiple slots 1702, the distal tip 1800 can provide additional fluid flow from the pusher shaft lumen to the sleeve shaft lumen, as compared to the distal tip 1700 of FIGS. 36 and 37. However, the distal tip 1800 of FIG. 38 may increase a risk of opening (e.g., widening) the polymeric distal end portion 1544 of the distal tip 1800, due to the multiple slots 1702, which can result in difficulties in releasing a docking device after positioning at a target implantation site.

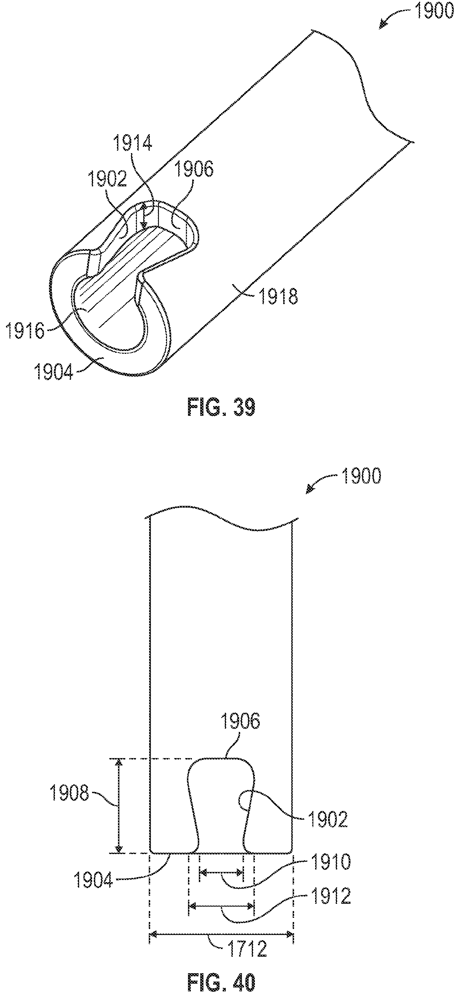

FIGS. 39 and 40 show another example of a distal tip 1900 of the pusher shaft 1500 including one or more slots 1902 (one shown in FIGS. 39 and 40) cut into the distal tip 1900. The slot 1902 can be similar to slot 1702, as described above, but slot 1902 can have an increasing width from a distal end 1904 of the distal tip 1900 to a proximal end 1906 of the slot 1902. This configuration of the slot 1902 can reduce stress concentrations at the distal tip 1900, as well as reducing a risk of opening the distal tip 1900 due to excessive suture tension of a suture extending from the pusher shaft to the docking device, as compared to the more uniform width slots 1702 of FIGS. 36-38.

As shown in FIG. 40, the slot 1902 can have one or more customizable dimensions, including an axial length 1908, a first (narrower) width 1910, and a second (wider) width 1912. The second width 1912 (at the proximal end 1906) can be wider than the first width 1910 (at the distal end 1904) and smaller than the total diameter 1712 of the distal tip 1900. As discussed above, these dimensions of the slot 1902 can be selected based on a specified (e.g., desired) amount of flow between the pusher shaft lumen and the sleeve shaft lumen and maximum dimensions that can maintain a structural integrity of the distal tip 1900 (e.g., and reduce a risk of opening or widening of the distal tip 1900).

In some examples, as shown in FIG. 39, the distal tip 1900 can include a single slot 1902. In other examples, the distal tip 1900 can include two or more slots 1902 spaced around a circumference of the distal tip 1900 (e.g., similar to the example shown in FIG. 38).

In some examples, the one or more slots 1902 can have a depth 1914 (in a radial direction) such that it extends through a thickness of the distal tip 1900. For example, the slot 1902 can extend between and through an inner surface 1916 and an outer surface 1918 of the distal tip 1900 (and pusher shaft 1500).

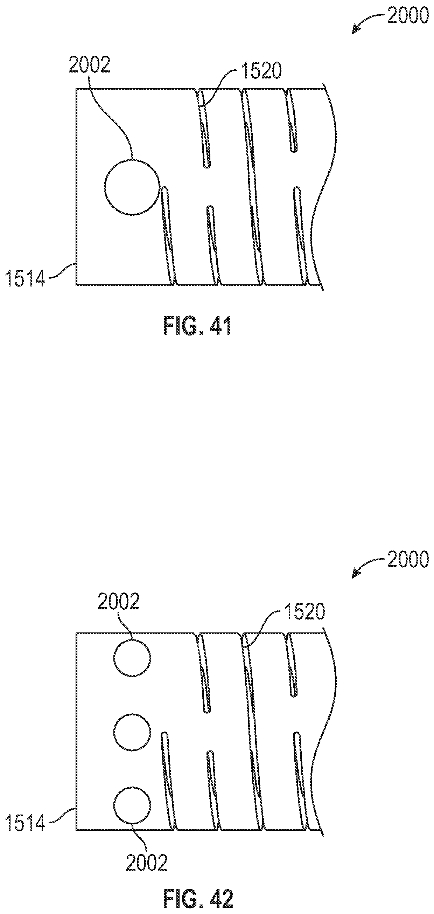

FIGS. 41 and 42 show examples of a distal end portion 2000 of the main tube 1502 of the pusher shaft 1500 with one or more apertures 2002 disposed therein that are configured to provide a path for fluid to flow out of the pusher shaft (e.g., out of the pusher shaft lumen and into the sleeve shaft lumen 1557, as shown in FIGS. 34 and 35). The one or more apertures 2002 can extend through each of a thickness of the main tube 1502, the inner liner 1538, and the outer polymer layer 1540 (FIG. 31). For example, each aperture 2002 can extend between and through an inner surface and an outer surface (e.g., inner surface 1563 and outer surface 1561 shown in FIG. 34) of the pusher shaft 1500 (e.g., the outer surface can be defined by the outer polymer layer 1540 and the inner surface can be defined by the inner liner 1538). As a result, when the pusher shaft 1500 is included within the pusher shaft and sleeve shaft assembly 1600 of FIGS. 34 and 35 (or another pusher shaft and sleeve shaft assembly of another delivery apparatus), fluid can pass from the pusher shaft lumen 1555 to the sleeve shaft lumen 1557 via the one or more apertures 1537.

As shown in FIGS. 41 and 42, the one or more apertures 2002 can be arranged at the distal end 1514 of the main tube 1502, distal to the cuts 1520. Further, the one or more apertures 2002 can be arranged proximal to the polymeric distal end portion 1544, as shown in FIG. 31.

In some examples, the one or more apertures 2002 can include at least two apertures 2002 spaced apart from one another around a circumference of distal end portion 2000.

FIG. 41 shows an example where the distal end portion 2000 includes one or two apertures 2002 (e.g., one aperture 2002 can be cut through an entirety of the pusher shaft, thereby creating two apertures 2002 arranged 180° apart from one another around a circumference of the distal end portion 2000).

FIG. 42 shows another example where the distal end portion 2000 includes multiple apertures 2002 spaced around a circumference of the distal end portion 2000. In some examples, a diameter or width of the apertures 2002 can be smaller than the one or more apertures 2002 in FIG. 41.

The apertures 2002 can have various sizes (e.g., diameters or widths) and/or shapes (e.g., circular, as shown in FIGS. 41 and 42, or square, oval, rectangular, or the like). The size and/or shape of each of the one or more apertures 2002 can be selected to achieve a desired flow of fluid out of the pusher shaft lumen and into the sleeve shaft lumen.

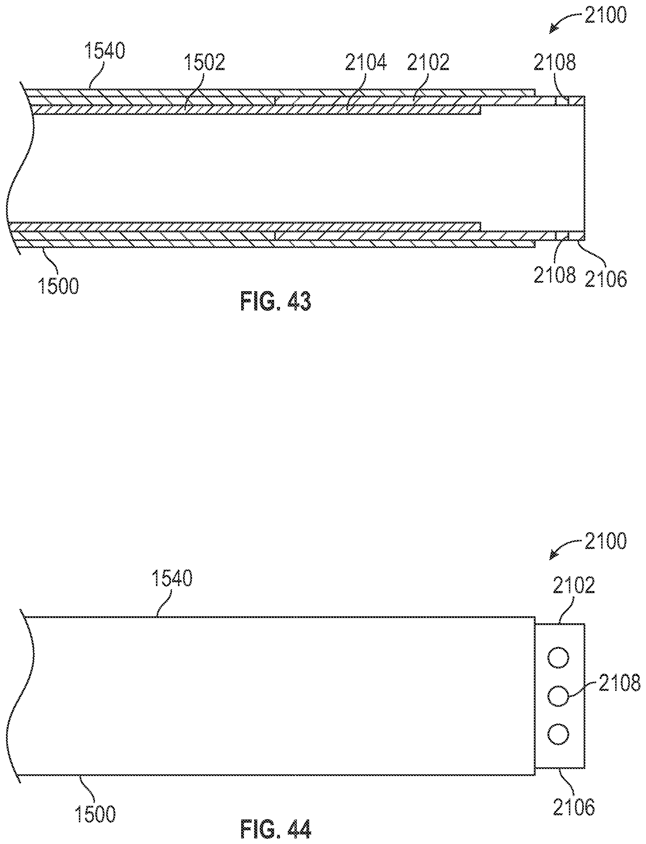

FIGS. 43 and 44 show an example of a distal end portion (e.g., tip or tip portion) 2100 of the pusher shaft 1500 that is configured to provide one or more additional paths for fluid to flow out of the pusher shaft (e.g., out of the pusher shaft lumen 1555 and into the sleeve shaft lumen 1557, as shown in FIGS. 34 and 35). FIG. 43 is a cross-sectional view and FIG. 44 is a side view of the distal end portion 2100 which includes a distal tip 2102 arranged around a distal end portion 2104 of the main tube 1502 of the pusher shaft 1500 and the outer polymer layer 1540 reflowed over the distal tip 2102 and a portion of the main tube 1502 arranged proximal to the distal tip 2102.

In some examples, as shown in FIG. 43, the distal tip 2102 can be arranged and/or coupled around the distal end portion 2104 of the main tube 1502. The outer polymer layer 1540 can surround (e.g., cover) the main tube 1502 and a portion (e.g., a proximal portion, which can be a majority portion) of the distal tip 2102. A tip portion 2106 of the distal tip 2102 can extend distally past the main tube 1502. Further, as shown in FIGS. 43 and 44, the tip portion 2106 is not covered by the outer polymer layer 1540.

The tip portion 2106 of the distal tip 2102 can include one or more apertures 2108 disposed therein (FIGS. 43 and 44). The one or more apertures 2108 can extend through a thickness of the distal tip 2102 (FIG. 43).

In some examples, the one or more apertures 2108 can be spaced apart from one another around a circumference of the tip portion 2106 (FIG. 44). The one or more apertures 2108 can have various sizes and/or shapes (e.g., circular, square, rectangular, or the like).

In other examples, instead of multiple apertures 2108, the tip portion 2106 can include one or more slots or an elongate aperture arranged therein that extends through a thickness of the distal tip 2102.

The distal tip 2102 can be molded or extruded from a polymeric material (e.g., nylon).

In some examples, the one or more apertures 2108 can be die cut or laser cut into the molded or extruded distal tip 2102.

In some examples, the assembled pusher shaft 1500 can be modified to include the distal tip 2102. For example, the outer polymer layer 1540 can be cut away/removed at the distal tip 1541 to expose the distal end portion 2104 of the main tube 1502. The distal tip 2102 can then be attached to and around the distal end portion 2104 of the main tube 1502. The outer polymer layer 1540 can then be reflowed over an outer surface of the distal tip 2102, but leaving the tip portion 2106 uncovered, thereby leaving the one or more apertures 2108 exposed.

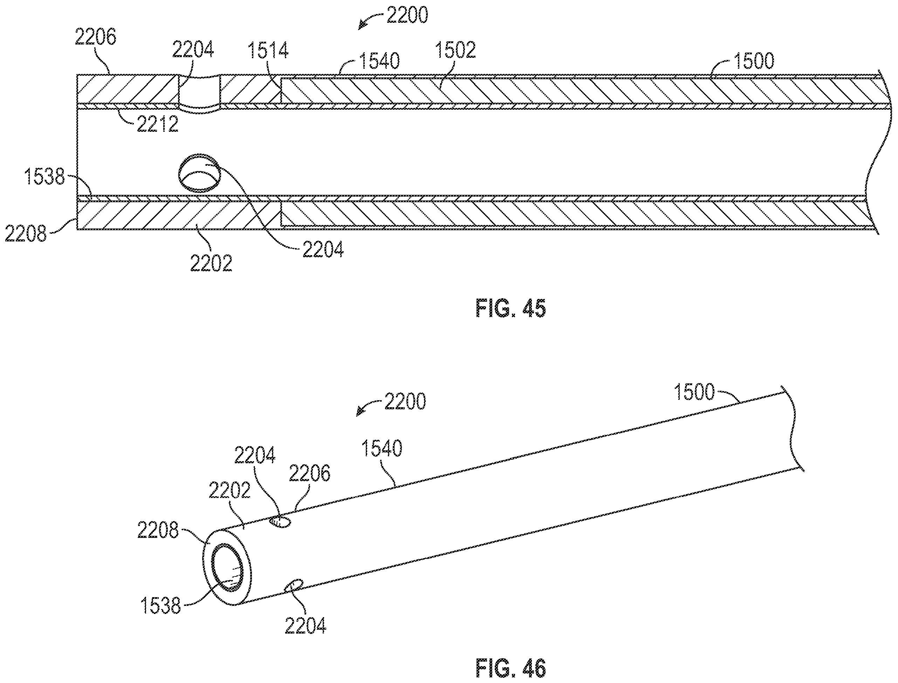

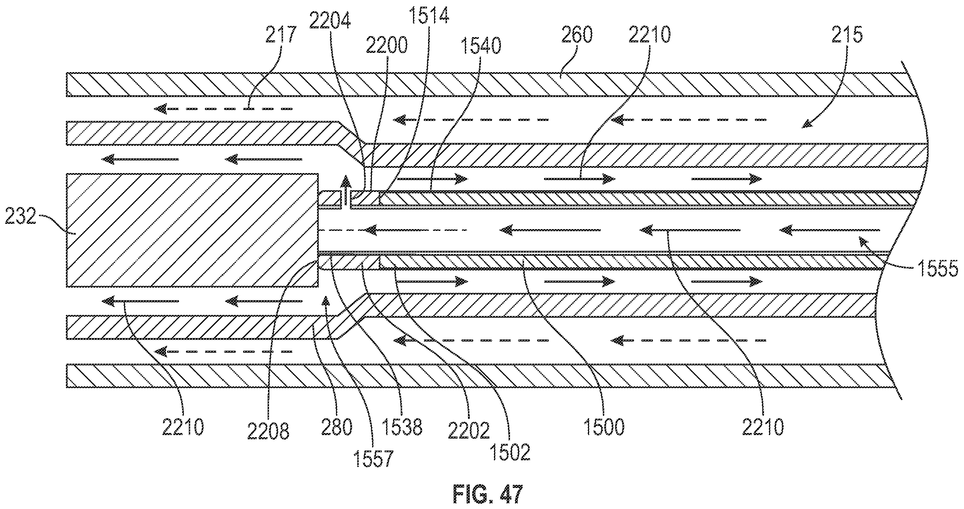

FIGS. 45-47 show another example of a distal end portion (e.g., distal tip or tip portion) 2200 of the pusher shaft 1500 that is configured to provide one or more additional paths for fluid to flow out of the pusher shaft (e.g., out of the pusher shaft lumen 1555 and into the sleeve shaft lumen 1557, as shown in FIGS. 34 and 35).

The distal end portion 2200 can include a more flexible, polymeric tip (or distal end) 2202 which comprises a flexible polymer (e.g., the same or similar to the polymeric tip 1544 shown in FIG. 31, as described above). In some examples, the polymeric tip 2202 can comprise the same flexible material as and/or be continuous with the outer polymer layer 1540 of the pusher shaft 1500 (e.g., as described above with reference to FIG. 31). Thus, the polymeric tip 2202 can be reflowed over the distal end 1514 of the main tube 1502 of the pusher shaft 1500 and bonded to the inner liner 1538.

In the example shown in FIGS. 45-47, one or more apertures 2204 can be disposed in the polymeric tip 2202, distal to the main tube 1502, with each of the one or more apertures 2204 having a central axis extending in a radial direction. In some examples, each of the one or more apertures 2204 can extend from an outer surface 2206 of the polymeric tip 2202 to an inner surface 2212 of the inner liner 1538, thereby extending through a thickness of each of the polymeric tip 2202 and the inner liner 1538.

In other examples, when the polymeric tip 2202 does not include the inner liner 1538, each of the one or more apertures 2204 can extend through a thickness of the polymeric tip 2202, from the outer surface 2206 to an inner surface of the polymeric tip 2202.

In some examples, as shown in cross-sectional and perspective views of FIGS. 45 and 46, respectively, one or more apertures 2204 can be disposed in the polymeric tip 2202, around a circumference of the polymeric tip 2202. For example, the polymeric tip 2202 can include 1-12 apertures 2204 (or 2-6, in certain examples). The apertures 2204 can comprise various sizes and can be spaced apart from one another around the circumference of the polymeric tip 2202. In some examples, the apertures can all comprise a uniform size (e.g., diameter) and/or be evenly distributed relative to each other (e.g., three apertures spaced 120 degrees apart). In other examples, one or more of the apertures can comprise a larger or smaller size than one or more other apertures and/or can be non-evenly distributed relative to each other.

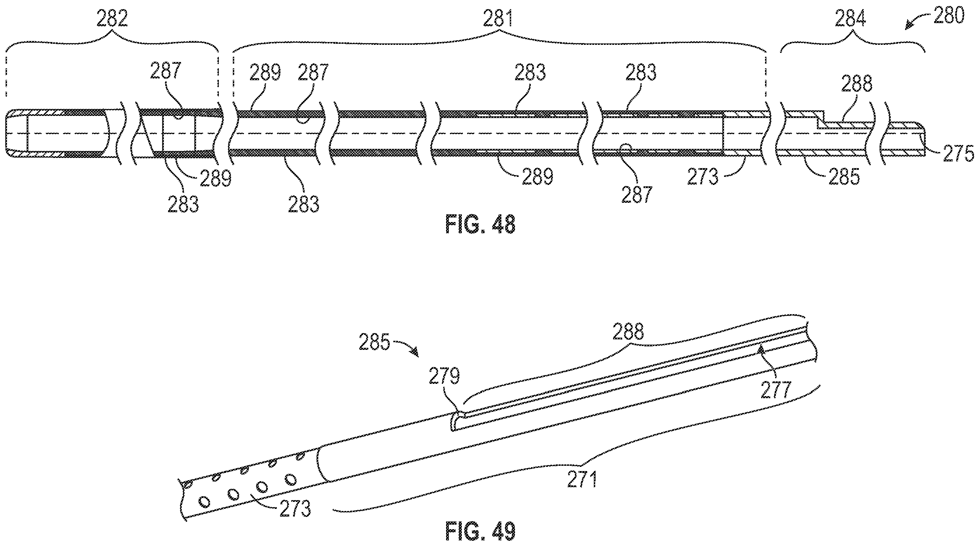

Turning now to FIGS. 48 and 49, a more detailed example of the sleeve shaft 280 is shown. In some examples, as illustrated in FIG. 48, the sleeve shaft 280 comprises three sections: the distal section 282 (or sleeve section), which comprises the lubricous sleeve to cover the docking device during deployment, the proximal section 284 used to manipulate or actuate the sleeve position, and a middle section 281 to connect the distal section 282 and proximal section 284. A portion of the proximal section 284 can be arranged in the handle assembly (as discussed above with reference to FIGS. 5-7). Further, the middle section 281 and at least a portion of the proximal section 284 can surround the pusher shaft (e.g., pusher shaft 290 shown in FIGS. 6-8 and/or pusher shaft 1500 shown in FIGS. 34 and 35).

The sleeve shaft 280 can be formed by a plurality of components and/or materials. In some examples, the sleeve shaft 280 can be formed by a flexible polymer jacket 283 (FIG. 48), a more rigid tube 285 (FIGS. 48 and 49), an inner liner 287 (FIG. 48), and a metal braid 289 (FIG. 48) (which may be part of or imbedded within portions of the polymer jacket 283). As shown in FIG. 48, the polymer jacket 283 can be part of the distal section 282 and middle section 281, the inner liner 287 can extend along and form an interior surface of the distal section 282 and the middle section 281, and the tube 285 can form the proximal section 284, with a portion that extends into a proximal portion of the middle section 281. In this way, each of the distal section 282, proximal section 284, and middle section 281 of the sleeve shaft 280 can include different layers and compositions of materials.

The proximal section 284 of the sleeve shaft 280 is designed to be more rigid and provide column strength to actuate the position of the distal section 282 relative to the docking device by pushing the middle section 281 and distal section 282 with the docking device (e.g., docking device 232) and retracting the distal section 282 after the docking device encircles the native anatomy. Since the proximal section 284 of the sleeve shaft 280 surrounds the pusher shaft (e.g., the pusher shaft 1500 shown in FIGS. 34 and 35 or the pusher shaft 290 shown in FIGS. 6-9B), the structure can be shaped and configured to be generally tubular in structure and more rigid. For example, the proximal section 284 can be formed by a relatively rigid tube 285 (FIGS. 48 and 49). In some examples, the tube 285 can be constructed of a surgical grade metal, such as stainless steel. In some examples, the tube 285 can be a hypo tube.

The tube 285 can include a first section 271 (FIG. 49) (which can form the entirety of the proximal section 284) and a second section 273 which extends into the middle section 281 (FIGS. 48 and 49). The first section 271 includes the cut portion 288 which has a cross-section (in a plane normal to a central longitudinal axis 275 of the sleeve shaft 280) that is not a complete circle (e.g., is open and does not form a closed tube), as introduced above. A remainder of the tube 285 can be tubular (e.g., a closed tube having a relatively circular cross-section). In this way, the tube 285 can be a hollow tube that has a fully circular cross-section in its second section 273 and a distal portion of the first section 271 and a partially circular cross-section in the cut portion 288 (which can also be referred to as a rail of the sleeve shaft 280).

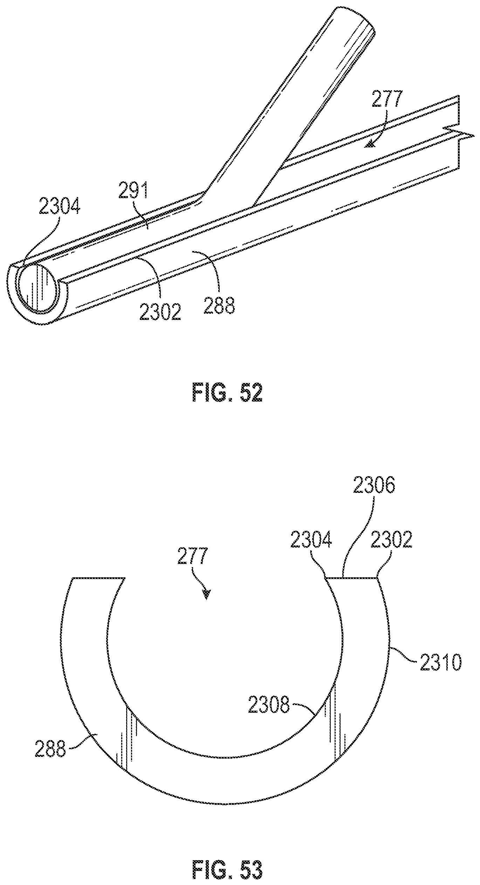

As described above with reference to FIGS. 5-7, the cut portion 288 of the sleeve shaft 280 extends into the hub assembly 230 of the handle assembly 200 and a portion (e.g., proximal extension 291) of the pusher shaft 290 extends along an inner surface of the cut portion 288 (also shown in FIG. 52). The cut (e.g., open) profile of the cut portion 288 can allow the proximal extension 291 of the pusher shaft 290 (or any of the other pusher shafts described herein) to extend out of a void space 277 (or opening) formed in the cut portion 288 (FIGS. 49 and 52) and branch off, at an angle relative to the cut portion 288, into the branch 204 of the hub assembly 230. As such, the pusher shaft 290 and sleeve shaft 280 can be operated in parallel with one another, as described above.

In some examples, as shown in FIGS. 49 and 51-53, the cut portion 288 can have a generally U or C-shaped cross-section with a portion of the complete tubular structure removed. For example, the cut portion 288 can form an open channel or conduit having void space 277 (FIGS. 49, 52, and 53). In various examples, the cut portion 288 can be cut using a laser, although any other means for removing part of the tubular structure can be used.

An end surface 279 is formed (e.g., exposed) on the full, tubular portion of the first section 271, at an interface between the cut portion 288 and the remainder of the first section 271 (FIG. 49). This end surface 279 can be arranged normal to the central longitudinal axis 275 and can be configured to come into face-sharing contact with a stop element (e.g., plug 1506) of the pusher shaft (e.g., as shown in FIG. 35, described above).

The proximal section 284 of the sleeve shaft 280 can be cut to form the partial circular cross-section of the cut portion 288. In some examples, the proximal section 284 can be cut by electrical discharge machining (EDM cut). However, cutting the tube 285 in this manner can leave a relatively flat (planar) cut surface 2306 on either side of the void space 277, each cut surface 2306 having a first edge 2302 on an outer diameter of the cut portion 288 (e.g., a corner between the cut surface 2306 of the cut portion 288 and an inner surface 2308 of the cut portion 288 of the tube 285) and a second edge 2304 on an inner diameter of the cut portion 288 (e.g., a corner between the cut surface 2306 and an outer surface 2310 of the cut portion 288 of the tube 285) that are relatively sharp (FIGS. 52 and 53). For example, the first and second edges 2302 and 2304 can be angled and non-rounded.

In some examples, the relatively sharp inner and outer edges (first and second edges 2302 and 2304) on the cut surface 2306 of the cut portion 288 of the sleeve shaft 280 can be rounded and/or deburred, thereby eliminating or reducing the sharpness of the first and second edges 2302 and 2304.

Creating more rounded and smoother edges at the cut surface 2306 can allow for a smoother interface between mating components and the cut portion 288. For example, the first edge 2302 (outer edge) and/or the second edge 2304 can interface with various gaskets, seals, and/or washers disposed around the cut portion 288 of the tube 285 of the sleeve shaft 280.

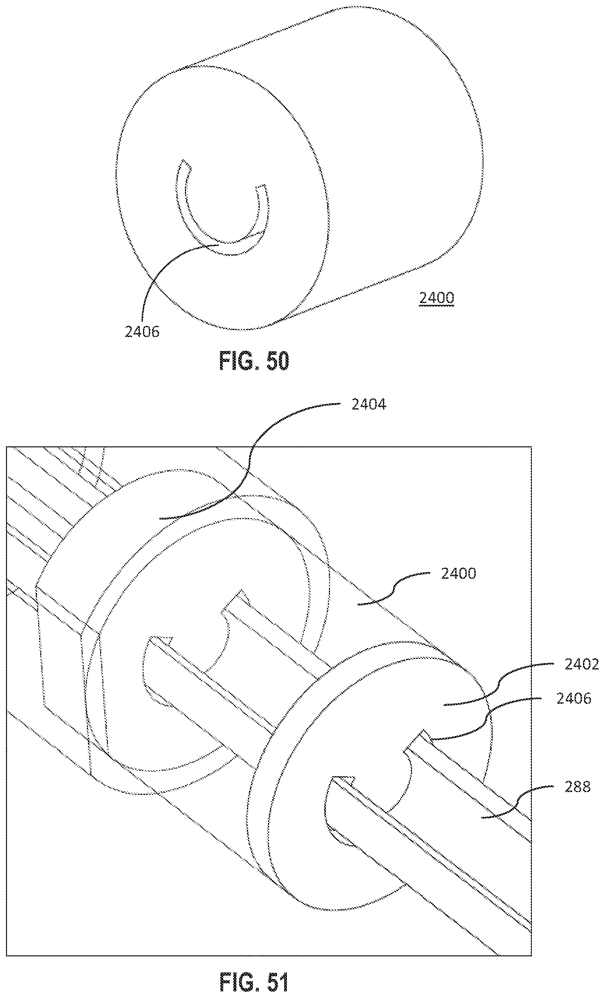

For example, as shown in FIGS. 50 and 51, a hemostatic seal 2400 can be used to seal around the cut portion 288 of the proximal section 284 of the sleeve shaft 280, proximate to the sleeve actuating handle (e.g., sleeve handle 208 shown in FIG. 5). As seen in FIG. 50, the hemostatic seal 2400 can possess an opening 2406 in the shape of a cross-section of the cut portion 288 of the sleeve shaft 280, such as a U or C-shape or incomplete (e.g., partial) annulus, configured to receive the cut portion 288 therein and to seal on all sides of the sleeve shaft 280. FIG. 51 illustrates an example of the hemostatic seal 2400 arranged within the straight section 202 of the hub assembly 230. In some examples, as shown in FIG. 51, two rigid washers 2402 and 2404 can support each end of the hemostatic seal 2400. The rigid washers 2402, 2404 can possess the same profile as the hemostatic seal 2400 to maintain the integrity of the hemostatic seal 2400. The rigid washers 2402, 2404 can place inward pressure on the hemostatic seal 2400 to ensure a seal between the hemostatic seal 2400 and the cut portion 288 of the sleeve shaft 280.

Thus, it is desirable to reduce or eliminate the sharp corners or edges at the inner and outer edges (first and second edges 2302 and 2304) of the cut portion 288 to create a smoother edge for interfacing with mating components. In some examples, it may be desirable to form fully rounded edges at the first and second edges 2302 and 2304 of the cut portion 288.

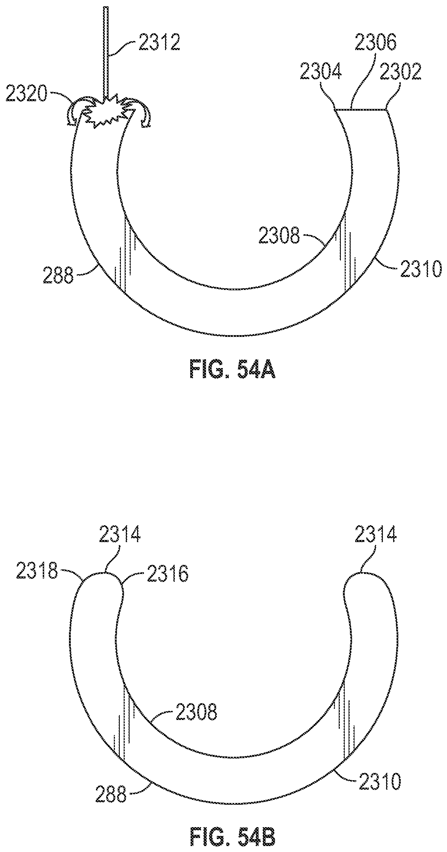

In some examples, the first and second edges 2302 and 2304 can be rounded by a laser. For example, FIGS. 54A and 54B illustrate an exemplary process for rounding and/or deburring the relatively sharp cut edges on the cut surface 2306 (first edge 2302 and second edge 2304) of the cut portion 288, thereby forming fully rounded edges or corners, or at least deburred edges, on the cut surface 2306 (or a fully rounded and/or deburred cut surface 2306 without sharp edges).

For example, a laser 2312 (e.g., a beam of a laser) can be directed at the cut surface 2306 and applied for a predetermined amount of time and/or at a predetermined power setting such that the metal of the cut portion 288 of the sleeve shaft 280 at the cut surface 2306 is melted and reflows toward/over the first and second edges 2302 and 2304 (as shown by arrows 2320 in FIG. 54A) until a desired geometry is achieved, such as the rounded surface 2314 shown in FIG. 54B. A laser may be applied to the cut surface in one pass, two passes, three passes or more, on the flat edge, the first edge, the second edge or both, more in order to achieve the desired roundness. The rounded surface 2314 can be defined by a first rounded corner 2316 (or edge) at the inner surface 2308 of the cut portion 288 and a second rounded corner 2318 at the outer surface 2310 of the cut portion 288. In some examples, the rounded surface 2314 can be a fully rounded surface without sharp corners or edges. For example, the first rounded corner 2316 and the second rounded corner 2318 can be continuous with one another and the inner surface 2308 and outer surface 2310 such that the rounded surface 2314 is formed as a fully rounded surface along the edges of the cut portion 288 of the sleeve shaft 280. In some examples, the rounded surface 2314 can curve between the inner surface 2308 and the outer surface 2310.

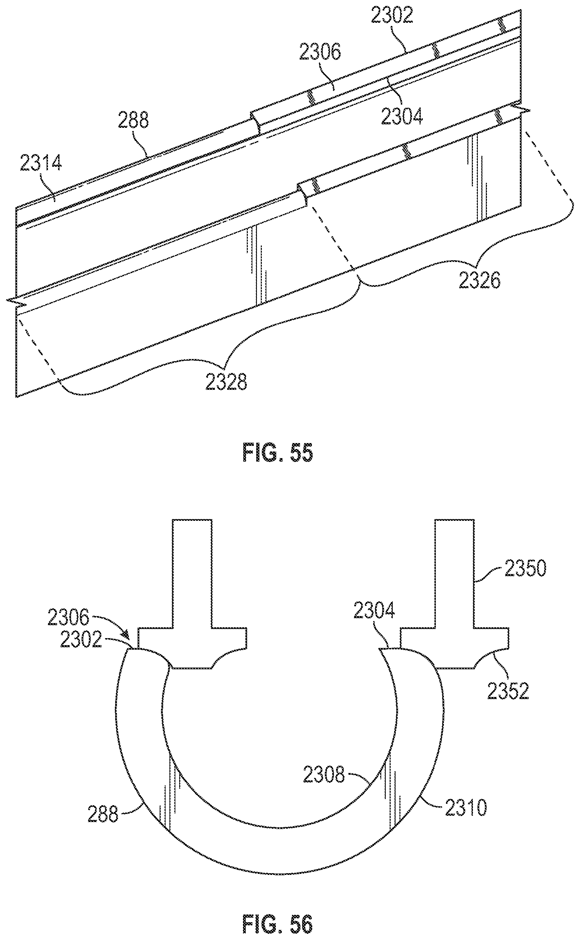

FIG. 55 shows an exemplary cut portion 288 of the sleeve shaft 280 where a first portion 2326 of the cut surface 2306 has been untreated (e.g., no laser welding or ablation has been applied) such that the first edge 2302 and the second edge 2304 remain relatively sharp and a second portion 2328 of the cut surface 2306 has been treated with the laser (e.g., as described above with reference to FIGS. 54A-54B). As shown in FIG. 55, a fully rounded surface 2314 can be achieved with the laser, thereby creating a smoother edge for the cut portion 288.

The process described above and shown in FIGS. 54A-54B can be referred to as a laser weld reflow process. Though the laser weld reflow process described above can be used to form the rounded surface 2314, which in some examples can be a fully rounded surface or edge, in other examples this process can be used to deburr the first edge 2302 and second edge 2304 of the cut surface 2306 to a selected radius (which may or may not result in a fully rounded edge at the cut surface 2306). In some examples, such a process may form rounded edges (similar to the first and second rounded corners 2316 and 2318) with a more planar surface extending between the rounded edges.

In other examples, the relatively sharp first and second edges 2302 and 2304 of the cut surface 2306 of the cut portion 288 of the sleeve shaft 280 (FIGS. 52 and 53) can be rounded and/or deburred by deburring machining (or deburring bit machining). For example, FIG. 56 illustrates an exemplary deburring machining process for rounding and/or deburring the relatively sharp cut edges on the cut surface 2306 (first edge 2302 and second edge 2304) of the cut portion 288, thereby forming fully rounded edges or corners, or at least deburred edges, on the cut surface 2306 (or a fully rounded and/or deburred cut surface 2306 without sharp edges).

For example, a deburring machine bit 2350 can be applied to and run along the cut surface 2306 in order to deburr and/or round the first and second edges 2302 and 2304 (FIG. 56). In some examples, the deburring machine bit 2350 can have a rounded edge 2352 on one or both sides (as shown in FIG. 56) of the deburring machine bit 2350, the rounded edge 2352 sized and shaped to deburr the first and second edges 2302 and 2304 and/or create the rounded edges or surface 2314 shown in FIG. 54B (e.g., with a specified radius of curvature).

FIG. 56 shows the deburring machine bit 2350 deburring and/or rounding the first (inner) edge 2302 on one side of the cut portion 288 and the second (outer) edge 2304 on the other, opposite side of the cut portion 288. **In** this way, in some examples, multiple passes of the deburring machine bit 2350 may be needed to deburr the first and second edges 2302 and 2304 on both sides of the cut portion 288.

In still other examples, the relatively sharp first and second edges 2302 and 2304 of the cut surface 2306 of the cut portion 288 of the sleeve shaft 280 (FIGS. 52 and 53) can be rounded and/or deburred by one or more of bead blasting, electropolishing, sinker electrical discharge machining (EDM), electrochemical machining (ECM), and/or burlytic deburring.

The features described herein with regard to any example can be combined with other features described in any one or more of the other examples, unless otherwise stated. For example, any one or more of the features of one flow mechanism can be combined with any one or more features of another flow mechanism. As another example, any one or more features of one pusher shaft of a delivery apparatus can be combined with any one or more features of another pusher shaft of a delivery apparatus.

In view of the many possible ways in which the principles of the disclosure may be applied, it should be recognized that the illustrated configurations depict examples of the disclosed technology and should not be taken as limiting the scope of the disclosure nor the claims. Rather, the scope of the claimed subject matter is defined by the following claims.

## Claims

1. A delivery apparatus configured to deliver a docking device (232) to a target implantation site in a patient, wherein the docking device (232) is configured to receive a prosthetic valve therein and securely hold the prosthetic valve in place at the implantation site, the delivery apparatus comprising:
an outer shaft (260) configured to retain the docking device (232) in a delivery configuration;
an inner shaft (1500) disposed within the outer shaft (260) and configured to interface with an end of the docking device (232) and move axially relative to the outer shaft (260); and
a sleeve shaft (280) disposed within the outer shaft (260), a portion of the sleeve shaft (280) disposed between the outer shaft (260) and the inner shaft (1500), the sleeve shaft (280) configured to cover the docking device (232) in the delivery configuration;
**characterized in that:**
the inner shaft (1500) includes one or more openings (2204, 2406) defined therein that extend between an inner surface (2212) and an outer surface (2206) of the inner shaft (1500) and that are configured to fluidly couple an inner lumen (1555) of the inner shaft (1500) with a lumen disposed between the outer surface (2206) of the inner shaft (1500) and an inner surface of the sleeve shaft (280).

2. The delivery apparatus of claim 1, wherein the one or more openings (2204, 2406) are disposed in a distal end portion (1514) of the inner shaft (1500).

3. The delivery apparatus of claim 1, wherein the one or more openings (2204, 2406) are disposed in a portion of the inner shaft (1500) that is spaced away from a distal end portion (1514) of the inner shaft (1500).

4. The delivery apparatus of any one of claims 1 to 3, wherein the one or more openings (2204, 2406) include at least two openings (2204, 2406) spaced apart from one another around a circumference of the inner shaft (1500).

5. The delivery apparatus of any one of claims 1 to 4, wherein the inner shaft (1500) comprises a main tube (1502), wherein a distal section of the main tube (1502) includes a plurality of cuts (1520) therein, spaced apart from one another along a length of the distal section, and wherein the one or more openings (2204, 2406) are disposed in a portion of the inner shaft (1500) arranged adjacent to the distal section.

6. The delivery apparatus of claim 5, wherein the one or more openings (2204, 2406) are configured as apertures than extend through the main tube (1502), an inner liner (1538) covering an inner surface of the main tube (1502), and an outer polymer layer (1540) covering an outer surface of the main tube (1502), wherein the main tube (1502) of the inner shaft (1500) includes an intermediate section (1535) arranged adjacent and proximal to the distal section, and wherein the one or more openings (2204, 2406) are disposed in the intermediate section (1535).

7. The delivery apparatus of either claim 5 or claim 6, wherein the one or more openings (2204, 2406) are configured as apertures than extend through the main tube (1502), an inner liner (1538) covering an inner surface of the main tube (1502), and an outer polymer layer (1540) covering an outer surface of the main tube (1502), wherein the one or more openings (2204, 2406) are disposed in a distal end portion (1514) of the inner shaft (1500) that is arranged adjacent and distal to the plurality of cuts (1520) of the distal section of the main tube (1502).

8. The delivery apparatus of either claim 1 or claim 2, wherein the one or more openings (2204, 2406) are one or more slots (1702) disposed in a distal end portion (1514) of the inner shaft (1500) and wherein each slot of the one or more slots (1702) extends from a distal end of the inner shaft (1500) to a distance away from the distal end, in a proximal direction.

9. The delivery apparatus of claim 8, wherein the distal end portion (1514) of the inner shaft (1500) comprises a polymeric distal end portion (1544) comprising a flexible polymer and a distal end of a rigid, main tube (1502) of the inner shaft (1500), the polymeric distal end arranged distal to the distal end of the main tube (1502), and wherein each slot extends through only the polymeric distal end portion (1544), distal to the distal end of the main tube (1502).

10. The delivery apparatus of either claim 8 or claim 9, wherein the one or more slots (1702) include two slots (1702) spaced apart from one another around a circumference of the distal end portion (1514) and wherein the two slots (1702) are arranged 180 degrees apart from one another around the circumference of the distal end portion (1514).

11. The delivery apparatus of either claim 1 or claim 2, wherein the inner shaft (1500) includes a distal tip (1541) arranged around a distal end portion (1514) of a main tube (1502) of the inner shaft (1500), wherein the distal tip (1541) is at least partially covered by a flexible polymer layer that also covers the main tube (1502), the distal tip (1541) including a tip portion extending distally past the main tube (1502) and the flexible polymer layer that includes the one or more openings (2204, 2406) therein.

12. The delivery apparatus of claim 11, wherein the distal tip (1541) comprises an extruded or molded polymeric material.

13. The delivery apparatus of either claim 1 or claim 2, wherein the one or more openings (2204, 2406) are disposed in a polymeric tip of the inner shaft (1500), the polymeric tip arranged at a distal end of the inner shaft (1500), wherein the inner shaft (1500) comprises a main tube (1502), an outer polymer layer (1540) covering an outer surface of the main tube (1502), and an inner liner (1538) covering an inner surface of the main tube (1502), and wherein the polymeric tip is continuous with the outer polymer layer (1540) and extends distally past a distal end of the main tube (1502).

14. The delivery apparatus of any one of the previous claims, wherein the inner surface and the outer surface of the inner shaft (1500) are circumferential surfaces, where a line normal to the inner surface and the outer surface intersects a central longitudinal axis of the delivery apparatus.

15. The delivery apparatus of any one of the previous claims, further comprising the docking device (232) arranged within the outer shaft (260) of the delivery apparatus and distal of the inner shaft (1500),
wherein the sleeve shaft (280) covers the docking device (232) in the delivery configuration, and
wherein the inner shaft (1500) is a pusher shaft configured to push the docking device (232) out of the outer shaft (260) to position the docking device (232) at the target implantation site.

## Patentansprüche

1. Zuführvorrichtung, die dazu ausgelegt ist, eine Andockvorrichtung (232) zu einer Zielimplantationsstelle in einem Patienten zuzuführen, wobei die Andockvorrichtung (232) dazu ausgelegt ist, eine Klappenprothese aufzunehmen und die Klappenprothese sicher an der Implantationsstelle zu halten, wobei die Zuführvorrichtung umfasst:
einen äußeren Schaft (260), der dazu ausgelegt ist, die Andockvorrichtung (232) in einer Zuführkonfiguration zu halten;
einen inneren Schaft (1500), der innerhalb des äußeren Schafts (260) angeordnet und dazu ausgelegt ist, mit einem Ende der Andockvorrichtung (232) zusammenzuwirken und sich axial relativ zum äußeren Schaft (260) zu bewegen; und
einen Hülsenschaft (280), der innerhalb des äußeren Schafts (260) angeordnet ist, wobei ein Abschnitt des Hülsenschafts (280) zwischen dem äußeren Schaft (260) und dem inneren Schaft (1500) angeordnet ist und der Hülsenschaft (280) dazu ausgelegt ist, die Andockvorrichtung (232) in der Zuführkonfiguration zu bedecken;
**dadurch gekennzeichnet, dass** der innere Schaft (1500) eine oder mehrere darin definierte Öffnungen (2204, 2406) aufweist, die sich zwischen einer inneren Oberfläche (2212) und einer äußeren Oberfläche (2206) des inneren Schafts (1500) erstrecken und die dazu ausgelegt sind, ein inneres Lumen (1555) des inneren Schafts (1500) fluidisch mit einem Lumen zu koppeln, das zwischen der äußeren Oberfläche (2206) des inneren Schafts (1500) und einer inneren Oberfläche des Hülsenschafts (280) angeordnet ist.

2. Zuführvorrichtung gemäß Anspruch 1, wobei die eine oder die mehreren Öffnungen (2204, 2406) in einem distalen Endabschnitt (1514) des inneren Schafts (1500) angeordnet sind.

3. Zuführvorrichtung gemäß Anspruch 1, wobei die eine oder die mehreren Öffnungen (2204, 2406) in einem Abschnitt des inneren Schafts (1500) angeordnet sind, der von einem distalen Endabschnitt (1514) des inneren Schafts (1500) beabstandet ist.

4. Zuführvorrichtung gemäß einem der Ansprüche 1 bis 3, wobei die eine oder die mehreren Öffnungen (2204, 2406) zumindest zwei Öffnungen (2204, 2406) aufweisen, die um einen Umfang des inneren Schafts (1500) voneinander beabstandet sind.

5. Zuführvorrichtung gemäß einem der Ansprüche 1 bis 4, wobei der innere Schaft (1500) ein Hauptrohr (1502) umfasst, wobei ein distaler Abschnitt des Hauptrohrs (1502) eine Vielzahl von Schnitten (1520) aufweist, die entlang einer Länge des distalen Abschnitts voneinander beabstandet sind, und wobei die eine oder die mehreren Öffnungen (2204, 2406) in einem Abschnitt des inneren Schafts (1500) angeordnet sind, der benachbart zu dem distalen Abschnitt angeordnet ist.

6. Zuführvorrichtung gemäß Anspruch 5, wobei die eine oder die mehreren Öffnungen (2204, 2406) als Durchgangsöffnungen ausgelegt sind, die sich durch das Hauptrohr (1502) erstrecken, wobei eine Innenauskleidung (1538) eine innere Oberfläche des Hauptrohrs (1502) bedeckt und eine äußere Polymerschicht (1540) eine äußere Oberfläche des Hauptrohrs (1502) bedeckt, wobei das Hauptrohr (1502) des inneren Schafts (1500) einen Zwischenabschnitt (1535) aufweist, der benachbart und proximal zum distalen Abschnitt angeordnet ist, und wobei die eine oder die mehreren Öffnungen (2204, 2406) im Zwischenabschnitt (1535) angeordnet sind.

7. Zuführvorrichtung gemäß Anspruch 5 oder 6, wobei die eine oder die mehreren Öffnungen (2204, 2406) als Durchgangsöffnungen ausgelegt sind, die sich durch das Hauptrohr (1502) erstrecken, wobei eine Innenauskleidung (1538) eine innere Oberfläche des Hauptrohrs (1502) bedeckt und eine äußere Polymerschicht (1540) eine äußere Oberfläche des Hauptrohrs (1502) bedeckt, wobei die eine oder die mehreren Öffnungen (2204, 2406) in einem distalen Endabschnitt (1514) des inneren Schafts (1500) angeordnet sind, der benachbart und distal zu der Vielzahl von Schnitten (1520) des distalen Abschnitts des Hauptrohrs (1502) angeordnet ist.

8. Zuführvorrichtung gemäß Anspruch 1 oder 2, wobei die eine oder die mehreren Öffnungen (2204, 2406) ein oder mehrere Schlitze (1702) sind, die in einem distalen Endabschnitt (1514) des inneren Schafts (1500) angeordnet sind, und wobei sich jeder Schlitz des einen oder der mehreren Schlitze (1702) von einem distalen Ende des inneren Schafts (1500) über eine Entfernung vom distalen Ende in proximaler Richtung erstreckt.

9. Zuführvorrichtung gemäß Anspruch 8, wobei der distale Endabschnitt (1514) des inneren Schafts (1500) einen polymeren distalen Endabschnitt (1544), der ein flexibles Polymer umfasst, und ein distales Ende eines starren Hauptrohrs (1502) des inneren Schafts (1500) umfasst, wobei der polymere distale Endabschnitt distal zu dem distalen Ende des Hauptrohrs (1502) angeordnet ist, und wobei sich jeder Schlitz nur durch den polymeren distalen Endabschnitt (1544) erstreckt, distal zu dem distalen Ende des Hauptrohrs (1502).

10. Zuführvorrichtung gemäß Anspruch 8 oder 9, wobei der eine oder die mehreren Schlitze (1702) zwei Schlitze (1702) umfassen, die von einander um einen Umfang des distalen Endabschnitts (1514) beabstandet sind, und wobei die zwei Schlitze (1702) 180 Grad um den Umfang des distalen Endabschnitts (1514) herum voneinander entfernt angeordnet sind.

11. Zuführvorrichtung gemäß Anspruch 1 oder 2, wobei der innere Schaft (1500) eine distale Spitze (1541) aufweist, die um einen distalen Endabschnitt (1514) eines Hauptrohrs (1502) des inneren Schafts (1500) angeordnet ist, wobei die distale Spitze (1541) zumindest teilweise von einer flexiblen Polymerschicht bedeckt ist, die auch das Hauptrohr (1502) bedeckt, wobei die distale Spitze (1541) einen Spitzenabschnitt umfasst, der sich distal über das Hauptrohr (1502) und die flexible Polymerschicht, die die eine oder die mehreren Öffnungen (2204, 2406) aufweist, hinaus erstreckt.

12. Zuführvorrichtung gemäß Anspruch 11, wobei die distale Spitze (1541) ein extrudiertes oder gegossenes polymeres Material umfasst.

13. Zuführvorrichtung gemäß Anspruch 1 oder 2, wobei die eine oder die mehreren Öffnungen (2204, 2406) in einer polymeren Spitze des inneren Schafts (1500) angeordnet sind, wobei die polymere Spitze an einem distalen Ende des inneren Schafts (1500) angeordnet ist, wobei der innere Schaft (1500) ein Hauptrohr (1502), eine äußere Polymerschicht (1540), die eine äußere Oberfläche des Hauptrohrs (1502) bedeckt, und eine Innenauskleidung (1538), die eine innere Oberfläche des Hauptrohrs (1502) bedeckt, umfasst, und wobei die polymere Spitze kontinuierlich mit der äußeren Polymerschicht (1540) ist und sich distal über ein distales Ende des Hauptrohrs (1502) hinaus erstreckt.

14. Zuführvorrichtung gemäß einem der vorhergehenden Ansprüche, wobei die innere Oberfläche und die äußere Oberfläche des inneren Schafts (1500) Umfangsflächen sind, wobei eine zu der inneren Oberfläche und der äußeren Oberfläche senkrechte Linie eine zentrale Längsachse der Zuführvorrichtung schneidet.

15. Zuführvorrichtung gemäß einem der vorhergehenden Ansprüche, ferner umfassend die Andockvorrichtung (232), die innerhalb des äußeren Schafts (260) der Zuführvorrichtung und distal zu dem inneren Schaft (1500) angeordnet ist,
wobei der Hülsenschaft (280) die Andockvorrichtung (232) in der Zuführkonfiguration bedeckt,
und wobei der innere Schaft (1500) ein Schubschaft ist, der dazu ausgelegt ist, die Andockvorrichtung (232) aus dem äußeren Schaft (260) zu schieben, um die Andockvorrichtung (232) an der Zielimplantationsstelle zu positionieren.

## Revendications

1. Appareil de pose conçu pour poser un dispositif d'ancrage (232) à un site d'implantation cible chez un patient, le dispositif d'ancrage (232) étant conçu pour recevoir une valvule prothétique en son sein et pour maintenir fermement la valvule prothétique en place au niveau du site d'implantation, l'appareil de pose comprenant :
une tige externe (260) conçue pour retenir le dispositif d'ancrage (232) dans une configuration de pose ;
une tige interne (1500) disposée à l'intérieur de la tige externe (260) et conçue pour interagir avec une extrémité du dispositif d'ancrage (232) et pour se déplacer axialement par rapport à la tige externe (260) ; et
une tige (280) de manchon disposée à l'intérieur de la tige externe (260), une partie de la tige (280) de manchon étant disposée entre la tige externe (260) et la tige interne (1500), la tige (280) de manchon étant conçu pour recouvrir le dispositif d'ancrage (232) dans la configuration de pose ;
**caractérisé en ce que** :
la tige interne (1500) comprend une ou plusieurs ouvertures (2204, 2406) définies en son sein qui s'étendent entre une surface interne (2212) et une surface externe (2206) de la tige interne (1500) et qui sont conçues pour accoupler de manière fluidique une lumière interne (1555) de la tige interne (1500) et une lumière disposée entre la surface externe (2206) de la tige interne (1500) et une surface interne de la tige (280) de manchon.

2. Appareil de pose selon la revendication 1, ladite une ou lesdites plusieurs ouvertures (2204, 2406) étant disposées dans une partie extrémité distale (1514) de la tige interne (1500).

3. Appareil de pose selon la revendication 1, ladite une ou lesdites plusieurs ouvertures (2204, 2406) étant disposées dans une partie de la tige interne (1500) qui est écartée d'une partie extrémité distale (1514) de la tige interne (1500).

4. Appareil de pose selon l'une quelconque des revendications 1 à 3, ladite une ou lesdites plusieurs ouvertures (2204, 2406) comprenant au moins deux ouvertures (2204, 2406) écartées l'une de l'autre autour d'une circonférence de la tige interne (1500).

5. Appareil de pose selon l'une quelconque des revendications 1 à 4, la tige interne (1500) comprenant un tube principal (1502), une section distale du tube principal (1502) comprenant une pluralité de découpes (1520) en son sein, écartées les unes des autres le long d'une longueur de la section distale et ladite une ou lesdites plusieurs ouvertures (2204, 2406) étant disposées dans une partie de la tige interne (1500) agencée de manière adjacente par rapport à la section distale.

6. Appareil de pose selon la revendication 5, ladite une ou lesdites plusieurs ouvertures (2204, 2406) étant conçues sous la forme d'orifices qui s'étendent à travers le tube principal (1502), une doublure interne (1538) recouvrant une surface interne du tube principal (1502) et une couche polymère externe (1540) recouvrant une surface externe du tube principal (1502), le tube principal (1502) de la tige interne (1500) comprenant une section intermédiaire (1535) agencée de manière adjacente et proximale par rapport à la section distale et ladite une ou lesdites plusieurs ouvertures (2204, 2406) étant disposées dans la section intermédiaire (1535).

7. Appareil de pose selon soit la revendication 5 soit la revendication 6, ladite une ou lesdites plusieurs ouvertures (2204, 2406) étant conçues sous la forme d'orifices qui s'étendent à travers le tube principal (1502), une doublure interne (1538) recouvrant une surface interne du tube principal (1502) et une couche polymère externe (1540) recouvrant une surface externe du tube principal (1502), ladite une ou lesdites plusieurs ouvertures (2204, 2406) étant disposées dans une partie extrémité distale (1514) de la tige interne (1500) qui est agencée de manière adjacente et distale par rapport à la pluralité de découpes (1520) de la section distale du tube principal (1502).

8. Appareil de pose selon soit la revendication 1 soit la revendication 2, ladite une ou lesdites plusieurs ouvertures (2204, 2406) étant une ou plusieurs encoches (1702) disposées dans une partie extrémité distale (1514) de la tige interne (1500) et chaque encoche parmi ladite une ou lesdites plusieurs encoches (1702) s'étendant à partir d'une extrémité distale de la tige interne (1500) vers une distance à l'opposé de l'extrémité distale, dans une direction proximale.

9. Appareil de pose selon la revendication 8, la partie extrémité distale (1514) de la tige interne (1500) comprenant une partie extrémité distale (1544) polymère comprenant un polymère flexible et une extrémité distale d'un tube principal (1502) rigide de la tige interne (1500), l'extrémité distale polymère étant agencée de manière distale par rapport à l'extrémité distale du tube principal (1502) et chaque encoche s'étendant uniquement à travers la partie extrémité distale (1544) polymère, de manière distale par rapport à l'extrémité distale du tube principal (1502).

10. Appareil de pose selon soit la revendication 8 soit la revendication 9, ladite une ou lesdites plusieurs encoches (1702) comprenant deux encoches (1702) écartées l'une de l'autre autour d'une circonférence de la partie extrémité distale (1514) et les deux encoches (1702) étant agencées à 180 degrés l'une de l'autre autour de la circonférence de la partie extrémité distale (1514).

11. Appareil de pose selon soit la revendication 1 soit la revendication 2, la tige interne (1500) comprenant une pointe distale (1541) agencée autour d'une partie extrémité distale (1514) d'un tube principal (1502) de la tige interne (1500), la pointe distale (1541) étant au moins partiellement recouverte par une couche polymère flexible qui recouvre également le tube principal (1502), la pointe distale (1541) comprenant une partie pointe s'étendant de manière distale au-delà du tube principal (1502) et la couche polymère flexible qui comprend ladite une ou lesdites plusieurs ouvertures (2204, 2406) en son sein.

12. Appareil de pose selon la revendication 11, la pointe distale (1541) comprenant un matériau polymère extrudé ou moulé.

13. Appareil de pose selon soit la revendication 1 soit la revendication 2, ladite une ou lesdites plusieurs ouvertures (2204, 2406) étant disposées dans une pointe polymère de la tige interne (1500), la pointe polymère étant agencée au niveau d'une extrémité distale de la tige interne (1500), la tige interne (1500) comprenant un tube principal (1502), une couche polymère externe (1540) recouvrant une surface externe du tube principal (1502) et une doublure interne (1538) recouvrant une surface interne du tube principal (1502) et la pointe polymère étant continue avec la couche polymère externe (1540) et s'étendant de manière distale au-delà d'une extrémité distale du tube principal (1502).

14. Appareil de pose selon l'une quelconque des revendications précédentes, la surface interne et la surface externe de la tige interne (1500) étant des surfaces circonférentielles, une ligne perpendiculaire à la surface interne et à la surface externe croisant un axe longitudinal central de l'appareil de pose.

15. Appareil de pose selon l'une quelconque des revendications précédentes, comprenant en outre le dispositif d'ancrage (232) agencé à l'intérieur de la tige externe (260) de l'appareil de pose et de manière distale par rapport à la tige interne (1500),
la tige (280) de manchon recouvrant le dispositif d'ancrage (232) dans la configuration de pose et
la tige interne (1500) étant une tige de poussée conçue pour pousser le dispositif d'ancrage (232) hors de la tige externe (260) afin de positionner le dispositif d'ancrage (232) au niveau du site d'implantation cible.
